# EUROPEAN PATENT APPLICATION

(11) **EP 4 317 143 A1**
(43) Date of publication of application: **07.02.2024**
(21) Application number: 21932653.5
(22) Date of filing: 10.11.2021
(51) Int. Cl.: C07D 401/14, C07D 471/04, A61K 31/519, A61P 35/00, A61P 35/02

(54) **BICYCLIC HETEROCYCLIC FGFR4 INHIBITOR, PHARMACEUTICAL COMPOSITION AND PREPARATION COMPRISING SAME, AND APPLICATION THEREOF**

(30) Priority: 26.03.2021 CN 202110326618
(71) Applicant: Hangzhou Apeloa Medicine Research Institute Co., Ltd., Hangzhou, Zhejiang 310016 (CN); Apeloa Pharmaceutical Co., Ltd., Zhejiang 322118 (CN)
(72) Inventor: ZHAN, Weiqiang, Hengdian Dongyang, Zhejiang 322118 (CN); WANG, Zhanguo, Hengdian Dongyang, Zhejiang 322118 (CN); CHEN, Weiliang, Hengdian Dongyang, Zhejiang 322118 (CN); JIN, Yaofeng, Hengdian Dongyang, Zhejiang 322118 (CN); LV, Jianhua, Hengdian Dongyang, Zhejiang 322118 (CN); LIANG, Sai, Hengdian Dongyang, Zhejiang 322118 (CN); GUO, Xuejie, Hengdian Dongyang, Zhejiang 322118 (CN); ZHU, Fangmeng, Hengdian Dongyang, Zhejiang 322118 (CN)
(74) Representative: Perani & Partners S.p.A.
(86) International application number: PCT/CN2021/129741
(87) International publication number: WO 2022/199045

(57) **Abstract**

The present invention belongs to the field of medicinal chemistry, specifically relates to bicyclic heterocycles as FGFR4 inhibitors, including the pharmaceutical composition and preparation, and applications thereof. In particular, the present invention provides a compound having a structure of formula (I), which may act as an FGFR4 inhibitor to prevent and/or treat the diseases at least partially mediated by FGFR4 (e.g., cancer).

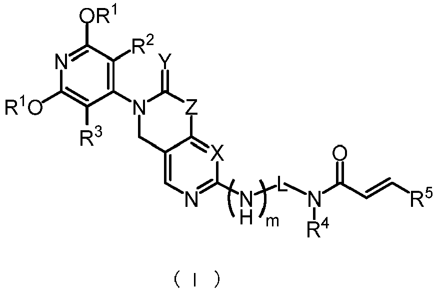

## Description

### Cross-reference of relevant applications

The present invention claims the priority of the invention patent application filed in China on March 26, 2021, named "Bicyclic heterocycles as FGFR4 inhibitors, including the pharmaceutical composition and preparation, and a use thereof' with the application number 202110326618.7, The entire content of the patent application is hereby incorporated by reference.

### Technical Field

The present invention related to the field of pharmaceutical chemistry, specifically relates to bicyclic heterocycles as FGFR4 inhibitors, including the pharmaceutical composition and preparation, and a use of thereof

### Technology Background

Fibroblast growth factor receptors (FGFR) is a receptor tyrosine kinase (RTKs) with four FGFR protein members in the family: FGFR1, FGFR2, FGFR3 and FGFR4, which are involved in different stages of embryonic development, organ formation, tissue homeostasis, angiogenesis, and inflammation. When bound to fibroblast growth factor (FGF) ligands, FGFR undergoes dimerization and phosphorylation, resulting in stimulation of protein kinase activity and recruitment of many intracellular docking proteins, and these interactions affect cell growth, proliferation, differentiation, and other functions through activation of a series of intracellular signaling pathways including Ras-MAPK, AKT-PI3K, and phospholipase C (Eswarakumar V P et, al. Cytokine Growth Factor Reviews, 2005, 16 (2):139-149).

FGFR4 plays an important role in embryonic development, central nervous system control, tissue repair, tumor invasion and angiogenesis (Ho, H. K. et, al. Journal of Hepatology, 2009, 50:118-127).

In addition, overexpression of FGFR4 has been observed in a variety of tumor types, including hepatocellular carcinoma, gastric cancer, renal cell carcinoma, colorectal cancer, breast cancer, pancreatic cancer, prostate cancer, lung cancer, ovarian cancer, etc. FGFR4 is currently believed to be the only receptor that shows specificity for FGF19, which exerts activity by binding to and activating FGFR4. Under pathological conditions, overactivity of MAPK and PI3K/AKT pathways caused by overexpressed FGF19, FGFR4 or mutations in FGFR4 activation will cause tumor development, progression, and resistance to conventional cancer therapies (Heinzle et. al. Cur. Pharm. Des. 2014, 20: 2881).

The study found that about 30% of patients with hepatocellular carcinoma had abnormally activated FGFR4 in their tumors. Desnoyers et al. found that FGF19 monoclonal antibody can selectively block the interaction between FGF19 and FGFR4, which can inhibit the growth of transplanted tumor of human colon cancer in nude mouse and effectively prevent liver cancer in FGF19 transgenic mice (Desnoyers, L. R. et, al. Oncogene, 2008, 27: 85-97), which also proves the feasibility of using small molecule FGFR4 inhibitors to block the binding of extracellular ligand molecules to FGFR4 or intracellular kinase signaling to inhibit FGFR4-mediated signaling, thereby enabling the treatment of malignant tumors such as liver cancer. At present, a number of FGFR4 inhibitors are under clinical study, such as FGF401 developed by Novartis, which can selectively inhibit FGFR4 and shows good prospects; the FGFR4-specific inhibitor BLU554 developed by Blueprint Medicines has strong anti-tumor activity and selectivity, and good safety; the FGFR4-specific inhibitor H3B6527 developed by H3 Biomedicine also exhibits good antitumor activity.

Based on the continuous understanding of the structure, function and mechanism of action of FGFR4 and the interaction of other kinases, the development of FGFR4 inhibitors with strong specificity, good therapeutic effect and low adverse reactions will be of great significance for the prevention and/or treatment of FGFR4-related tumors and other diseases

### Invention Content

### Disclosure of invention

The present invention is intended to provide a series of novel compounds having an inhibitory effect on FGFR4 activity, including the pharmaceutical compositions and preparations, and pharmaceutical uses of the series of compounds.

### Solutions to the Problem

### <Aspect 1>

The present invention provides a compound having a structure in formula (I) or a pharmaceutically acceptable salt, ester, stereoisomer, tautomer, solvate, chelate, non-covalent complex or prodrug thereof, the structural general formula of the compound in formula (I) is: 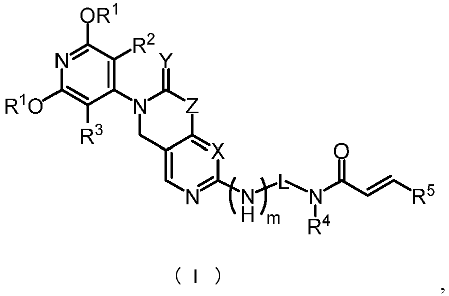 in which,
X is CH or N;
Y is 2 Hs or 1 O;
Z is C(R⁶)₂ or N(R⁶);
m is 0 or 1;
L is -C(R⁷)₂- or 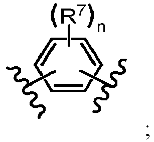
n is 0, 1, or 2;
Each R¹ is independently C₁₋₃ alkyl or C₁₋₃ haloalkyl;
Each R² and R³ are independently hydrogen, halogen, C₁₋₃ alkyl, C₁₋₃ haloalkyl, cyano, or C₁₋₃ alkoxy;
R⁴ is hydrogen, C₁₋₆ alkyl or C₆₋₁₀ aryl;
R⁵ is hydrogen, halogen, cyano, nitro, trifluoromethyl, amino, C₁₋₆ alkyl, C₂₋₈ alkenyl, C₆₋₁₀ aryl, C₁₋₈ alkyl amino, bis (C₂₋₈ alkyl) amino, C₂₋₈ alkynyl, C₁₋₈ haloalkyl or C₃₋₈ cycloalkyl;
Each R⁶ is hydrogen, C₁₋₈ alkyl, C₂₋₈ alkenyl, C₂₋₈ alkynyl, C₁₋₈ haloalkyl, C₆₋₁₀ aryl, C₆₋₁₀ aryl with substituents, C₃₋₁₀ cycloalkyl, 5 to 10 membered heteroaryl or 4 to 10 membered heterocycloalkyl; wherein each 5 to 10 membered heteroaryl or 4 to 10 membered heterocycloalkyl independently contains 1 to 3 cyclic heteroatoms, the heteroatoms are N, O or S independently;
Or two R⁶ and the carbon atoms connected thereto together form a C₃₋₈ cycloalkyl or 4 to 10 membered heterocycloalkyl; wherein the 4 to 10 membered heterocycloalkyl contains 1 to 3 cyclic heteroatoms, the heteroatoms are N, O or S; each C₃₋₈ cycloalkyl and 4 to 10 membered heterocycloalkyl are optionally replaced by 1 to 4 substituents, the substituents are each independently halogen, cyano, hydroxyl, amino, C₁₋₈ carboxyamide, C₁₋₈ carboxylacyl, C₁₋₈ alkyl or C₁₋₈ alkoxy;
Each R⁷ is independently hydrogen, halogen, amino, cyano, C₁₋₈ alkyl, C₁₋₈ alkyl with substituent, C₁₋₈ alkoxy, C₁₋₈ alkoxy with substituents, C₁₋₈ alkyl amino, bis (C₂₋₈ alkyl) amino, C₂₋₈ alkenyl, C₂₋₈ alkenyl with substituents, C₂₋₈ alkynyl, C₂₋₈ alkynyl with substituents, C₆₋₁₀ aryl, C₆₋₁₀ aryl with substituents, C₃₋₈ cycloalkyl, C₃₋₈ cycloalkyl with substituents, 3 to 10 membered heterocycloalkyl, 3 to 10 membered heterocycloalkyl with substituents, 5 to 10 membered heteroaryl or 5 to 10 membered heteroaryl with substituents; wherein each 3 to 10 membered heterocycloalkyl and 5 to 10 membered heteroaryl independently contains 1 to 3 cyclic heteroatoms, and the heteroatoms are N, O or S independently.

Preferably, the compound has a structure as shown in formula (II):

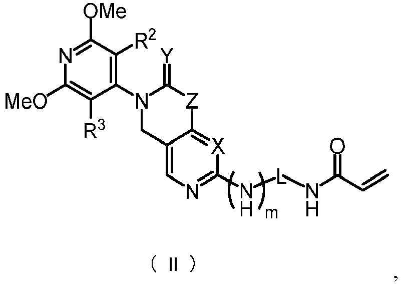

Where X, Y, Z, m, L, R² and R³ are as defined above.

Preferably, the compound has a structure as shown in formula (III):

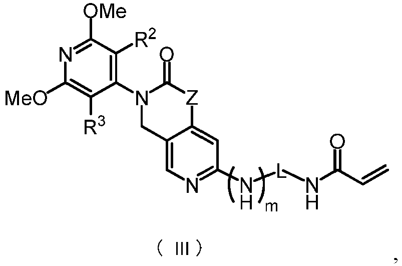

Where Z, m, L, R² and R³ are as defined above.

Further preferably, the compound has a structure as shown in formula (III-1) or formula (III-2):

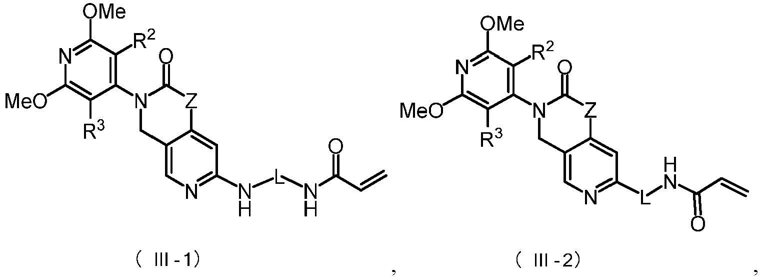

Where Z, L, R² and R³ are as defined above;
Further preferably, the compound of Formula (III-1) has a structure as shown in formula (III-1-1):

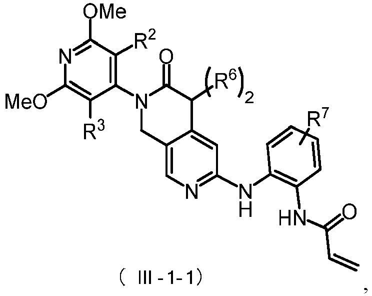

Where R², R³ and R⁶ are as defined above, R⁷ is hydrogen, C₁₋₄ alkyl, piperazinyl, piperidinyl or morpholinoyl, wherein each C₁₋₄ alkyl, piperazinyl, piperidinyl and morpholinoyl are optionally replaced by at least one R⁸. Each R⁸ is independently hydrogen, C₁₋₄ alkyl (preferably methyl or ethyl), morpholinyl, bridged-ring morpholinyl, piperazinyl, piperazinyl with substituents, bridge-ring piperazinyl, bridged-ring piperazinyl with substituents, oxetalkyl or oxetalkyl with the substituents, the substituent is C₁₋₄ alkyl (preferably methyl or ethyl);
Preferably, R², R³ and R⁶ are as defined above, R⁷ is hydrogen, C₁₋₄ alkyl, piperazinyl, piperidinyl or morpholinoyl, wherein each C₁₋₄ alkyl, piperazinyl, piperidinyl and morpholinoyl are optionally replaced by at least one R⁸. Each R⁸ is independently hydrogen, morpholinyl, bridged-ring morpholinyl, piperazinyl, piperazinyl with substituents, bridge-ring piperazinyl, bridged-ring piperazinyl with substituents, oxetalkyl or oxetalkyl with substituents, the substituent is C₁₋₄ alkyl.

More preferably, R², R³ and R⁶ are as defined above, R⁷ is one of the following fragments:

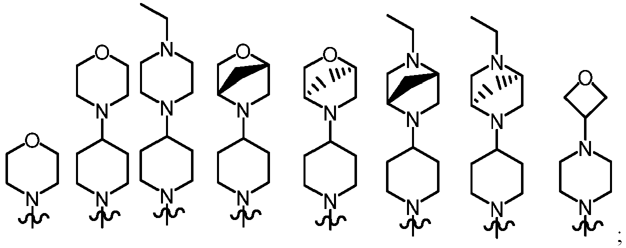

Further preferably, the compound of formula (III-2) has a structure as shown in formula (III-2-1) or formula (III-2-2):

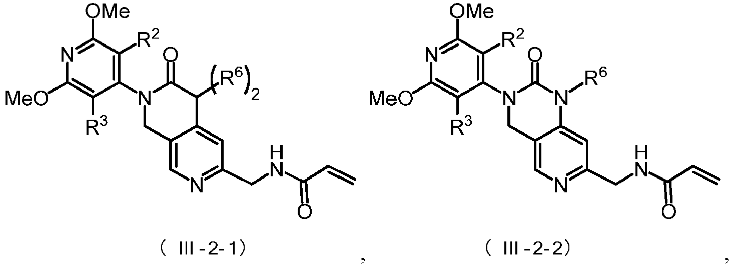

Where, R², R³ and R⁶ are as defined above.

Preferably, the compound has a structure as shown in formula (IV):

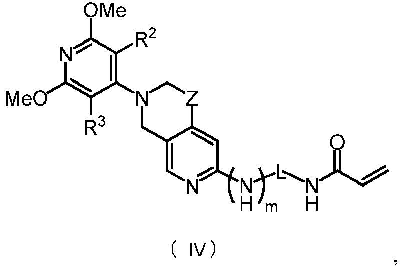

Where Z, m, L, R² and R³ are as defined above.

Further preferably, the compound has a structure as shown in formula (IV-1) or formula (IV-1):

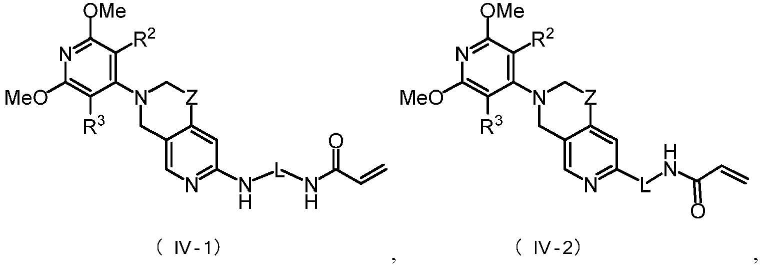

Where Z, L, R² and R³ are as defined above;
Further preferably, the compound of formula (IV-1) has a structure as shown in formula (IV-1-1):

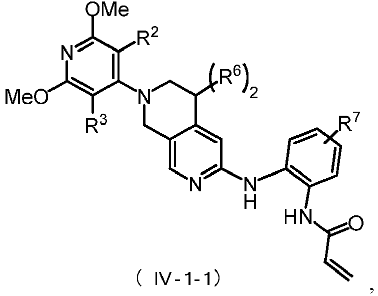

Where, R², R³ and R⁶ are as defined above, R⁷ is hydrogen, C₁₋₄ alkyl, piperazinyl, piperidinyl or morpholinoyl, wherein each C₁₋₄ alkyl, piperazinyl, piperidinyl and morpholinoyl are optionally replaced by at least one R⁸. Each R⁸ is independently hydrogen, morpholinyl, bridged-ring morpholinyl, piperazinyl, piperazinyl with substituents, bridge-ring piperazinyl, bridged-ring piperazinyl with substituents, oxetalkyl or oxetalkyl with substituents, the substituent is C₁₋₄ alkyl.

More preferably, R², R³ and R⁶ are as defined above, R⁷ is one of the following fragments:

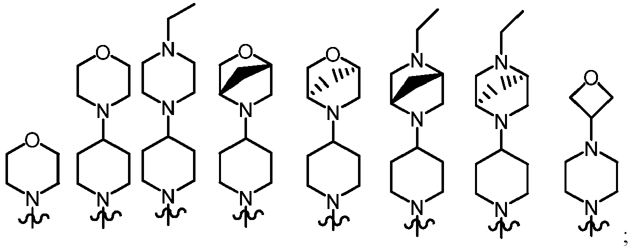

Further preferably, the compound of formula (IV-2) has a structure as shown in formula (IV-2-1) or formula (IV-2-2):

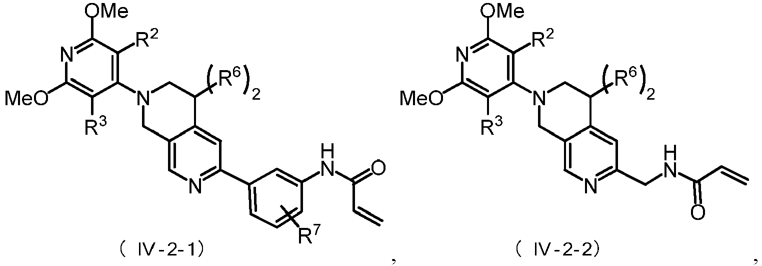

Where, R², R³ and R⁶ are as defined in formula (IV-2-1), R⁷ is hydrogen, C₁₋₄ alkyl or

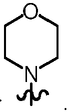

In formula (IV-2-2), R², R³ and R⁶ are as defined above.

### <Aspect 2>

The present invention also provides the following compounds or pharmaceutically acceptable salts, esters, stereoisomers, tautomers, solvates, chelates, non-covalent complexes or prodrugs thereof. 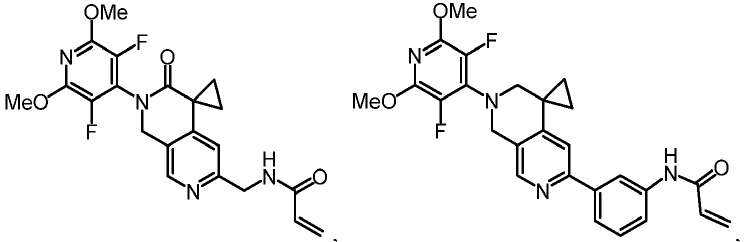 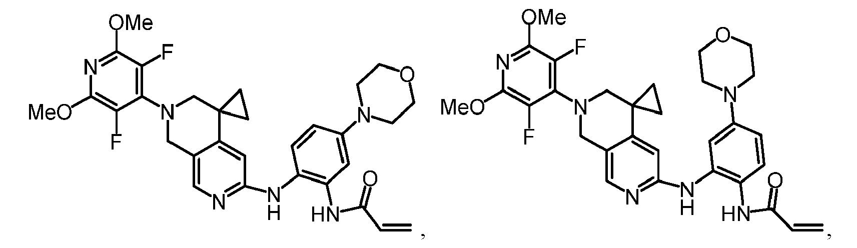 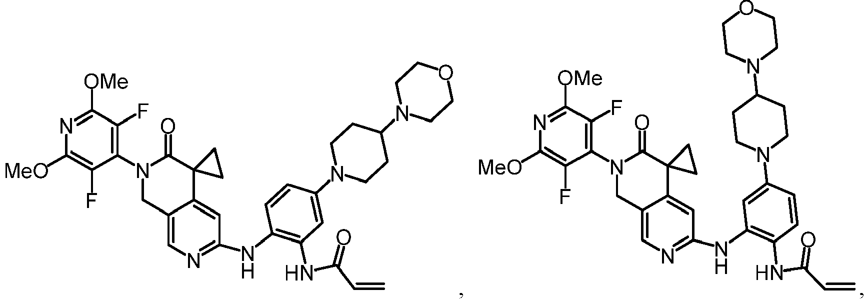 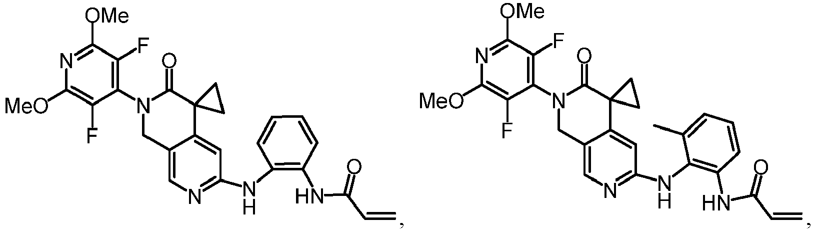 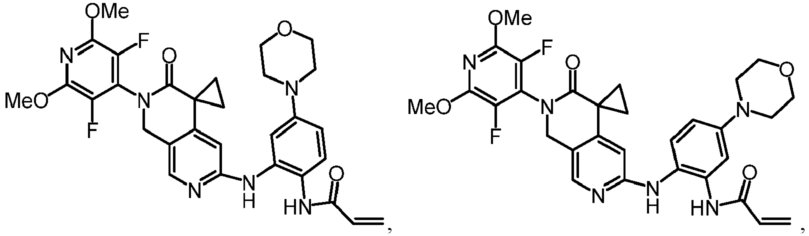 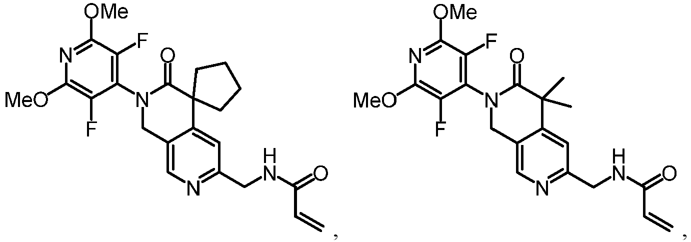 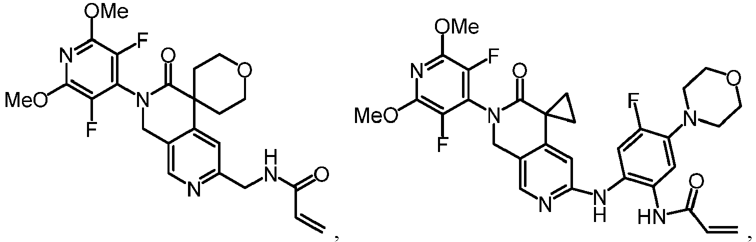 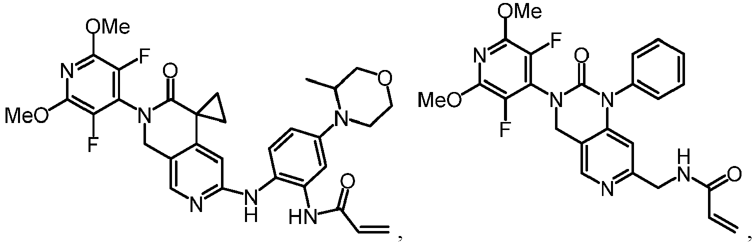 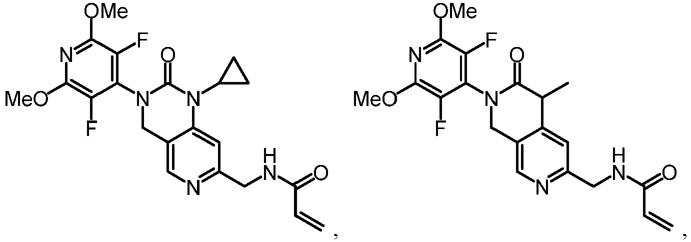 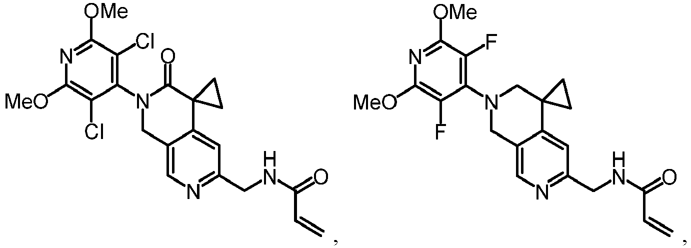 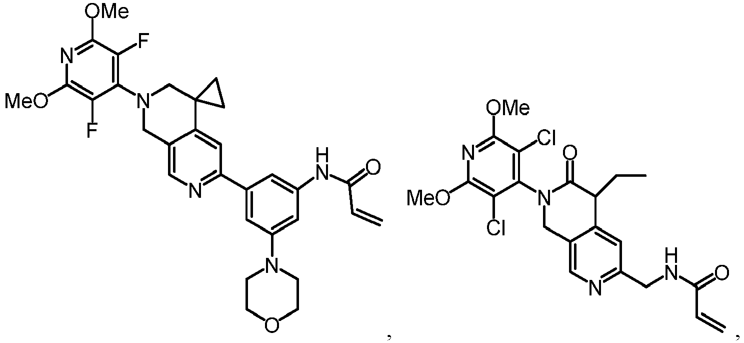 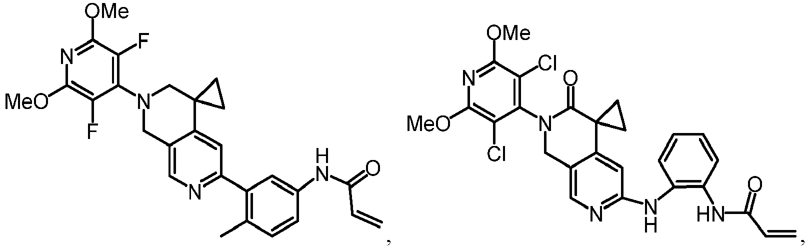 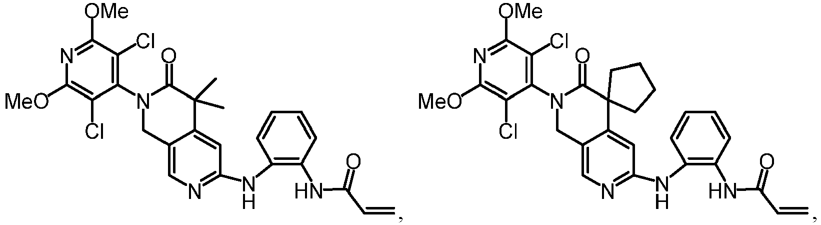 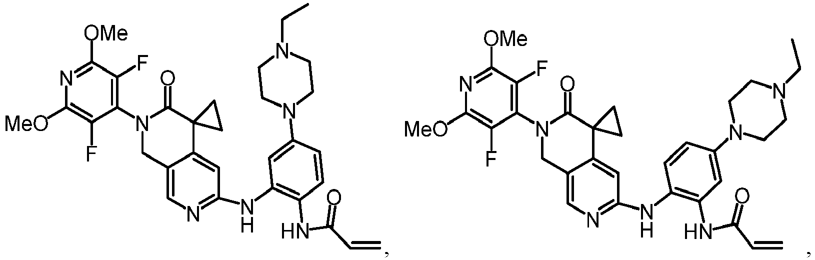 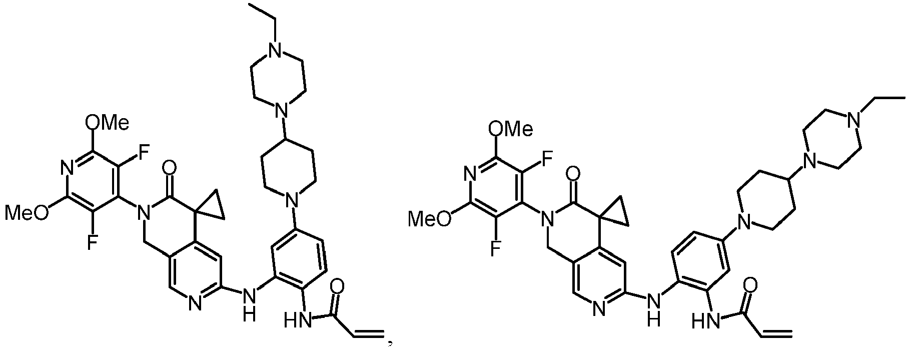 and

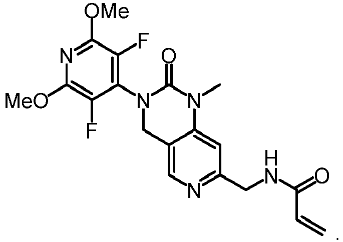

### <Aspect 3>

The present invention also provides a pharmaceutical composition comprising the compound in <Aspect 1> and <Aspect 2>, or pharmaceutically acceptable salts, esters, stereoisomers, tautomers, solvates, chelates, non-covalent complexes or prodrugs thereof.

### <Aspect 4>

The present invention provides a pharmaceutical preparation comprising a compound in <Aspect 1> and <Aspect 2>, or pharmaceutically acceptable salts, esters, stereoisomers, tautomers, solvates, chelates, non-covalent complexes or prodrugs thereof, or a pharmaceutical composition in <Aspect 3>, and the pharmaceutical preparation is any one of tablet, capsule, injection, granule, powder, suppository, pill, gel, pulvis, oral solution, inhalant, suspension, or dry suspension.

### <Aspect 5>

The prevention provides the compound in <Aspect 1> and <Aspect 2>, or pharmaceutically acceptable salts, esters, stereoisomers, tautomers, solvates, chelates, non-covalent complexes or prodrugs thereof, or the pharmaceutical composition in <Aspect 3>, or the pharmaceutical preparation in <Aspect 4> in the preparation of drugs for preventing and/or treating the disease at least partially mediated by FGFR4.

Preferably, the disease at least partially mediated by FGFR4 includes cancer.

Preferably, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

### <Aspect 6>

The prevention provides the compound in <Aspect 1> and <Aspect 2>, or pharmaceutically acceptable salts, esters, stereoisomers, tautomers, solvates, chelates, non-covalent complexes or prodrugs thereof, or the pharmaceutical composition in <Aspect 3>, or the pharmaceutical preparation in <Aspect 4>, as an EGFR4 inhibitor.

### <Aspect 7>

The prevention provides a method for preventing and/or treating the disease at least partially mediated by FGFR4, which comprises the following steps: the prophylactic and therapeutic effective amount for prevention/or treatment of the compound in <Aspect 1> and <Aspect 2>, or the pharmaceutically acceptable salts, esters, stereoisomers, tautomers, solvates, chelates, non-covalent complexes or prodrugs thereof, or the pharmaceutical composition in <Aspect 3>, or the pharmaceutical preparation in <Aspect 4>, is administered to patients in need.

### <Aspect 8>

The prevention provides a method for preventing and/or treating cancer, which comprises the following steps: the prevention/or treatment of effective amounts of compound in <Aspect 1> and <Aspect 2>, or the pharmaceutically acceptable salts, esters, stereoisomers, tautomers, solvates, chelates, non-covalent complexes or prodrugs thereof, or the pharmaceutical composition in <Aspect 3>, or the pharmaceutical preparation in <Aspect 4>, administered to patients in need.

Preferably, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

### <Aspect 9>

The present invention provides a drug combination comprising the compound in <Aspect 1> and <Aspect 2>, or pharmaceutically acceptable salts, esters, stereoisomers, tautomers, solvates, chelates, non-covalent complexes or prodrugs thereof, or the pharmaceutical composition in <Aspect 3>, or the pharmaceutical preparation in <Aspect 4>, and at least one additional cancer therapeutic agent.

### Effect of the Invention

The present invention provides a compound of formula (I) with novel structure, which can be used to prepare pharmaceutical compositions and pharmaceutical preparations etc. for use in human or veterinary medicine to prevent and/or treat diseases (such as tumors, etc.) at least partially mediated by FGFR4 with high specificity, which has strong specificity, good therapeutic effect, and low adverse reaction rate.

### Specific Embodiment

Before further describing the present invention, it should be understood that the present invention is not limited to the specific embodiments described herein; It should also be understood that the terms used herein are intended only to describe and are not limited to a particular embodiment.

### [Terms and Definitions]

Unless otherwise indicated, the following terms and definitions are as follows:
The term "pharmaceutically acceptable salt" is a salt that is substantially non-toxic to an organism of a compound of the present invention. Pharmaceutically acceptable salts generally include (but are not limited to) salts formed by the reaction of compounds of the present invention with pharmaceutically acceptable inorganic/organic acids or inorganic/organic bases, such salts are also called acid addition salts or alkali addition salts. Common inorganic acids include (but are not limited to) hydrochloric acid, hydrobromic acid, sulfuric acid, phosphoric acid, etc., common organic acids include (but are not limited to) trifluoroacetic acid, citric acid, maleic acid, fumaric acid, succinic acid, tartaric acid, lactic acid, pyruvic acid, oxalic acid, formic acid, acetic acid, benzoic acid, methanesulfonic acid, benzenesulfonic acid, p-toluenesulfonic acid, etc., common inorganic bases include (but are not limited to) sodium hydroxide, potassium hydroxide, calcium hydroxide, barium hydroxide, etc., common organic bases include (but are not limited to) diethylamine, triethylamine, ethambutol, etc.

The term "ester" refers to organic esters, including monoesters, diesters, triesters, and more commonly polyesters.

The term "stereoisomer" (or "optical isomer") refers to a stable isomer that has a vertical asymmetric plane due to having at least one chiral factor (including chiral center, chiral axis, chiral face, etc.), which enables the plane to rotate polarized light. Since there are asymmetric centers and other chemical structures in the compounds of the present invention that may cause stereoisomers, the present invention also includes these stereoisomers and mixtures thereof. Since the compounds of the present invention and their salts include asymmetric carbon atoms, they may exist in the form of a single stereoisomer, mixtures of racemates, enantiomers and diastereomers. Typically, these compounds can be prepared in the form of racemic mixtures. However, if desired, such compounds can be prepared or separated to obtain pure stereoisomers, i.e., single enantiomers or diastereomers, or mixtures enriched with single stereoisomers (purity ≥ 98%, ≥ 95%, ≥93%, ≥90%, ≥88%, ≥85% or ≥80%). The single stereoisomer of a compound is prepared by synthesis of optical starting materials containing the desired chiral center, or by separation or fractionation of a mixture of enantiomer products, such as a mixture converted to a diastereomer followed by separation or recrystallization, chromatography, use of chiral selectors, or direct separation of enantiomers on a chiral column. Starting compounds with specific stereochemistry may be commercially available or prepared in accordance with the methods described below and then separated by methods well known in the art.

The term "tautomer" (or "tautomeric form") refers to structural isomers with different energies that can be mutually transformed by low-energy barriers. If tautomerism is possible (e.g. in solution), the chemical equilibrium of the tautomer can be achieved. For example, proton tautomers (or proton transfer tautomers) include (but are not limited to) mutual transformations through proton migration, such as ketone-enol isomerization, imino-enamine isomerization, amide-iminol isomerization, etc. Unless otherwise indicated, all tautomer forms of compounds of the present invention are within the scope of the present invention.

The term "solvate" refers to a substance formed by combining a compound of the present invention or a pharmaceutically acceptable salt with at least one solvent molecule by non-covalent intermolecular forces. Common solvates include (but are not limited to) hydrates, ethanol compounds, acetone compounds, etc.

The term "chelate" is a complex with a cyclic structure, obtained by chelating two or more ligands with the same metal ion to form a chelating ring.

The term "non-covalent complex" is formed by the interaction of a compound with another molecule, where no covalent bond is formed between the compound and the molecule. For example, recombination can achieved through van der Waals interactions, hydrogen bonding, and electrostatic interactions (also known as ion bonding).

The term "prodrug" refers to a derivative compound capable of directly or indirectly providing a compound of the present invention after application to a patient. Particularly preferred derivative compounds or prodrugs thereof are compounds that may enhance the bioavailability of compounds of the present invention when administered to patients (e.g., more easily absorbed into the blood), or compounds that promote delivery of parent compounds to the site of action (e.g., lymphatic system). Unless otherwise indicated, all prodrug forms of compounds of the present invention are within the scope of the present invention, and various prodrug forms are well known in the art.

The term "independently" means that at least two groups (or rings) with the same or similar range of values present in a structure can have the same or different meanings in specific situations. For example, each substituent X and substituent Y are independently hydrogen, halogen, hydroxyl, cyano, alkyl or aryl, then when substituent X is hydrogen, substituent Y can be either hydrogen, halogen, hydroxyl, cyano, alkyl or aryl; similarly, when the substituent Y is hydrogen, the substituent X can be either hydrogen, halogen, hydroxyl, cyanogenic, alkyl or aryl.

The term "halogen" refers to four atoms: fluorine (F), chlorine (Cl), bromine (Br) and iodine (I).

The term "alkyl" includes straight-chain and branched-chain saturated alkyls. For example, alkyl include, but are not limited to, methyl, ethyl, propyl, n-butyl, isobutyl, sec-butyl, tert-butyl, n-pentyl and other similar groups. "C₁₋₈" in "C₁₋₈ alkyl" refers to a group containing 1, 2, 3, 4, 5, 6, 7, or 8 carbon atoms arranged in a straight-chain and branched-chain form."

The term "alkenyl" herein refers to a group that has at least one alkenyl unsaturated site. For example, alkenyl include, but are not limited to, vinyl, propenyl, allyl, isopropenyl, butenyl, isobutenyl, etc. "C₂₋₈ alkenyl" refers to an alkenyl group containing 2, 3, 4, 5, 6, 7 or 8 carbon atoms in a straight-chain, branched-chain or cyclic form, respectively.

The term "alkynyl" herein refers to a group that has at least one alkynyl unsaturated site. For example, alkynyl include, but are not limited to, ethynyl, propyn-1-yl, propyn-2-yl, etc. "C₂₋₈ alkynyl" refers to a alkynyl group containing 2, 3, 4, 5, 6, 7 or 8 carbon atoms in a straight-chain, branched-chain or cyclic form, respectively.

The term "haloalkyl" refers to an alkyl group with up to full valence of halogen atom substituents, which may be same or different. Non-limiting examples of haloalkyl include -CF₃, -C₂F₅, -CHF₂, -CCl₃, -CHCl₂, - C₂Cl₅, etc. "C₁₋₈ haloalkyl" refers to an alkyl, alkenyl or alkynyl containing 1, 2, 3, 4, 5, 6, 7 or 8 carbon atoms arranged in traight-chain, branched-chain or cyclic form containing up to a full-valent halogen atom substituent.

The term "aryl" herein refers to unsubstituted or substituted monocyclic or polycyclic aryl comprising carbon atoms, 6 to 10 full-carbon monocyclic or fused polycyclic (i.e., rings sharing adjacent carbon atom pairs) groups. For example, heteroaryl include, but are not limited to, phenyl, 1-naphthyl, 2-naphthyl and other similar groups.

The term "heteroaryl" herein refers to unsubstituted or substituted monocyclic or multicyclic (e.g., having 2 or 3 condensed rings) aromatic hydrocarbon fractions having one or more heteroatomic ring members independently selected from N, S and O. For example, heteroaryl include, but are not limited to, pyridinyl, pyrimidine, pyrazinyl, pyridazinyl, triazinyl, furyl, thienyl, imidazolyl, thiazolyl, indolyl, pyrryl, oxazolyl, benzofuryl, benzothienyl, benzothiazolyl, isoxazolyl, pyrazolyl, triazolyl, tetrazyl, indazolyl, 1,2,4-thiadiazolyl, isothiazolyl, purinyl, carbazolyl, benzimidazolyl, indolinyl, quinolyl, isoquinolyl, benzoxazoly, imidazo [2,1-b] thiazole and other similar groups.

The term "cycloalkyl" herein refers to non-aromatic cyclic alkyl having monocyclic or polycyclic (including condensed rings, bridge rings, and spirocyclic systems), including cyclic alkyl and alkenyl. For example, cycloalkyl include, but are not limited to, cyclopropyl, cyclobutyl, cyclopentyl, cyclohexylheptyl, cyclopentenyl, cyclohexenyl, cyclohexadienyl, cycloheptatrienyl, norbornene, tetralyl, octahydronaphthyl, indanyl, norpinanyl, etc.

The term "heterocycloalkyl" herein refers to a non-aromatic ring or ring system containing at least one heteroatom selected from O, N and S and optionally containing one or more alkenylene or alkynylene as part of the ring structure. Heterocycloalkyl as a whole can have 3 to 10 ring atoms. Heterocycloalkyl can be covalently linked to a defined chemical structure on any heteroatom or carbon atom that produces a stable structure. For example, heterocycloalkyl include, but are not limited to: pyrrolinyl, piperidyl, piperazinyl, tetrahydrofuranyl, tetrahydropyranyl, morpholinyl, pyranyl, etc. One or more N or S atoms on the heterocycloalkyl can be oxidized (e.g., morpholine N-oxide, thiomorpholine S-oxide, thiomorpholine S, S-dioxide). Heterocycloalkyl may also contain one or more oxo groups, such as phthalimido, piperidinonyl, oxazolidinonyl, 2,4(1H,3H)-dioxo-pyrimidinyl, pyridine-2(1H)-keto, etc.

The term "alkyl amino" refers to a group with the formula -NH (alkyl). In some embodiments, the alkyl amino group has 1 to 8 carbon atoms. Non-limiting examples of alkyl amino groups include methyl amino, ethyl amino, propyl amino (e.g., n-propyl amino and isopropyl amino) etc.

The term "dialkyl amino" refers to a group with the formula -NH (alkyl)₂. Non-limiting examples of dialkyl amino include dimethyl amino, diethyl amino, dipropyl amino (e.g., di (n-propyl) amino and di (isopropyl) amino), etc.

The term "alkoxy" herein refers to the alkyl group linked to the rest of the molecule by oxygen atoms (-O-alkyl), wherein the alkyl is defined herein. Non-limiting examples of alkoxy groups include methoxy, ethoxy, trifluoromethoxy, difluoromethoxy, n-propoxy, isopropoxy, n-butoxy, tert-butoxy, n-pentyloxy, etc.

The term "substituent" in the R⁶ optional group "C₆₋₁₀ aryl with substituents" and the term "substituent" in the R⁷ optional group include non-cyclic and cyclic, branched-chain and unbranched-chain, carboatomic ring and heterocyclic ring, aromatic and non-aromatic substituents of organic compounds. For appropriate organic compounds, the allowable substituents are one or more and may be the same or different. Non-limiting examples of substituents include any of the substituents described herein, e.g., halogens, hydroxyl, carbon group (such as carboxyl, alkoxycarbonyl, formyl, or acyl), thiono (such as thioesters, thioacetate, or thioformate), alkyl, alkenly, alkynyl, alkoxy, haloalkyl, phosphoryl, phosphate, phosphonate, phosphonite, amino, alkyl amino, dialkyl amino, amide, amido, imino, cyano, nitro, azido, sulfydryl, alkylthio, sulfate group, sulfonate group, sulfamido, sulfonamido, sulfonyl, cycloalkyl, heterocyclic, aralkyl, aryl or heteroaryl. Those skilled in the art should understand that the replaced portion of the hydrocarbon chain may itself be substituted as necessary. For example, cycloalkyl may be further substituted by alkyl, alkenyl, alkoxy, alkylthio, aminoalkyl, carbon-substituted alkyl, cyano, etc.; for example, alkenyl and alkynyl may be similarly substituted to produce aminoalkenyl, aminoalkynyl, amidoalkynyl, amidoalkynyl, iminoalkenyl, iminoalkynyl, thioalkenyl, thioalkynyl, carbon-substituted alkenyl or alkynyl.

The term "protective group" in "protective groups (PG) related to hydroxyl, amino, sulfydryl, carboxyl, etc.", refers to the hydroxyl, amino, sulfhydryl, carboxyl, etc. are protected by functional groups to avoid undesired reactions, and the protective groups used are well known to those skilled in the art, such as those in Protective Groups in Organic Synthesis (John Wiley & Sons, New York, Third Edition, 1999).

The term "prevention" means complete or nearly complete prevention of a disease or condition (e.g., infection, ischemia or reperfusion injury), for example when the patient or subject is susceptible to or at risk for a disease or condition; prevention can also include inhibition, i.e. stopping the development of pathologies.

The term "treatment" means: 1) suppression of the disease; for example, the inhibition of the pathology or symptoms of a disease, pathology, or condition of an individual who is experiencing or exhibiting a disease, pathology, or condition (i.e., preventing further development of pathology and/or symptomatology); or 2) improvement of the disease; for example, the improvement of the pathology or symptoms of the disease, pathology, or condition of an individual who is experiencing or exhibiting a disease, pathology, or condition (i.e., reversal pathology and/or symptomatology).

The term "therapeutically effective amount" refers to the amount of active compound or agent sought by a researcher, veterinarian, physician, or other clinician to elicit a biological or medical response in a tissue, system, animal, individual, or person.

The following abbreviations may be used for the present invention: (Boc)₂O (di-tert-butyl dicarbonate); DCM (dichloromethane); tBuOH (tert-butanol); NaBH(OAc)₃ (sodium triacetoxyborohydride); DIPEA (N,N-diisopropylethanamine); DMAP (4-Dimethylaminopyridine); DMF (N,N-dimethylformamide); DMSO (dimethyl sulfoxide); EA or EtOAc (ethyl acetate); HOAc (acetic acid); LCMS or LC-MS (liquid chromatography-mass spectrometry); TLC (thin layer chromatography); MeOH (methanol); NaOMe (sodium methoxide); NaH (sodium hydride); Pd(dcpf)Cl₂ (dichloro[1,1'-bis(dicyclohexylphosphino)ferrocene]palladium(II)); Pd₂(dba)₃ (tris(dibenzylideneacetone) dipalladium); PdCl₂(dppf)CH₂Cl₂ ([1,1'-bis(diphenylphosphino)ferrocene]palladium dichloride dichloromethane complex); Pd(OAc)₂ (palladium(II) acetate); Pd(PPh₃)₄ (tetrakis(triphenylphosphino)palladium_{);} Pd(OH)₂ (palladium hydroxide); DPPF (1,1'-bis(diphenylphosphino)ferrocene); Rt, r.t. or RT (room temperature); h, hr or hrs (hours); min (minutes); BnNH₂ (benzylamine); TEA (triethylamine): TFA (trifluoroacetic acid); BH₃·THF (borane tetrahydrofuran solution); THF (tetrahydrofuran); NaOtBu (sodium tert-butoxide); Cs₂CO₃ (cesium carbonate); BrettPhos (dicyclohexyl[3,6-dimethoxy-2',4',6'-triisopropyl[1,1'-biphenyl]-2-yl]phosphine).

The starting materials of embodiments of the present invention may generally be obtained commercially, for example, purchased from companies such as J&K, Energy Chemical, Aladdin, Bide, etc., or prepared by methods well known to those skilled in the art. Substituents that do not match the reaction conditions are obvious to those skilled in the art, so alternative methods are indicated in the text.

Unless otherwise indicated, the commercial solvents and reagents used in the test are used without further purification or treatment. When referring to other embodiments or synthesis methods, reaction conditions (reaction temperature, reaction solvent, reactant molar ratio or/and reaction duration) may differ. In general, the reaction progress can be monitored by TLC to terminate the reaction and post-process it at an appropriate time. The purification conditions of compounds may also vary, in general, select a suitable column chromatography eluent based on the Rf value of the TLC, or separate and purify the corresponding compound by preparing TLC.

### [General Formula Compound]

The present invention provides a compound having a structure in formula (I) or a pharmaceutically acceptable salt, ester, stereoisomer, tautomer, solvate, chelate, non-covalent complex or prodrug thereof, the structural general formula of the compound in formula (I) is: 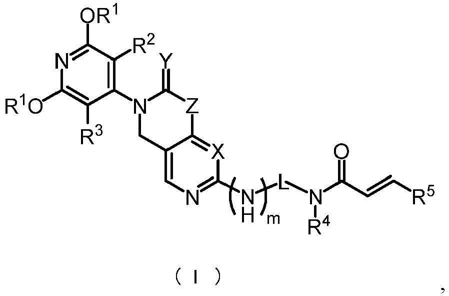 in which,
X is CH or N;
Y is 2 Hs or 1 O;
Z is C(R⁶)₂ or N(R⁶);
m is 0 or 1;
L is -C(R⁷)₂- or 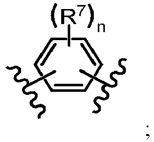
n is 0, 1, or 2;
Each R¹ is independently C₁₋₃ alkyl or C₁₋₃ haloalkyl;
Each R² and R³ are independently hydrogen, halogen, C₁₋₃ alkyl, C₁₋₃ haloalkyl, cyano, or C₁₋₃ alkoxy;
R⁴ is hydrogen, C₁₋₆ alkyl or C₆₋₁₀ aryl;
R⁵ is hydrogen, halogen, cyano, nitro, trifluoromethyl, amino, C₁₋₆ alkyl, C₂₋₈ alkenyl, C₆₋₁₀ aryl, C₁₋₈ alkyl amino, bis (C₂₋₈ alkyl) amino, C₂₋₈ alkynyl, C₁₋₈ haloalkyl or C₃₋₈ cycloalkyl;
Each R⁶ is hydrogen, C₁₋₈ alkyl, C₂₋₈ alkenyl, C₂₋₈ alkynyl, C₁₋₈ haloalkyl, C₆₋₁₀ aryl, C₆₋₁₀ aryl with substituents, C₃₋₁₀ cycloalkyl, 5 to 10 membered heteroaryl or 4 to 10 membered heterocycloalkyl; wherein each 5 to 10 membered heteroaryl or 4 to 10 membered heterocycloalkyl independently contains 1 to 3 cyclic heteroatoms, the heteroatoms are N, O or S independently;
Or two R⁶ and the carbon atoms connected thereto together form a C₃₋₈ cycloalkyl or 4 to 10 membered heterocycloalkyl; wherein the 4 to 10 membered heterocycloalkyl contains 1 to 3 cyclic heteroatoms, the heteroatoms are N, O or S; each C₃₋₈ cycloalkyl and 4 to 10 membered heterocycloalkyl are independently replaced by 1 to 4 substituents, the substituents are each independently halogen, cyano, hydroxyl, amino, C₁₋₈ carboxyamide, C₁₋₈ carboxylacyl, C₁₋₈ alkyl or C₁₋₈ alkoxy;
Each R⁷ is independently hydrogen, halogen, amino, cyano, C₁₋₈ alkyl, C₁₋₈ alkyl with substituent, C₁₋₈ alkoxy, C₁₋₈ alkoxy with substituents, C₁₋₈ alkyl amino, bis (C₂₋₈ alkyl) amino, C₂₋₈ alkenyl, C₂₋₈ alkenyl with substituents, C₂₋₈ alkynyl, C₂₋₈ alkynyl with substituents, C₆₋₁₀ aryl, C₆₋₁₀ aryl with substituents, C₃₋₈ cycloalkyl, C₃₋₈ cycloalkyl with substituents, 3 to 10 membered heterocycloalkyl, 3 to 10 membered heterocycloalkyl with substituents, 5 to 10 membered heteroaryl or 5 to 10 membered heteroaryl with substituents; wherein each 3 to 10 membered heterocycloalkyl and 5 to 10 membered heteroaryl independently contains 1 to 3 cyclic heteroatoms, and the heteroatoms are N, O or S independently.

In some specific embodiments of the present invention, in the compound of formula (I), each R¹ is independently C₁₋₃ alkyl or C₁₋₃ haloalkyl, preferably C₁₋₃ alkyl. Wherein, the C₁₋₃ alkyl is methyl, ethyl or propyl, preferably methyl; C₁₋₃ haloalkyl is -CF₃, -C₂F₅, -CHF₂, -CCl₃, -CHCl₂ or -C₂Cl₅.

In some specific embodiments of the present invention, in the compounds of formula (I), each R² and R³ are independently hydrogen, halogen, C₁₋₃ alkyl, C₁₋₃ haloalkyl, cyano or C₁₋₃ alkoxy, preferably halogen. Wherein the halogen is -F, -Cl or -Br, preferably -F or -Cl; C₁₋₃ alkyl is methyl, ethyl or propyl; C₁₋₃ haloalkyl is -CF₃, -C₂F₅, -CHF₂, -CCl₃, -CHCl₂ or -C₂Cl₅; C₁₋₃ alkoxy is methoxy, ethoxy, n-propoxy or isopropoxy.

In some specific embodiments of the present invention, in the compound of formula (I), R⁴ is hydrogen, C₁₋₆ alkyl or C₆₋₁₀ aryl, preferably H. Wherein C₁₋₆ alkyl is methyl, ethyl or propyl; C₆₋₁₀ aryl is phenyl, 1-naphthyl or 2-naphthyl.

In some specific embodiments of the present invention, in the compound of formula (I), R⁵ is hydrogen, halogen, cyano, nitro, trifluoromethyl, amino, C₁₋₆ alkyl, C₂₋₈ alkenyl, C₆₋₁₀ aryl, C₁₋₈ alkyl amino, bis(C₂₋₈ alkyl) amino, C₂₋₈ alkynyl, C₁₋₈ haloalkyl or C₃₋₈ cycloalkyl, preferably hydrogen. Wherein the halogen is -F, -Cl or -Br; C₁₋₆ alkyl is methyl, ethyl or propyl; C₂₋₈ alkenyl is vinyl, propenyl, allyl or isopropenyl; C₆₋₁₀ aryl is phenyl, 1-naphthyl or 2-naphthyl; C₁₋₈ alkyl amino is methyl amino, ethyl amino or propyl amino; bis(C₂₋₈ alkyl) amino is dimethylamino, diethylamino or dipropyl amino; C₂₋₈ alkynyl is ethynyl, propyn-1-yl, propyn-2-yl; C₁₋₈ haloalkyl is -CF₃, -C₂F₅, -CHF₂, -CCl₃-CHCl₂ or -C₂Cl₅; C₃₋₈ cycloalkyl is cyclopropyl, cyclobutyl, cyclopentyl or cyclopentenyl.

In some specific embodiments of the present invention, in the compound of formula (I), each R⁶ is independently hydrogen, C₁₋₈ alkyl, C₂₋₈ alkenyl, C₂₋₈ alkynyl C₁₋₈ haloalkyl, C₆₋₁₀ aryl, C₆₋₁₀ aryl containing substituents, C₃₋₁₀ cycloalkyl, 5 to 10 membered heteroaryl or 4 to 10 membered heterocycloalkyl, preferably hydrogen, C₁₋₈ alkyl, C₆₋₁₀ aryl, or C₃₋₁₀ cycloalkyl. Wherein C₁₋₈ alkyl is methyl, ethyl or propyl, preferably methyl or ethyl; C₂₋₈ alkenyl is vinyl, propenyl, allyl or isopropenyl; C₂₋₈ alkynyl is ethynyl, propyn-1-yl or propyn-2-yl; C₁₋₈ haloalkyl is -CF₃, -C₂F₅, -CHF₂, -CCl₃, -CHCl₂ or -C₂Cl₅; C₆₋₁₀ aryl is phenyl, 1-naphthyl or 2-naphthyl, preferably phenyl; C₆₋₁₀ aryl containing substituents is substituted phenyl, 1-naphthyl or 2-naphthyl, the substituents of which can be halogens, hydroxyl, carbon group (e.g., carboxy, alkoxycarbon, formyl, or acyl), thiono (e.g., thioester, thioacetate, or thioformate), alkyl, alkenly, alkynyl, alkoxy, haloalkyl, phosphoryl, phosphate, phosphonate, phosphonite, amino, alkyl amino, dialkyl amino, amide, amido, imino, cyano, nitro, azido, sulfydryl, alkylthio, sulfate group, sulfonate group, sulfamido, sulfonamido, sulfonyl, cycloalkyl, heterocyclic, aralkyl, aryl or heteroaryl; C₃₋₁₀ cycloalkyl is cyclopropyl, cyclobutyl or cyclopentyl, preferably cyclopropyl; 5 to 10 membered heteroaryl is pyridinyl, pyrimidine, pyrazinyl, pyridazinyl, triazinyl, furyl, thienyl, imidazolyl, thiazolyl, indolyl, pyrryl, or oxazolyl; 4 to 10 membered heterocycloalkyl is pyrrolinyl, piperidyl, piperazinyl, tetrahydrofuranyl, tetrahydropyranyl, morpholinyl, pyranyl, morpholine N-oxide, thiomorpholine S-oxide, thiomorpholine S, S-dioxide, phthalimido, piperidinone, oxazolidinone, 2,4-(1H,3H)-dioxo-pyrimidine or pyridine-2(1H)-keto.

In some embodiments of the present invention, in the compound of formula (I) above, two R⁶ and the carbon atoms and their linked carbon atoms together form a C₃₋₈ cycloalkyl or a 4 to 10 membered heterocycloalkyl, preferably a C₃₋₈ cycloalkyl. Wherein, 4 to 10 membered heterocycloalkyl contains 1 to 3 cyclic heteroatoms, and the heteroatoms are N, O or S. C₃₋₈ cycloalkyl and 4 to 10 membered heterocycloalkyl are independently replaced by 1 to 4 substituents, each independently of halogen, cyano, hydroxyl, amino, C₁₋₈ carboxyamido, C₁₋₈ carboxylacyl, C₁₋₈ alkyl or C₁₋₈ alkoxy; wherein, C₃₋₈ cycloalkyl is cyclopropyl, cyclobutyl or cyclopentyl, preferably cyclopropyl; 4 to 10 membered heterocycloalkyl is pyrrolinyl, piperidyl, piperazinyl, tetrahydrofuranyl, tetrahydropyranyl, morpholinyl, pyranyl, morpholine N-oxide, thiomorpholine S-oxide, thiomorpholine S, S-dioxide, phthalimido, piperidinone, oxazolidinone, 2,4-(1H,3H)-dioxo-pyrimidine or pyridine-2(1H)-keto; C₁₋₈ carboxyamide is formamide or acetamide; C₁₋₈ carboxyacyl is formyl or acetyl; C₁₋₈ alkyl is methyl, ethyl or propyl; C₁₋₈ alkoxy is methoxy, ethoxy, trifluoromethoxy, difluoromethoxy, n-propoxy, isopropoxy, n-butoxy, tert-butoxy or n-pentyloxy.

In some embodiments of the present invention, each R⁷ in the compound of formula (I) above is hydrogen, halogen, amino, cyano, C₁₋₈ alkyl, C₁₋₈ alkyl with substituents, C₁₋₈ alkoxy, C₁₋₈ alkoxy with substituents, C₁₋₈ alkyl amino, bis(C₂₋₈ alkyl) amino, C₂₋₈ alkenyl, C₂₋₈ alkenyl with substituents, C₂₋₈ alkynyl, C₂₋₈ alkynyl with substituents, C₆₋₁₀ aryl, C₆₋₁₀ aryl with substituents, C₃₋₈ cycloalkyl, C₃₋₈ cycloalkyl with substituents, 3 to 10 membered heterocycloalkyl group, 3 to 10 membered heterocycloalkyl group containing substituents, 5 to 10 membered heteroaryl group or 5 to 10 membered heteroaryl group containing substituents, preferably hydrogen, halogens, C₁₋₈ alkyl, C₆₋₁₀ aryl, C₆₋₁₀ aryl with substituents, 3 to 10 membered heterocycloalkyl group or 3 to 10 membered heterocycloalkyl group with substituents, more preferably hydrogen, C₆₋₁₀ aryl, C₆₋₁₀ aryl with substituents, 3 to 10 membered heterocycloalkyl group or 3 to 10 membered heterocycloalkyl group with substituents. Wherein the halogen is -F, -Cl or -Br; C₁₋₈ alkyl is methyl, ethyl or propyl; C₁₋₈ alkoxy group is methoxy, ethoxy, n-propoxy, isopropoxy, n-butoxy, tert-butoxy or n-pentyl; C₁₋₈ alkyl amino group is methyl amino, ethyl amino or propyl amino; Bis(C₂₋₈ alkyl) amino group is dimethylamino, diethylamino or dipropyl amino; C₂₋₈ alkenyl is vinyl, propenyl, allyl or isopropenyl; C₂₋₈ alkynyl is ethynyl, propyne-1-yl or propyne-2-yl; C₆₋₁₀ aryl is phenyl, 1-naphthyl or 2-naphthyl; C₃₋₈ cycloalkyl is cyclopropyl, cyclobutyl, cyclopentyl or cyclopentenyl; 3 to 10 membered heterocycloalkyl group is pyrroline, piperidine, piperazine, tetrahydrofuranyl, tetrahydropyranyl, morpholino or pyranol; 5 to 10 membered heteroaryl group is pyridinyl, pyrimidine, pyrazinyl, pyridazine, triazinyl, furanol, thienyl, imidazole, thiazole, indolyl, pyrro group or oxazole group; Among them, C₁₋₈ alkyl containing substituents, C ₁₋₈ alkenyl containing substituents, C₂₋₈ alkenyl with substituents, C₂₋₈ alkynyl with substituents, C₆₋₁₀ aryl with substituents, C₃₋₈ cycloalkyl with substituents, 3 to 10 membered heterocycloalkyl group containing substituents and the 5 to 10 membered heteroaryl group containing substituents are respectively substituted by the substituent for the above C₁₋₈ alkyl and C₁₋₈ alkoxy, C₂₋₈ alkenyl, C₂₋₈ alkynyl, C₆₋₁₀ aryl, C₃₋₈ cycloalkyl, 3 to 10 membered heterocycloalkyl group and 5 to 10 membered heteroaryl group, the substituents can be halogens, hydroxyl, carbon group (such as carboxyl, alkoxycarbonyl, formyl, or acyl), thiono (such as thioesters, thioacetate, or thioformate), alkyl, alkenly, alkynyl, alkoxy, haloalkyl, phosphoryl, phosphate, phosphonate, phosphonite, amino, alkyl amino, dialkyl amino, amide, amido, imino, cyano, nitro, azido, sulfydryl, alkylthio, sulfate group, sulfonate group, sulfamido, sulfonamido, sulfonyl, cycloalkyl, heterocyclic, aralkyl, aryl or heteroaryl, preferably alkyl or heterocyclic.

In some specific embodiments of the present invention, the compound of formula (I) has the structure shown in formula (II):

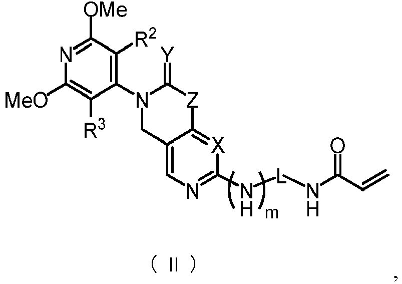

Where X, Y, Z, m, L, R2 and R3 are as defined in formula (I).

In some specific embodiments of the present invention, the compound of formula (II) has the structure shown in formula (III):

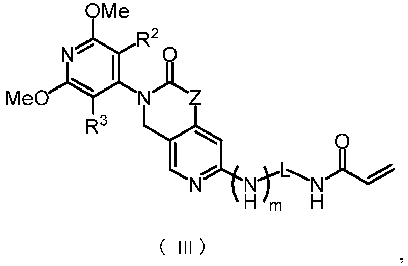

Where Z, m, L, R2 and R3 are as defined in formula (II).

In some specific embodiments of the present invention, the compound of formula (III) has the structure shown in formula (III-1):

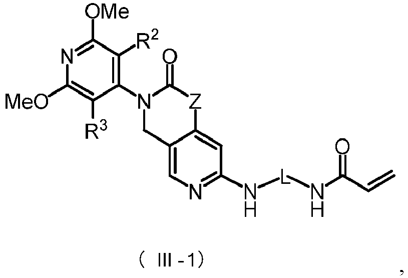

Where Z, L, R2 and R3 are as defined in formula (III).

In some specific embodiments of the present invention, the compound of formula (III-1) has the structure shown in formula (III-1-1):

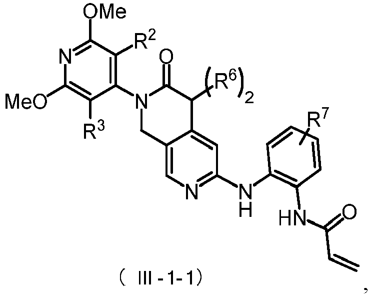

Where R2, R3 and R6 are as defined in formula (III-1), R7 is hydrogen, C₁₋₄ alkyl, piperazinyl, piperidinyl or morpholinoyl, wherein each C1-4 alkyl, piperazinyl, piperidinyl and morpholinoyl are optionally replaced by at least one R⁸. Each R⁸ is independently hydrogen, C₁₋₄ alkyl (preferably methyl or ethyl), morpholinyl, bridged-ring morpholinyl, piperazinyl, piperazinyl with substituents, bridge-ring piperazinyl, bridged-ring piperazinyl with substituents, oxetalkyl or oxetalkyl with the substituents, the substituent is C₁₋₄ alkyl (preferably methyl or ethyl).

In some specific embodiments of the present invention, in the compounds of formula (III-1-1), R2, R3 and R6 are as defined in formula (III-1). R⁷ is hydrogen, C1-4 alkyl, piperazinyl, piperidinyl or morpholinoyl, wherein each C₁₋₄ alkyl, piperazinyl, piperidinyl and morpholinoyl are optionally replaced by at least one R⁸. Each R⁸ is independently hydrogen, morpholinyl, bridged-ring morpholinyl, piperazinyl, piperazinyl with substituents, bridge-ring piperazinyl, bridged-ring piperazinyl with substituents, oxetalkyl or oxetalkyl with substituents, the substituent is C₁₋₄ alkyl.

In some specific embodiments of the present invention, in the compounds of formula (III-1-1), R2, R3 and R6 are as defined in the formula (III-1), R7 is one of the following fragments:

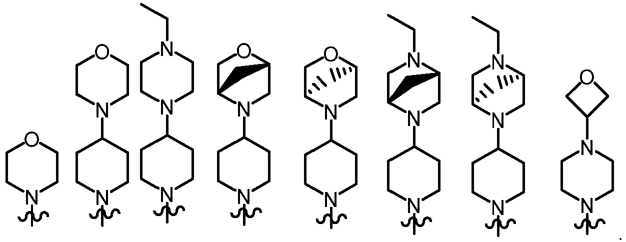

In some specific embodiments of the present invention, the compound of formula (III) has the structure shown in formula (III-2):

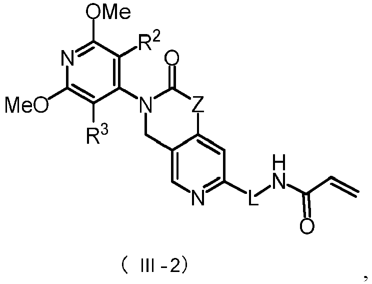

Where Z, L, R2 and R3 are as defined in formula (III).

In some specific embodiments of the present invention, the compound of formula (III-2) has the structure shown in formula (III-2-1):

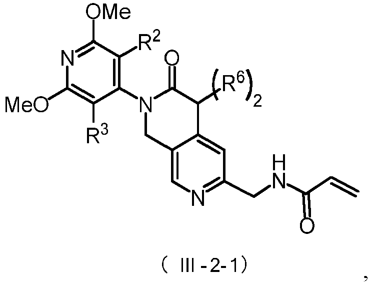

Where, R3 and R6 are as defined in formula (III-2).

In some specific embodiments of the present invention, the compound of formula (III-2) has the structure shown in formula (III-2-2):

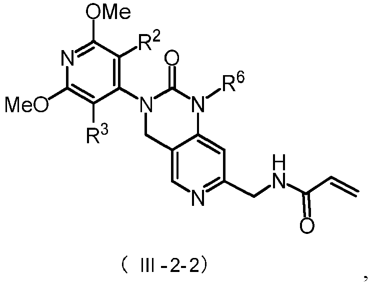

Where, R3 and R6 are as defined in formula (III-2).

In some specific embodiments of the present invention, the compound of formula (II) has the structure shown in formula (II):

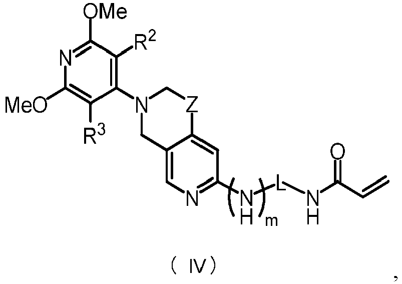

Where Z, m, L, R2 and R3 are as defined in formula (II).

In some specific embodiments of the present invention, the compound of formula (IV) has the structure shown in formula (IV-1):

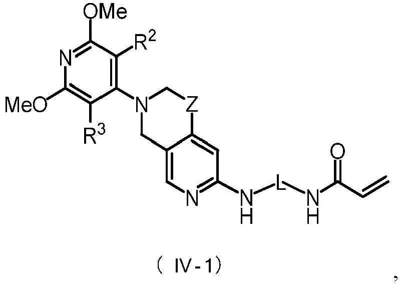

Where Z, L, R2 and R3 are as defined in formula (IV).

In some specific embodiments of the present invention, the compound of formula (IV-1) has the structure shown in formula (IV-1-1):

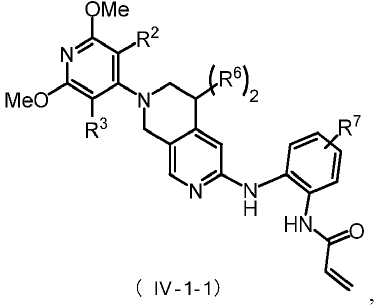

Where R2, R3 and R6 are as defined in formula (IV-1), R⁷ is hydrogen, C1-4 alkyl, piperazinyl, piperidinyl or morpholinoyl, wherein each C₁₋₄ alkyl, piperazinyl, piperidinyl and morpholinoyl are optionally replaced by at least one R⁸. Each R⁸ is independently hydrogen, morpholinyl, bridged-ring morpholinyl, piperazinyl, piperazinyl with substituents, bridge-ring piperazinyl, bridged-ring piperazinyl with substituents, oxetalkyl or oxetalkyl with substituents, the substituent is C₁₋₄ alkyl.

In some specific embodiments of the present invention, in the compounds of formula (IV-1-1), R2, R3 and R6 are as defined in the formula (IV-1), R7 is one of the following fragments:

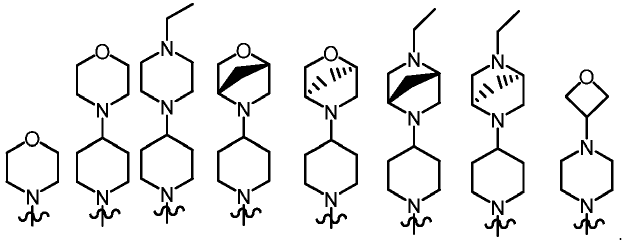

In some specific embodiments of the present invention, the compound of formula (IV) has the structure shown in formula (IV-2):

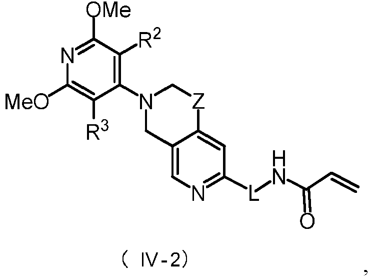

Where Z, L, R2 and R3 are as defined in formula (IV).

In some specific embodiments of the present invention, the compound of formula (IV)-2 has the structure shown in formula (IV-2-1):

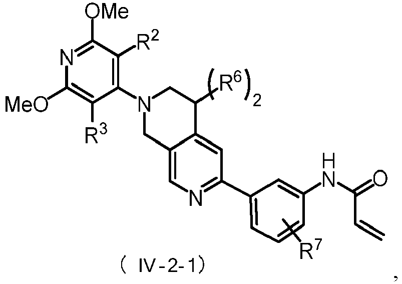

Where, R2,R3 and R6 are as defined in formula (IV-2), R7 is hydrogen, C1-4 alkyl or

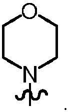

In some specific embodiments of the present invention, the compound of formula (IV)-2 has the structure shown in formula (IV-2-2):

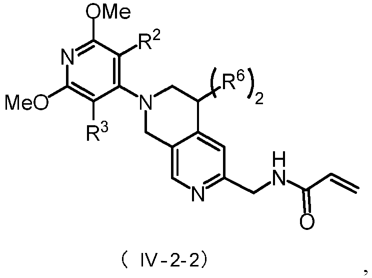

Where, R², R³ and R⁶ are as defined in formula (IV-2).

In some more preferred examples of the invention, the compound is any of the following: 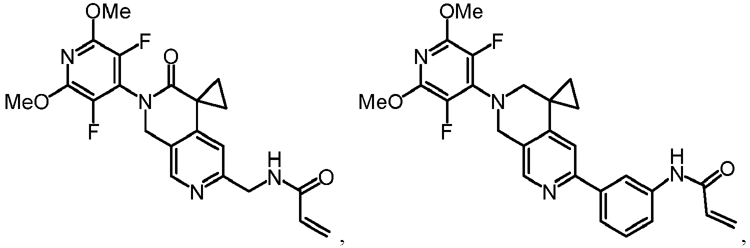 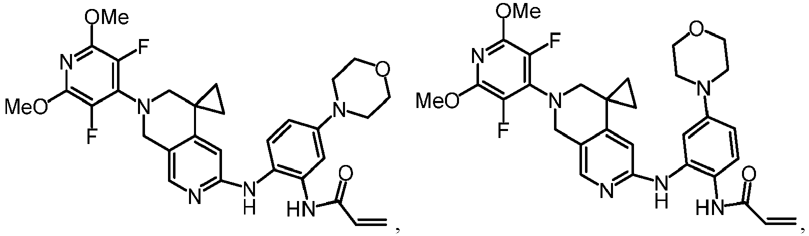 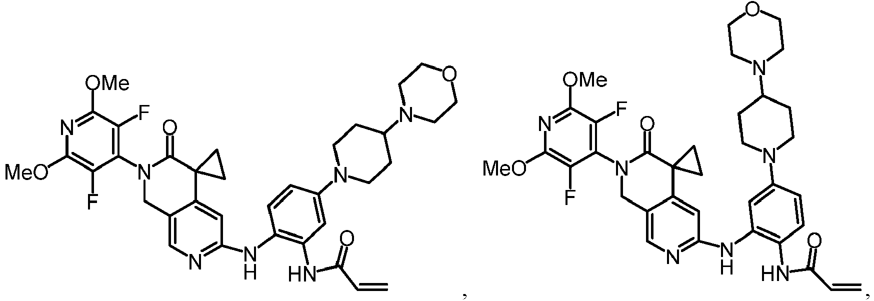 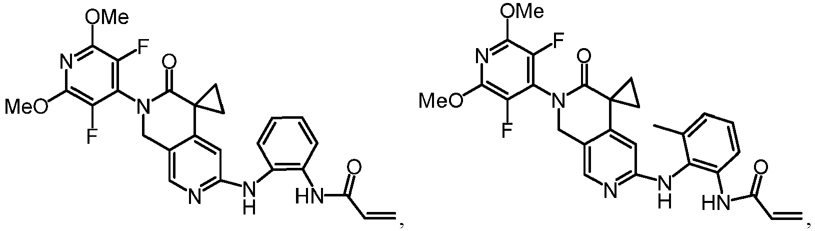 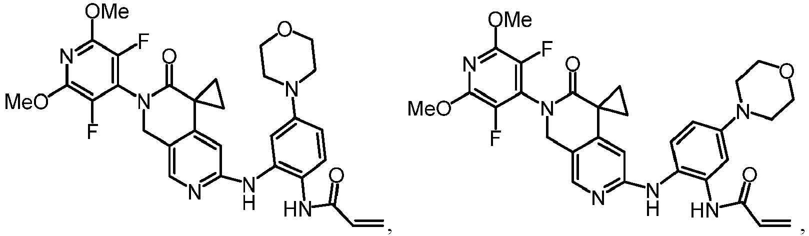 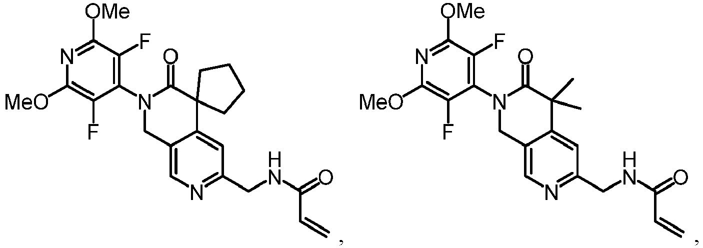 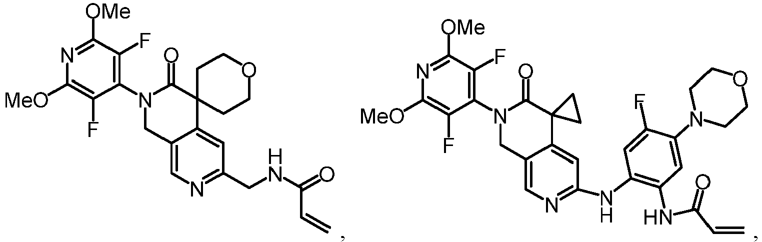 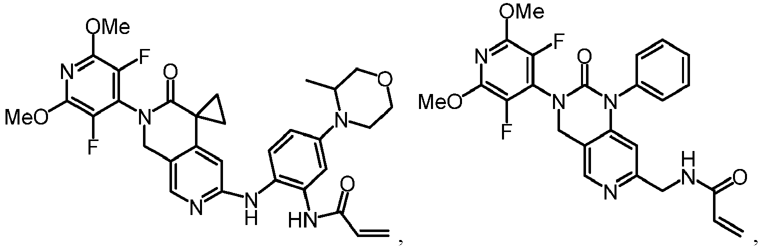 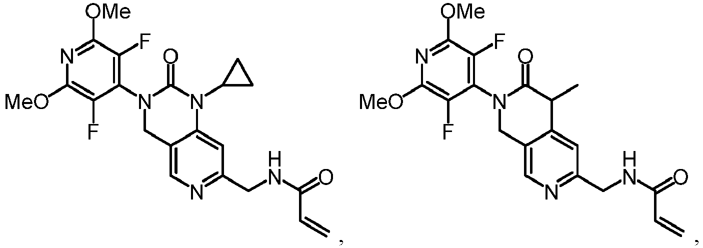 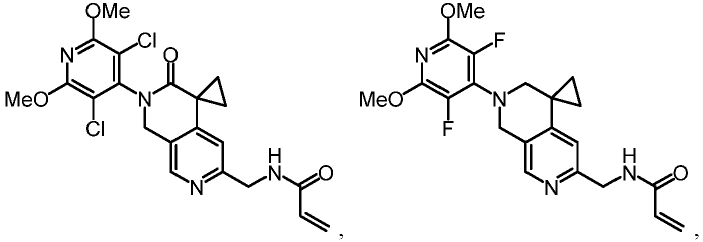 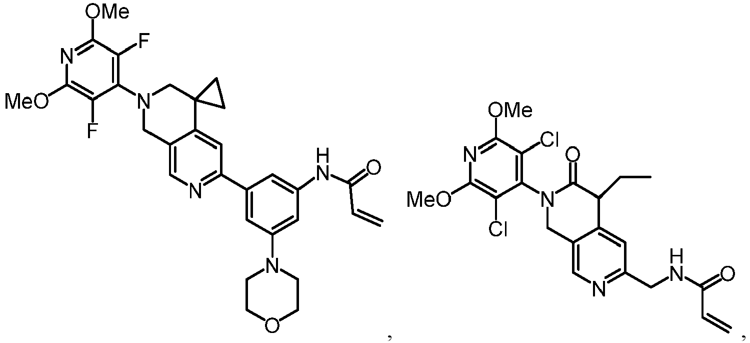 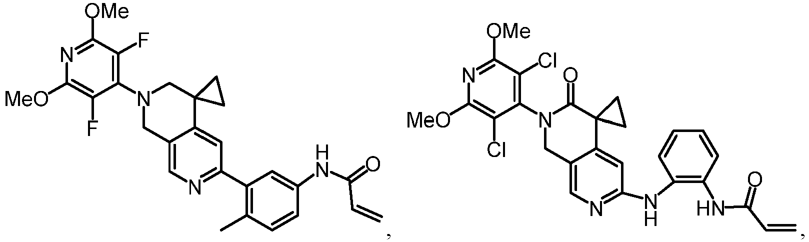 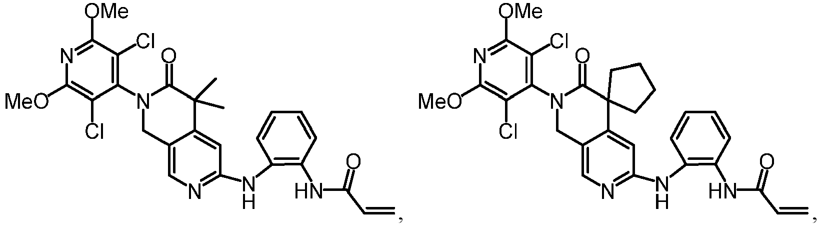 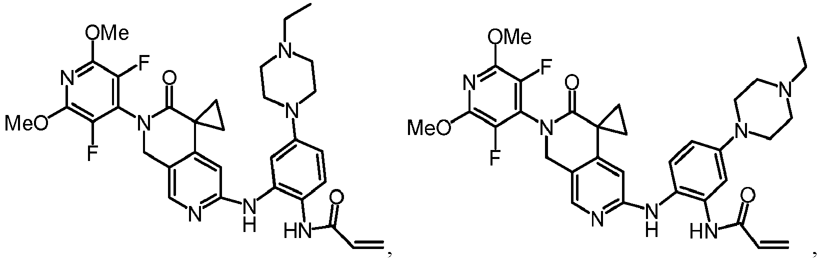 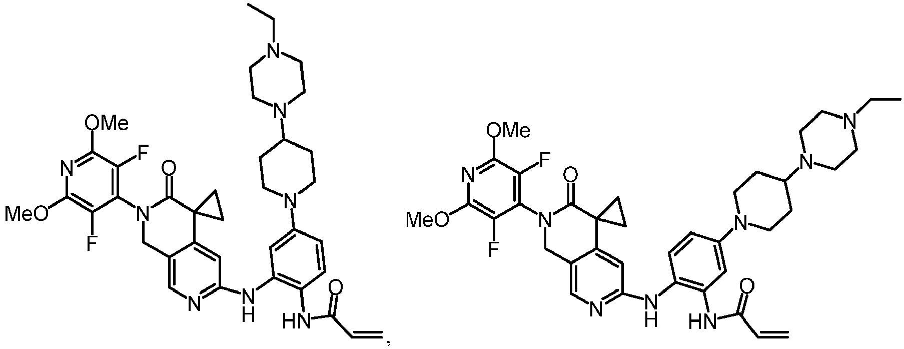 and

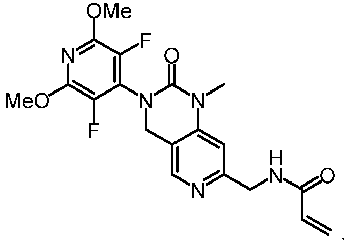

### [Pharmaceutical Composition]

The present invention also provides a pharmaceutical composition comprising the above compound or pharmaceutically acceptable salts, esters, stereoisomers, tautomers, solvates, chelates, non-covalent complexes or prodrugs thereof.

In some embodiments of the present invention, the pharmaceutical composition mentioned above further comprises a pharmaceutically acceptable carrier or diluent.

In some preferred embodiments of the present invention, the pharmaceutical composition mentioned above further comprises:
- Pharmaceutically acceptable carriers; and/or
- Excipient.

The term "pharmaceutically acceptable carrier" refers to pharmaceutical excipients that are compatible with the active ingredient of the drug and are harmless to the subject, including (but not limited to) diluents (or filling agent), adhesives, disintegrants, lubricants, wetting agents, thickeners, flow aids, flavor correctants, olfactory agents, preservatives, antioxidants, pH adjusters, solvents, co-solvents and surfactants.

Some embodiments of suitable excipients include lactose, dextrose, sucrose, sorbitol, mannitol, starch, gum arabic, calcium phosphate, alginate, astragalus gum, gelatin, calcium silicate, microcrystalline cellulose, polyvinyl pyrrolidone, cellulose, water, syrup, and methylcellulose.

### [Pharmaceutical Preparation]

The present invention also provides a pharmaceutical preparation comprising the above compound or pharmaceutically acceptable salts, esters, stereoisomers, tautomers, solvates, chelates, non-covalent complexes or prodrugs thereof.

In some embodiments of the present invention, the pharmaceutical preparation is any of tablets, capsules, injections, granules, powders, suppositories, pills, gels, pulvis, oral solutions, inhalants, suspensions or dry suspensions.

### [Medicinal Use]

Whether the above compounds or their pharmaceutically acceptable salts, esters, stereoisomers, tautomers, solvated compounds, chelates, non-covalent complexes or prodrugs thereof, or pharmaceutical compositions, or pharmaceutical preparations, exhibit inhibition of FGFR4 activity, therefore, the present invention provides the above compounds or pharmaceutically acceptable salts, stereoisomers, tautomers, solvates and chelators, non-covalent complexes or prodrugs thereof, or the pharmaceutical composition described above, or the pharmaceutical preparation described above, which is used as an FGFR4 inhibitor.

The present invention also provides a use of the above compounds or pharmaceutically acceptable salts, esters, stereoisomers, tautomers, solvates, chelates, non-covalent complexes or prodrugs thereof or pharmaceutical compositions described above, or pharmaceutical preparations described above in the preparation of drugs for preventing and/or treating the disease at least partially mediated by FGFR4..

In some embodiments of the present invention, the disease at least partially mediated by FGFR4 includes cancer.

In some embodiments of the present invention, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

### [Treatment]

The present invention also provides a method for preventing and/or treating the disease at least partially mediated by FGFR4, which comprises the following steps: the prevention / or treatment of effective amounts of the above compounds or their pharmaceutically acceptable salts, esters, stereoisomers, tautomers, solvates, chelates, non-covalent complexes or prodrugs thereof, or the above pharmaceutical compositions, or the above pharmaceutical preparations administered to patients in need.

The amount of compound, pharmaceutical composition or pharmaceutical preparation administered to a patient will vary depending on the drug administered, the purpose of the administration (eg, prevention or treatment), the state of the patient, the mode of administration, etc. In therapeutic application, compositions may be administered to patients who already suffer from the disease in an amount sufficient to cure or at least partially suppress the symptoms and complications of the disease. The effective amount of treatment will depend on the condition of the disease being treated and the judgment of the attending clinician depends on factors such as the severity of the disease, the patient's age, weight, and general condition.

The present invention also provides a method for preventing and / or treating cancer, which comprises the following steps: the prevention / or treatment of effective amounts of the above compounds or their pharmaceutically acceptable salts, esters, stereoisomers, tautomers, solvates, chelates, non-covalent complexes or prodrugs thereof, or the above pharmaceutical compositions, or the above pharmaceutical preparations and at least one additional cancer therapeutic agent administered to patients in need.

In some embodiments of the present invention, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

### [Drug Combination Form]

The present invention provides a pharmaceutical combination form comprising the above compounds or their pharmaceutically acceptable salts, esters, stereoisomers, tautomers, solvated compounds, chelators, non-covalent complexes or prodrugs thereof, or pharmaceutical compositions comprising the above compounds, or pharmaceutical preparations comprising the above compounds, and at least one additional cancer therapeutic agent.

### [Preparation Method]

The present invention provides a method for preparing salts, esters, stereoisomers, tautomers, solvates, chelates or non-covalent complexes or prodrugs thereof of the above compounds or their pharmaceutically acceptable substances, and the technical protocol of the invention is further described below by describing a typical synthetic route of the compound of general formula (I).

### Intermediate A5 Preparation Protocol

Intermediate **A5** can be prepared by the following protocol, and the specific synthesis route is as follows:

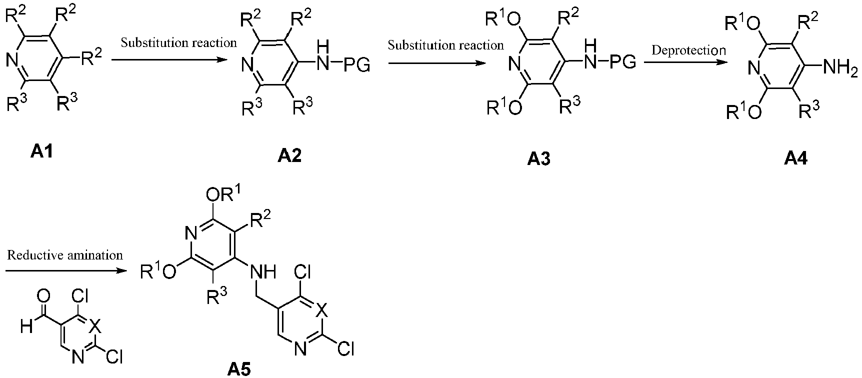

In the above protocol, raw material **A1** reacts with primary amine with protective group to obtain intermediate **A2,** which is replaced by alkoxy group to obtain intermediate A3, and intermediate **A3** is deprotected as an intermediate **A4,** which reacts **with** 4,6-dichloronicotinaldehyde (X is CH) or 2,4-dichloro-5-formylpyrimidine (X is N) to obtain intermediate **A5.** Among them, the starting material **A1** is obtained commercially.

### Intermediate A8 Preparation Protocol

The synthesis route of intermediate A8 is as follows:

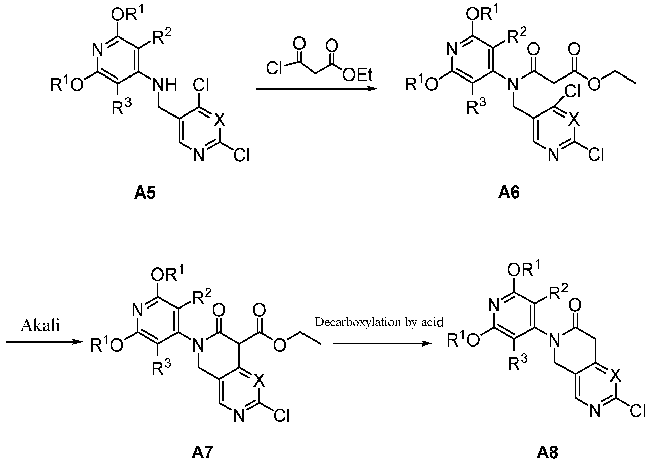

Intermediate **A6** can be prepared by treating intermediate A5 with ethylmalonyl chloride under alkaline conditions. Under strong alkaline conditions, intermediate **A6** will form a loop within the molecule to obtain intermediate A7 followed by acid (such as HCl)-mediated decarboxylation to prepare intermediate **A8.**

### Compound A13 Preparation Protocol

The synthesis route of compound **A13** is as follows:

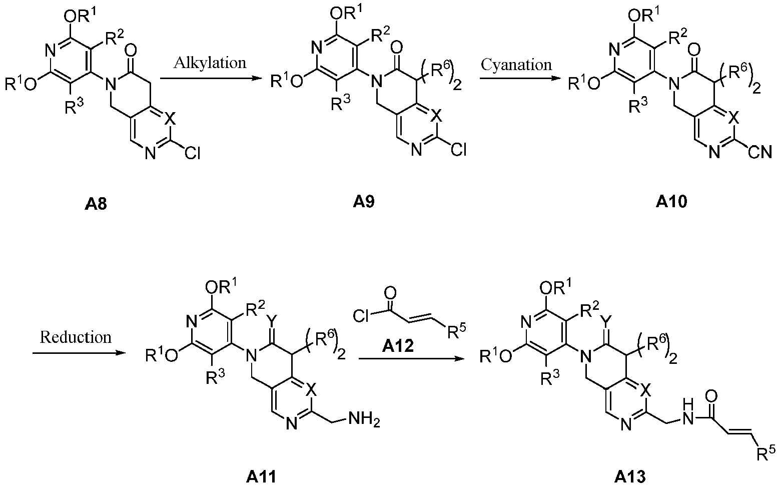

Under alkaline conditions, intermediate A9 containing R6 can be obtained by the reaction between intermediate A8 with R6 X (X is a halogenated group, such as Cl, Br or I). (Determine whether alkylation reaction is required based on the synthesis needs. If it is not required, proceed directly to the next step of reaction). Under the condition that Zn(CN)₂ is used as the reaction reagent and palladium is used as the catalyst, intermediate A9 is converted to intermediate A10. The cyano in intermediate A10 can be reduced under the conditions of nickel chloride hexahydrate/NaBH4 to obtain intermediate **A11** in which Y is an oxygen atom, or when the intermediate A10 is treated with BH₃·THF, the carbonyl group and the cyano are simultaneously reduced to obtain the intermediate A11 in which Y represents two hydrogen atoms. Intermediate **A11** is reacted with acid chloride A12 at low temperature to obtain compoundA13.

### Compound A18 Preparation Protocol

The synthesis route of compound **A18** is as follows:

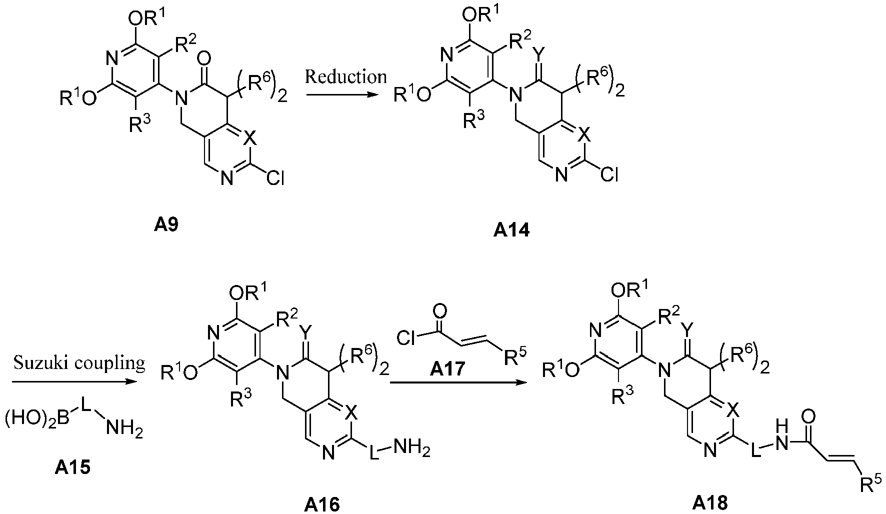

Treat compound A9 with BH₃·THF to obtain intermediate A14 (Determine whether a reduction reaction is required according to the synthesis needs, and if no reduction reaction is required, proceed directly to the next step of the reaction). The intermediate **A14** and intermediate **A15** are coupled with [1,1'-bis(diphenylphosphino)ferrocene]palladium dichloride as catalysts to obtain the intermediate **A16,** which reacts with acyl chloride **A17** at low temperatures to obtain compound **A18.**

### Compound A25 Preparation Protocol

The synthesis route of compound **A25** is as follows:

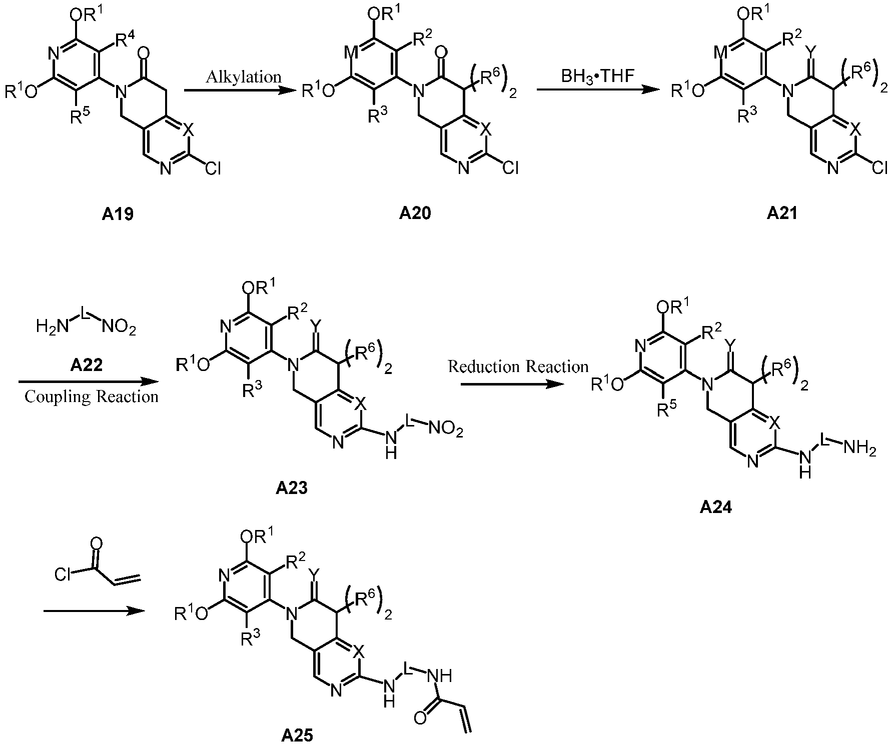

Under alkaline conditions, the compound A20 containing R⁶ can be obtained by adding R⁶X in the solution A19 (X is a halogenated group, such as Cl, Br or IX). (Determine whether alkylation reaction is required based on the synthesis needs. If it is not required, proceed directly to the next step of reaction). When using BH₃ THF as a reducing agent, the carbon group of A20 can be reduced to methylene to obtain compound A21 (if the reduction reaction is not required, skip this step and proceed directly to the next reaction). Compound **A21** is coupled with **A22** to obtain A23. The nitryl in compound A23 can be reduced to obtain A24. At low temperature, compound A25 can be successfully obtained by dropping acid chloride to the solution of A24.

### Compound A30 Preparation Protocol

The synthesis route of compound **A30** is as follows:

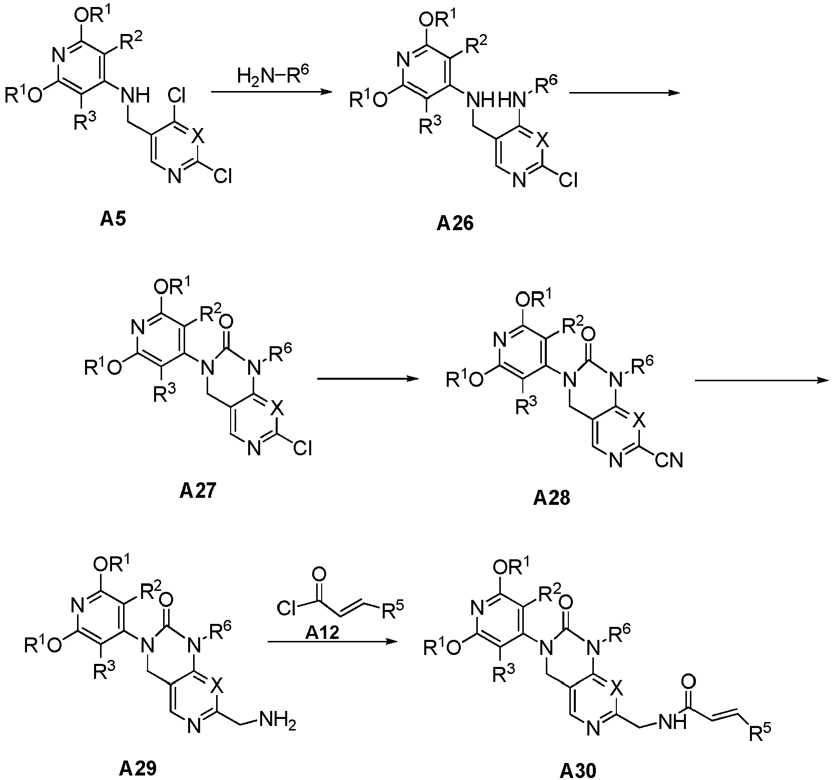

Under the condition of palladium as a catalyst, **A5** is coupled with an amine to obtain compound **A26.** Compound **A26** is formed in the molecule under the action of triphosgene to obtain compound **A27.** Under the condition that Zn(CN)₂ is used as the reaction reagent and palladium is used as the catalyst, intermediate A27 is converted to intermediate A28. Intermediate **A28** is reduced to intermediate **A29,** which reacts with acyl chloride **A12** at low temperature to obtain compound **A30.**

### Example 1: Synthesis of N-((2'-(3,5-difluoro-2,6-dimethoxypyridine-4-yl)-3'-oxo-2',3'-dihydro-1'H-spiro[cyclopropane-1,4'-[2,7]naphthine]-6'-yl) methyl) acrylamide (compound 1)

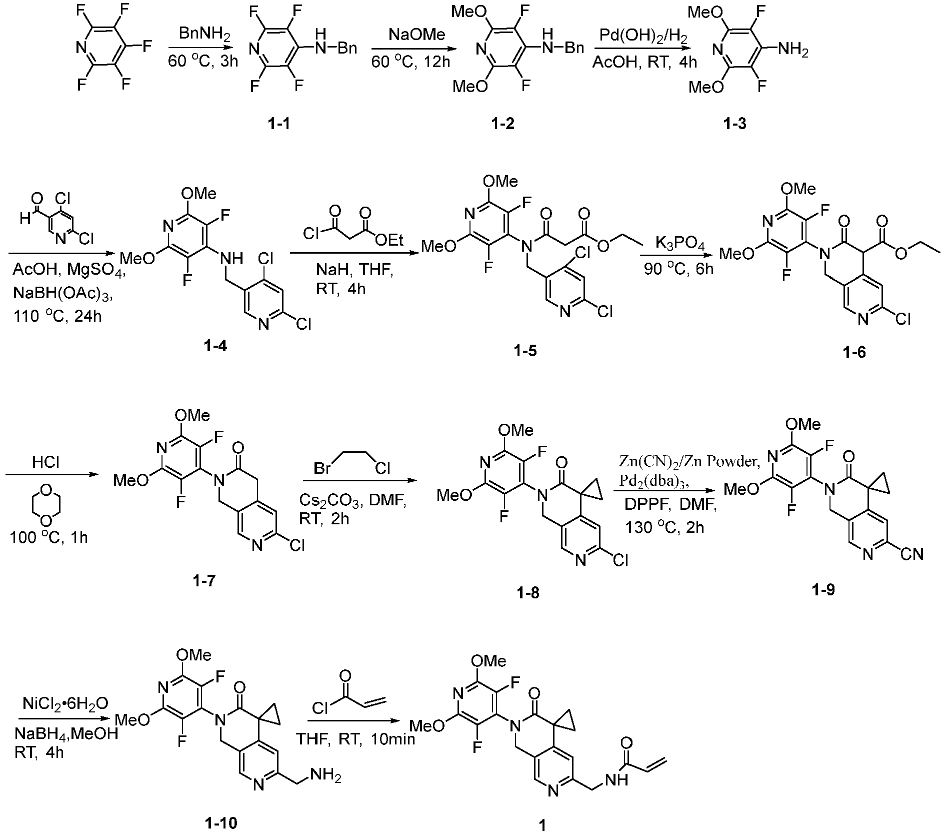

### Step 1: Synthesis of intermediate 1-1

Add pentafluoropyridine (3.6 g, 21.30 mmol) to acetonitrile (150 mL) and stir at room temperature, then add benzylamine (4.5 g, 42.00 mmol), stir continually for 1 h at room temperature and heat to 60 °C for the reaction. After 3 hours, cool the reactants to room temperature for vacuum concentration. Add DCM (100mL) and water (100mL) to the concentrate, stir to dissolve, stratify, and collect the organic phase. Wash the organic phase once with saturated NaCl solution (100mL), dry and concentrate with anhydrous Na₂SO4 to obtain a white solid product (5.3g, 20.69mmol) with a yield of 97.1%.

Identification data for specific compounds are as follows:
LC-MS: m/z 257.1 [M+H]⁺ (calculated value 257.1, C₁₂H₈F₄N₂).

### Step 2: Synthesis of intermediate 1-2

Add sodium methoxide (28.5 g, 527.54 mmol) to anhydrous methanol (250 mL) and stir at room temperature, then add intermediate 1-1 (9.0g, 35.13mmol), and heat the obtained mixture to 60 °C for reaction. After 12 hours, cool the reactants to room temperature, quench with glacial acetic acid (31.6g, 526.23mmol), concentrate the reaction solution, add water (600mL) and DCM (300mL) to the residue, stir to dissolve, and stratify, collect the organic phase, extract the aqueous phase once with DCM (300mL), and combne the organic phase. Wash with water (3× 300 mL), dry and concentrate with anhydrous Na₂SO₄ to obtain the desired product (9.5 g, 33.89 mmol) with a yield of 96.5%.

Identification data for specific compounds are as follows:
LC-MS: m/z 281.1 [M+H]⁺ (calculated value 281.1, C₁₄H₁₄F₂N₂O₂).

### Step 3: Synthesis of intermediate 1-3

Add intermediate 1-2 (3.0 g, 10.71 mmol) to acetic acid (60 mL) and stir at room temperature, then add Pd(OH)₂ (0.6 g, 4.28 mmol), replace with H₂ three times, protect with H₂ balloon, stir at room temperature for 4 h. Filter the reaction solution and concentrate to obtain acetate (2.1g, 8.60mmol) of the desired product with a yield of 80.3%.

Identification data for specific compounds are as follows:
LC-MS: m/z 191.1 [M+H]⁺ (calculated value 191.1, C₇H₈F₂N₂O₂).

### Step 4: Synthesis of intermediate 1-4

Add acetate (10.6 g, 42.37 mmol) and 4,6-dichloronicotinaldehyde (6.0 g, 34.09 mmol) of intermediate 1-3 to toluene (70 mL) and stir at room temperature, then add glacial acetic acid (3 mL) and anhydrous magnesium sulfate (24.0 g, 199.40 mmol), heat the obtained mixture to 110 °C for reaction. After 24 hours, cool the reactants to room temperature and filter, vacuum concentrate the filtrate. Add DCM (70mL) to the obtained concentrate, stir to dissolve, and add trifluoroacetic acid (11.7g, 102.61mmol) to cool down to - 5~0 °C. Slowly add sodium triacetoxyborohydride (26.5 g, 125.04 mmol) to the reaction solution. After 4 hours of stirring at room temperature, quench the reaction solution with saturated NH₄Cl aqueous solution, and extract with DCM (3×100mL), combine the organic layers, and dry and concentrate with Na₂SO₄. Purify the residue on silica gel (elute with hexane containing 0-5% (v/v) EtOAc) to obtain the desired product (10.3g, 29.42mmol) with a yield of 69.4%.

Identification data for specific compounds are as follows:
LC-MS: m/z 349.9 [M+H]⁺ (calculated value 350.0, C₁₃H₁₁Cl₂F₂N₃O₂).

### Step 5: Synthesis of intermediate 1-5

Add intermediate 1-4 (5.0 g, 14.28 mmol) to tetrahydrofuran (25 mL) and stir at room temperature, then add NaH (60% w/w in mineral oil, 600.0mg, 15.0 mmol). After 10 min, add ethylmalonyl chloride (2.64 mL, 18.68 mmol) dropwise. After adding dropwise and reacting for 4 hours, quench the reaction with saturated NH₄Cl aqueous solution, and extract with EA (2×100mL), combine the organic layer, and dry and concentrate with Na₂SO_{4.} Purify the residue on silica gel (elute with hexane containing 0-20% (v/v) EtOAc) to obtain the desired product (5.7g, 12.28mmol) with a yield of 86.0%.

Identification data for specific compounds are as follows:
LC-MS: m/z 463.9 [M+H]⁺ (calculated value 464.1, C₁₈H₁₇Cl₂F₂N₃O₅).

### Step 6: Synthesis of intermediate 1-6

Add intermediate 1-5 (3.0g, 6.46mmol) to DMF (100mL) to dissolve at room temperature, then add potassium phosphate (9.0g, 42.40mmol), and heat the obtained mixture to 90 °C. After 6 h, cool the reactants to room temperature, quench the reaction by adding 2% aqueous acetic acid solution (100 mL), and extract with EA (2×100 mL), combine the organic layers, dry and concentrate with Na₂SO₄. Purify the residue on silica gel (elute with hexane containing 0-20% (v/v) EtOAc) to obtain the desired product (1.5g, 3.51mmol) with a yield of 54.3%.

Identification data for specific compounds are as follows:
LC-MS: m/z 428.0 [M+H]⁺(calculated value 428.1, C₁₈H₁₆ClF₂N₃O₅).

### Step 7: Synthesis of intermediate 1-7

Add intermediate 1-6 (120mg, 0.28mmol) to 1,4-dioxane (6mL) and stir at room temperature, then add concentrated hydrochloric acid (4mL) and heat the obtained mixture to 100 °C. After 1 h, cool the reactants to room temperature, quench with saturated NaHCOs aqueous solution, and extract with EA (2×100 mL), combine the organic layers, dry and concentrate with Na₂SO₄. Purify the residue on silica gel (elute with hexane containing 0-25% (v/v) EtOAc) to obtain the desired product (62.3mg, 0.18mmol) with a yield of 62.5%.

Identification data for specific compounds are as follows:
LC-MS: m/z 356.0 [M+H]⁺(calculated value 356.1, C₁₅H₁₂ClF₂N₃O₃).

### Step 8: Synthesis of intermediates 1-8

Add intermediate 1-7 (2.6 g, 7.31 mmol) to DMF (45 mL) and stir at room temperature, then add cesium carbonate (5.2 g, 15.95 mmol) and 1-bromo-2-chloroethane (12.1 mL, 140.48 mmol) in order. After 2h, quench the reaction with saturated NH₄Cl aqueous solution, and extract with EA (2×100mL), combine the organic layer, and dry and concentrate with Na₂SO₄. Purify the residue on silica gel (elute with hexane containing 0-20% (v/v) EtOAc) to obtain the desired product (2.0g, 5.23mmol) with a yield of 71.7%.

Identification data for specific compounds are as follows:
LC-MS: m/z 382.0 [M+H]⁺(calculated value 382.1, C₁₇H₁₄ClF₂N₃O₃).

### Step 9: Synthesis of intermediates 1-9

Stir the mixture of intermediate **1-8** (150mg, 0.39mmol), zinc cyanide (100mg, 0.85mmol), zinc powder (14.3mg, 0.22mmol), Pd₂(dba)₃ (40mg, 0.04mmol) and DPPF (47mg, 0.08mmol) and N,N-dimethylformamide (3mL) for 2 hours at 130 °C in N₂ atmosphere Cool the reactant to room temperature, quench with saturated NaHCO₃ aqueous solution, and extract with ethyl acetate (3×50 mL), wash the combined organic layer with saline, dry with Na₂SO₄, filter and concentrate under reduced pressure. Purify the residue on silica gel (elute with hexane containing 0-20% (v/v) EtOAc) to obtain the desired product (115mg, 0.31mmol) with a yield of 79.2%.

Identification data for specific compounds are as follows:
LC-MS: m/z 373.0 [M+H]⁺ (calculated value 373.1, C₁₈H₁₄F₂N₄O₃).

### Step 10: Synthesis of intermediate 1-10

Add intermediate **1-9** (60 mg, 0.16 mmol), nickel chloride hexahydrate (7.7 mg, 0.03 mmol) and trifluoroacetic acid (147 mg, 1.29 mmol) to anhydrous methanol (6 mL) at room temperature in N₂ atmosphere, then add sodium borohydride (182.9mg, 4.83 mmol) slowly. After stirring at room temperature for 4 hours, filter and concentrate the reaction solution to obtain the desired product (54.0mg, 0.14mmol) with a yield of 87.5%.

Identification data for specific compounds are as follows:
LC-MS: m/z 377.1 [M+H]⁺ (calculated value 377.1, C₁₈H₁₈F₂N₄O₃).

### Step 11: Synthesis of compound 1

Add acryloyl chloride (12.6mg, 0.14mmol) to the stirred solution of intermediate 1-10 (54.0mg, 0.14mmol) and tetrahydrofuran (6mL) in N₂ atmosphere at 0-5 °C, heat to room temperature, and stir for 10 minutes, quench with saturated NaHCO₃ aqueous solution and extract with ethyl acetate (3×50 mL), wash the combined organic layer with saline, dry with Na₂SO₄, filter and concentrate under reduced pressure. Purify the residue on silica gel (elute with hexane containing 0-75% (v/v) EtOAc) to obtain the desired product (27mg, 0.06mmol) with a yield of 44.8%.

Identification data for specific compounds are as follows:
¹H NMR (500 MHz, DMSO-*d₆*): δ 8.60 (t, *J* = 5.8 Hz, 1H), 8.37 (s, 1H), 6.93 (s, 1H), 6.33 (dd, *J* = 17.1, 10.2 Hz, 1H), 6.12 (dd, *J* = 17.1, 2.1 Hz, 1H), 5.62 (dd, *J* = 10.2, 2.2 Hz, 1H), 5.01 (s, 2H), 4.42 (d, *J* = 5.8 Hz, 2H), 3.99 (s, 6H), 1.77 (q, *J* = 4.0 Hz, 2H), 1.48 (q, *J* = 4.1 Hz, 2H).
LC-MS: m/z 431.0 [M+H]⁺ (calculated value 431.2, C₂₁H₂₀F₂N₄O₄).

### Example 2: Synthesis of N-(3-(2'-3,5-difluoro-2,6-dimethoxypyridine-4-yl)-2',3'-dihydro-1'H-spiro[cyclopropyl-1,4'-[2,7]naphthyridine]-6'-yl) phenyl) acrylamide (compound 2)

The synthesis route is as follows:

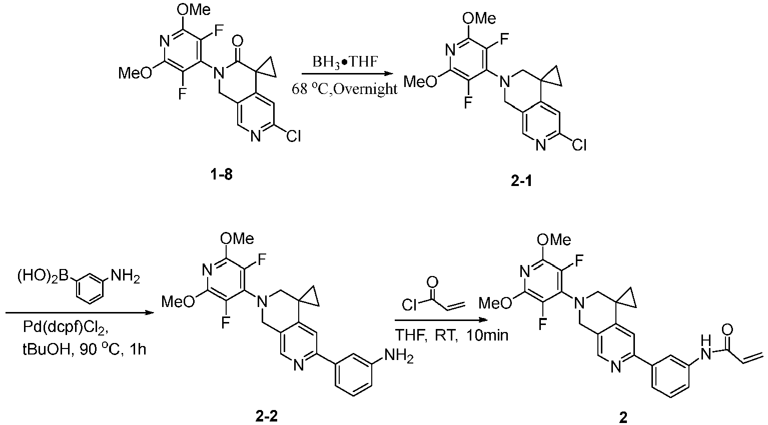

### Step 1: Synthesis of intermediate 2-1

Add intermediate**1-8** (300 mg, 0.79 mmol) to the borane tetrahydrofuran complex (7 mL) and stir overnight at 68 °C. Cool the reactant to room temperature, quench with methanol, add hydrochloric acid aqueous solution (1.3ml, 1mol/L), reheat to 68 °C, and stir for 1 hour. Cool the reactant to room temperature, quench with saturated NaHCO₃ aqueous solution, and extract with ethyl acetate (2×15 mL), wash the combined organic layer with saline, dry with Na₂SO₄, filter and concentrate under reduced pressure. Purify the residue on silica gel (elute with hexane containing 0-10% (v/v) EtOAc) to obtain the desired product (110mg, 0.30mmol) with a yield of 38.0%.

Identification data for specific compounds are as follows:
LC-MS: m/z 368.0 [M+H]⁺(calculated value 368.1, C₁₇H₁₆ClF₂N₃O₂).

### Step 2: Synthesis of intermediate 2-2

Add intermediate **2-1**(60 mg, 0.16 mmol), 3-aminophenylboronic acid (27 mg, 0.20 mmol), [1,1'-bis(diphenylphosphino)ferrocene]palladium dichloride (18 mg, 0.02 mmol) and Na₂CO₃ (38 mg, 0.36 mmol) to tert-butanol (6 mL) and water (6 ml) and stir for 1 hour at 90 °C in N₂ atmosphere Quench the reaction with saturated aqueous NH₄Cl solution and extract with DCM (3×10 mL), combine the organic layers, dry with Na₂SO₄ and concentrate to obtain the desired product (55.0 mg, 0.13 mmol) with a yield of 81.3%.

Identification data for specific compounds are as follows:
LC-MS: m/z 425.1 [M+H]⁺ (calculated value 425.2, C₂₃H₂₂F₂N₄O₂).

### Step 3: Synthesis of compound 2

Add intermediate 2-2 (55.0mg, 0.13mmol) to tetrahydrofuran (10mL) at 0~5°C in N₂ atmosphere and stir, then add acryloyl chloride (11.1mg, 0.12mmol) and heat to room temperature. After stirring for 10 minutes, quench with saturated NaHCO₃ aqueous solution, and extract with DCM (2×20mL), wash the combined organic layer with saline, dry with Na₂SO₄, filter and concentrate under reduced pressure. Prepare, separate and purify the residue to obtain the desired product (44mg, 0.09mmol) with a yield of 70.7%.

Identification data for specific compounds are as follows:
¹H NMR (500 MHz, DMSO-*d₆*): δ 10.26 (s, 1H), 8.41 (s, 1H), 8.29 (t, *J* = 2.0 Hz, 1H), 7.87-7.83 (m, 1H), 7.75 (dt, *J* = 8.0, 1.3 Hz, 1H), 7.41 (t, *J* = 7.9 Hz, 1H), 7.24 (s, 1H), 6.46 (dd, *J* = 17.0, 10.1 Hz, 1H), 6.28 (dd, *J* = 17.0, 2.0 Hz, 1H), 5.77 (dd, *J* = 10.1, 2.0 Hz, 1H), 4.70 (s, 2H), 3.90 (s, 6H), 3.48 (s, 2H), 1.23 (t, *J* = 4.5 Hz, 2H), 1.07 (q, *J* = 4.5 Hz, 2H).
LC-MS: m/z 479.1 [M+H]⁺ (calculated value 479.2, C₂₆H₂₄F₂N₄O₃).

### Example 3: Synthesis of N-(2-((2'-(3,5-difluoro-2,6-dimethoxypyridine-4-yl)-2',3'-dihydro-1'H-spiro[cyclopropane-1,4'-[2,7]naphthyridin]-6'-yl)amino)-5-morpholinophenyl)acrylamide (compound 3)

The synthesis route is as follows:

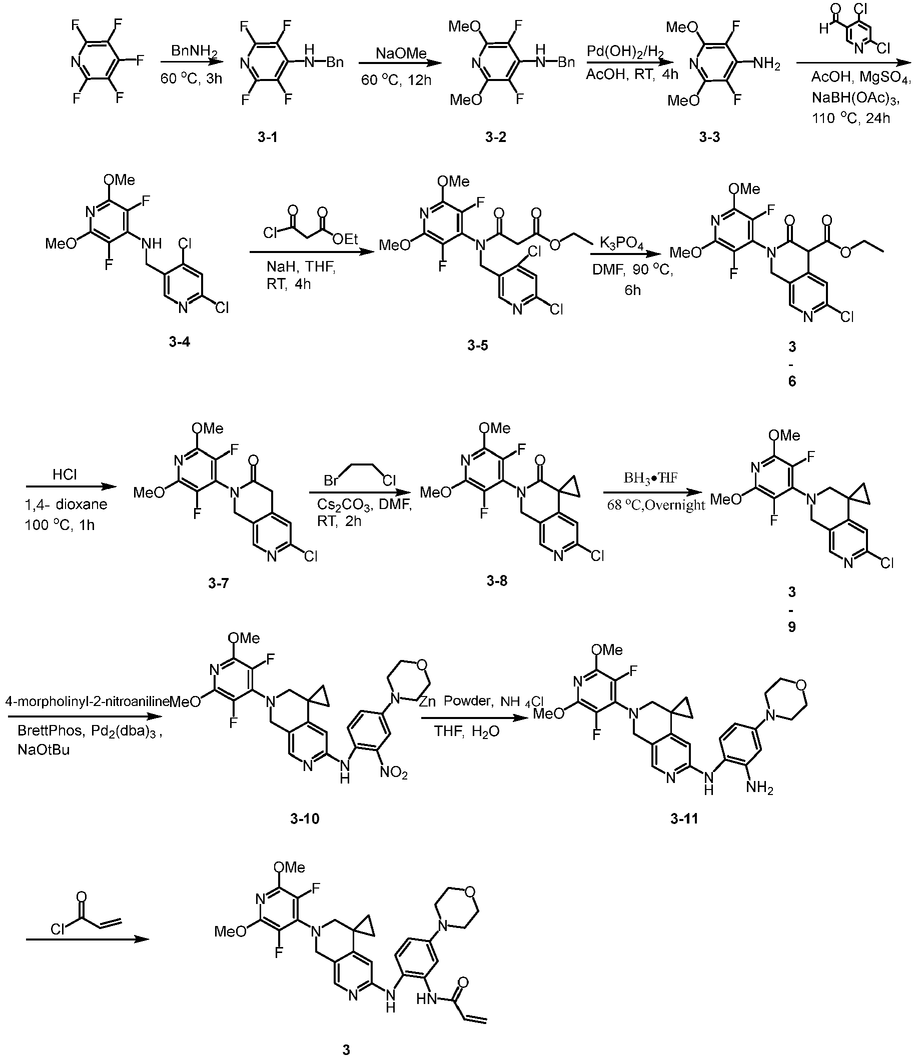

### Step 1: Synthesis of intermediate 3-1

Add pentafluoropyridine (7.2g, 42.59mmol) to acetonitrile (300ml) and stir at room temperature, then add benzylamine (9.0g, 83.99mmol), stir for 1 hour at room temperature and heat to 60 °C. After 3 hours, cool the reactants to room temperature for vacuum concentration. Add DCM (200mL) and water (200mL) to the concentrate, stir to dissolve, stratify, and collect the organic phase. Wash the organic phase once with saturated NaCl solution (200mL), dry and concentrate with anhydrous Na₂SO₄ to obtain a white solid product (10.6g, 41.37mmol) with a yield of 97.1%.

Identification data for specific compounds are as follows:
LC-MS: m/z 257.1 [M+H]⁺ (calculated value 257.1, C₁₂H₈F₄N₂).

### Step 2: Synthesis of intermediate 3-2

Add sodium methoxide (28.5 g, 527.54 mmol) to anhydrous methanol (250 mL) and stir at room temperature, then add compound **3-1** (9.0 g, 35.13 mmol) and heat the obtained mixture to 60 °C. After 12 hours, cool the reactants to room temperature, quench with glacial acetic acid (31.6g, 526.23mmol), concentrate the reaction solution, add water (600mL) and DCM (300mL) to the residue, stir to dissolve, stratify, and collect the organic phase. Extract the aqueous phase once with DCM (300mL), combine the organic phase, wash with water (3×300mL) and dry with anhydrous Na₂SO₄ and concentrate to obtain the desired product (9.5g, 33.89mmol) with a yield of 96.5%.

Identification data for specific compounds are as follows:
LC-MS: m/z 281.1 [M+H]⁺ (calculated value 281.1, C₁₄H₁₄F₂N₂O₂).

### Step 3: Synthesis of intermediate 3-3

Add intermediate **3**-**2** (6.0 g, 21.41 mmol) to acetic acid (120 mL) and stir at room temperature, then add Pd(OH)₂ (1.2g, 8.55 mmol), replace three times with H₂, protect with H₂ balloon, and stir at room temperature for 4 hours. Filter and concentrate the reaction solution to obtain acetate (4.3g, 17.19mmol) of the desired product with a yield of 80.3%.

Identification data for specific compounds are as follows:
LC-MS: m/z 191.1 [M+H]⁺ (calculated value 191.1, C₇H₈F₂N₂O₂).

### Step 4: Synthesis of intermediate 3-4

Add acetate (10.6g, 42.37 mmol) and 4,6-dichloronicotinaldehyde (6.0 g, 34.09 mmol) of intermediate **3-3** to toluene (70 mL) and stir at room temperature, then add glacial acetic acid (3ml) and anhydrous magnesium sulfate (24.0 g, 199.40 mmol) to heat the obtained mixture to 110 °C. After 24 hours, cool the reactants to room temperature and filter, vacuum concentrate the filtrate. Add DCM (70mL) to the obtained concentrate, stir to dissolve, and add trifluoroacetic acid (11.7g, 102.61mmol) to cool down to -5-0 °C. Slowly add sodium triacetoxyborohydride (26.5 g, 125.04 mmol) to the reaction solution. After 4 hours of stirring at room temperature, quench the reaction with saturated NH₄Cl aqueous solution and extract with DCM (3×100mL). Combine the organic layers, dry and concentrate with Na₂SO₄. Purify the residue on silica gel (elute with hexane containing 0-5% (v/v) EtOAc) to obtain the desired product (10.3g, 29.42mmol) with a yield of 69.4%.

Identification data for specific compounds are as follows:
LC-MS: m/z 349.9 [M+H]⁺ (calculated value 350.0, C₁₃H₁₁Cl₂F₂N₃O₂).

### Step 5: Synthesis of intermediate 3-5

Add intermediate **3-4** (5.0 g, 14.28 mmol) to tetrahydrofuran (25 mL) and stir, then add NaH (60% w/w in mineral oil, 600.0 mg, 15.0 mmol) at room temperature. After 10 min, add ethylmalonyl chloride (2.64 mL, 18.68 mmol) dropwise. After 4 h of reaction, quench with the saturated NH₄Cl aqueous solution and extract with EA (2×100 mL), combine the organic layer, dry and concentrate with Na₂SO₄. Purify the residue on silica gel (elute with hexane containing 0-20% (v/v) EtOAc) to obtain the desired product (5.7g, 12.28mmol) with a yield of 86.0%.

Identification data for specific compounds are as follows:
LC-MS: m/z 463.9 [M+H]⁺(calculated value 464.1, C₁₈H₁₇Cl₂F₂N₃O₅).

### Step 6: Synthesis of intermediate 3-6

Add intermediate **3-5** (3.0 g, 6.46 mmol) to DMF (100 mL) and stir at room temperature, then add potassium phosphate (9.0 g, 42.40 mmol), heat the obtained mixture to 90 °C. After 6 h, cool the reactants to room temperature, quench the reaction by adding 2% aqueous acetic acid solution (100 mL), and extract with EA (2×100 mL), combine the organic layers, dry and concentrate with Na₂SO₄. Purify the residue on silica gel (elute with hexane containing 0-20% (v/v) EtOAc) to obtain the desired product (1.5g, 3.5 1mmol) with ayield of 54.3%.

Identification data for specific compounds are as follows:
LC-MS: m/z 428.0 [M+H]⁺(calculated value 428.1, C₁₈H₁₆ClF₂N₃O₅).

### Step 7: Synthesis of intermediate 3-7

Stir intermediate **3-6** (120mg, 0.28mmol) in 1,4-dioxane (6mL) at room temperature, then add concentrated hydrochloric acid (4ml) and heat the obtained mixture to 100 °C. After 1 h, cool the reactants to room temperature, quench with saturated NaHCO₃ aqueous solution, and extract with EA (2×100 mL), combine the organic layers, dry and concentrate with Na₂SO₄. Purify the residue on silica gel (elute with hexane containing 0-25% (v/v) EtOAc) to obtain the desired product (62.3mg, 0.18mmol) with a yield of 62.5%.

Identification data for specific compounds are as follows:
LC-MS: m/z 356.0 [M+H]⁺(calculated value 356.1, C₁₅H₁₂ClF₂N₃O₃).

### Step 8: Synthesis of intermediate 3-8

Add intermediate 3-7 (2.6 g, 7.31 mmol) to DMF (45 mL) and stir at room temperature, then add cesium carbonate (5.2 g, 15.95 mmol) and 1-bromo-2-chloroethane (12.1 mL, 140.48 mmol) in order. After 2h, quench with the saturated NH₄Cl aqueous solution, and extract with EA (2×100mL), combine the organic layer, and dry and concentrate with Na₂SO₄ Purify the residue on silica gel (elute with hexane containing 0-20% (v/v) EtOAc) to obtain the desired product (2.0g, 5.23mmol) with a yield of 71.7%.

Identification data for specific compounds are as follows:
LC-MS: m/z 382.0 [M+H]⁺(calculated value 382.1, C₁₇H₁₄ClF₂N₃O₃).

### Step 9: Synthesis of intermediate 3-9

Add 1.0 mol/L of borane tetrahydrofuran complex (5.3 mL, 5.3 mmol) to a sealed tube containing intermediate **3-8** (250 mg, 0.65 mmol) at room temperature. Heat the obtained mixture to 67 °C and stir for 12 hours with insulation. Quench with methanol, add hydrochloric acid solution (1ml, 1mol/L), heat to 67 °C, stir for 1 hour with insulation, and cool to room temperature. Adjust the pH to ≥7 with saturated NaHCO₃ aqueous solution, extract with EA (2×15mL), combine the organic layers, dry and concentrate with Na₂SO₄. Purify the residue on silica gel (elute with hexane containing 0-10% (v/v) EtOAc) to obtain the desired product (134mg, 0.36mmol) with a yield of 55.6%.

Identification data for specific compounds are as follows:
LC-MS: m/z 368.0 [M+H]⁺(calculated value 368.1, C₁₇H₁₆ClF₂N₃O₂).

### Step 10: Synthesis of intermediate 3-10

Add intermediate **3-9** (60mg, 0.16mmol), 4-morpholinyl-2-nitroaniline (43.8mg, 0.20mmol), 2-(dicyclohexylphosphine)-3,6-dimethoxy-2'-4'-6'-triisopropyl-11'-biphenyl (9.0mg, 0.02mmol), Pd₂(dba)₃ (15.0mg, 0.02mmol) and sodium tert-butoxide (31.3mg, 0.33mmol) to toluene (6ml) and stir for 3 hours at 110 °C in N₂ atmosphere. Cool to room temperature, filter and concentrate, and purify the residue on silica gel (elute with hexane containing 0-20% (v/v) EtOAc) to obtain the desired product (40 mg, 0.07 mmol) with a yield of 45.0%.

Identification data for specific compounds are as follows:
LC-MS: m/z 555.2 [M+H]⁺ (calculated value 555.2, C₂₇H₂₈F₂N₆O₅).

### Step 11: Synthesis of intermediate 3-11

Add intermediate 3-10 (34 mg, 0.06 mmol) to THF (4 mL) and stir at room temperature in N₂ atmosphere, then add zinc powder (260.6 mg, 3.99 mmol) and 9% ammonium chloride aqueous solution (2.1 mL) in order. After 1 h, filter the reaction solution and dry and concentrate the filtrate with Na₂SO₄ to obtain the desired product (27.2 mg, 0.05 mmol).

Identification data for specific compounds are as follows:
LC-MS: m/z 525.3 [M+H]⁺ (calculated value 525.2, C₂₇H₃₀F₂N₆O₃).

### Step 12: Synthesis of compound 3

Add the intermediate 3-11 (27.2mg, 0.05mmol) to tetrahydrofuran (3mL) and stir at 0~5°C in N₂ atmosphere, then add acryloyl chloride (4.53mg, 0.05mmol), heat to room temperature. After stirring for 10 minutes, quench with saturated NaHCO₃ aqueous solution and extract with ethyl acetate (3×50 mL), wash the combined organic layer with saline, dry with Na₂SO₄, filter and concentrate under reduced pressure. Purify the residue on silica gel (elute with hexane containing 0-50% (v/v) EtOAc) to obtain the desired product (7mg, 0.01mmol) with a yield of 24.2%.

Identification data for specific compounds are as follows:
LC-MS: m/z 579.2 [M+H]⁺ (calculated value 579.3, C₃₀H₃₂F₂N₆O₄).

### Example 4: Sythesisi of N-(2-((2'-(3,5-difluoro-2,6-dimethoxypyridine-4-yl)-2',3'-dihydro-1'H-spiro[cyclopropane-1,4'-[2,7]naphthyridine]-6'-yl)amino)-4-morpholinophenyl)acrylamide (compound 4)

The synthesis route is as follows:

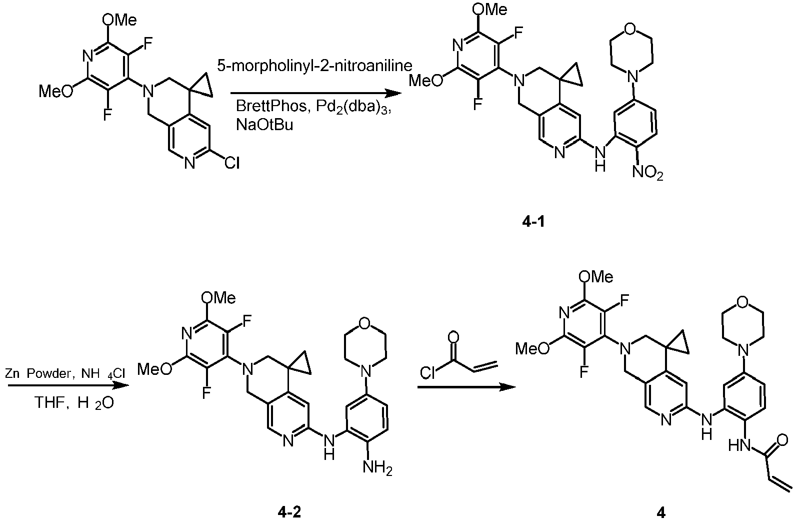

### Step 1: Synthesis of intermediate 4-1

Prepare intermediate **4**-**1** according to the method in step 10 of Example 3.

Identification data for specific compounds are as follows:
LC-MS: m/z 555.2 [M+H]⁺ (calculated value 555.2, C₂₇H₂₈F₂N₆O₅).

### Step 2: Synthesis of intermediate 4-2

Prepare intermediate **4-2** according to the method in step 11 of Example 3.

Identification data for specific compounds are as follows:
LC-MS: m/z 525.3 [M+H]⁺ (calculated value 525.2, C₂₇H₃₀F₂N₆O₃).

### Step 3: Synthesis of compound 4

Prepare compound **4** according to the method in step 12 of Example 3.

Identification data for specific compounds are as follows:
¹H NMR (500 MHz, DMSO-*d₆*): δ 9.64 (s, 1H), 7.98 - 7.94 (m, 2H), 7.39 (d, *J* = 8.8 Hz, 1H), 7.24 (d, *J* = 2.8 Hz, 1H), 6.71 (dd, *J* = 8.8, 2.8 Hz, 1H), 6.47 (dd, *J=* 17.0, 10.2 Hz, 1H), 6.26 (dd, *J* = 17.0, 2.0 Hz, 1H), 6.23 (s, 1H), 5.75 (dd, *J=* 10.2, 2.0 Hz, 1H), 4.59 (s, 2H), 3.95 (s, 6H), 3.79 (d, *J=* 4.4 Hz, 4H), 3.46 (s, 2H), 3.14 - 3.08 (m, 4H), 1.08 - 1.03 (m, 2H), 0.99 - 0.94 (m, 2H).
LC-MS: m/z 579.2 [M+H]⁺ (calculated value 579.3, C₃₀H₃₂F₂N₆O₄).

### Example 5: Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)-amino)-5-(4-morpholinopiperidin-1-yl)phenyl) acrylamide (compound 5)

The synthesis route is as follows:

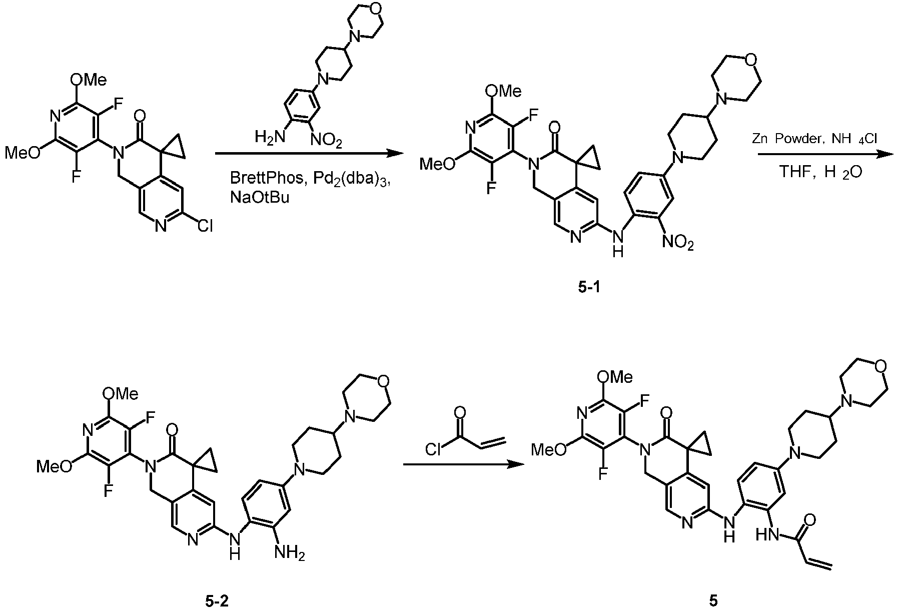

### Step 1: Synthesis of intermediate 5-1

Prepare intermediate **5**-**1** according to the method in step 10 of Example 3.

Identification data for specific compounds are as follows:
LC-MS: m/z 652.2 [M+H]⁺ (calculated value 652.3, C₃₂H₃₅F₂N₇O₆).

### Step 2: Synthesis of intermediate 5-2

Prepare intermediate **5-2** according to the method in step 11 of Example 3.

Identification data for specific compounds are as follows:
LC-MS: m/z 622.3 [M+H]⁺ (calculated value 622.3, C₃₂H₃₇F₂N₇O₄).

### Step 3: Synthesis of compound 5

Prepare compound **5** according to the method in step 12 of Example 3.

Identification data for specific compounds are as follows:
LC-MS: m/z 676.2 [M+H]⁺ (calculated value 676.3, C₃₅H₃₉F₂N₇O₅).

### Example 6: Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)-amino)-4-(4-morpholinopiperidin-1-yl)phenyl) acrylamide (compound 6)

The synthesis route is as follows:

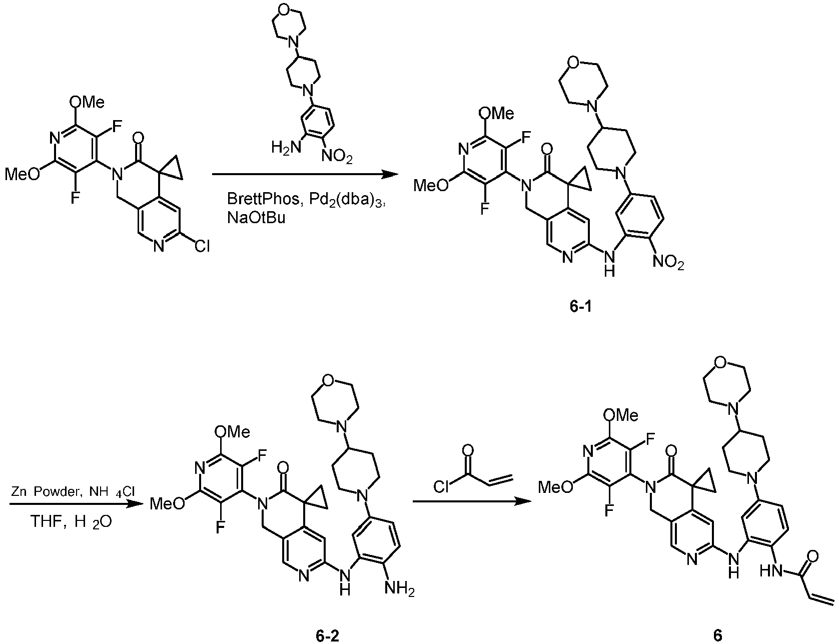

### Step 1: Synthesis of intermediate 6-1

Prepare intermediate **6-1** according to the method in step 10 of Example 3.

Identification data for specific compounds are as follows:
LC-MS: m/z 652.2 [M+H]⁺ (calculated value 652.3, C₃₂H₃₅F₂N₇O₆).

### Step 2: Synthesis of intermediate 6-2

Prepare intermediate **6-2** according to the method in step 11 of Example 3.

Identification data for specific compounds are as follows:
LC-MS: m/z 622.2 [M+H]⁺ (calculated value 622.3, C₃₂H₃₇F₂N₇O₄).

### Step 3: Synthesis of compound 6

Prepare compound 6 according to the method in step 12 of Example 3.

Identification data for specific compounds are as follows:
LC-MS: m/z 676.2 [M+H]⁺ (calculated value 676.3, C₃₅H₃₉F₂N₇O₅).

### Example 7: Synthesis of N-(2-((2'-(3,5-difluoro--2,6-dimethoxypyridine-4-yl)-3'-oxo-2',3'-dihydro-1'H-spiro[cyclopropane-1,4'-[2,7]naphthol]-6'-yl)amino)phenyl) acrylamide (compound 7)

The synthesis route is as follows:

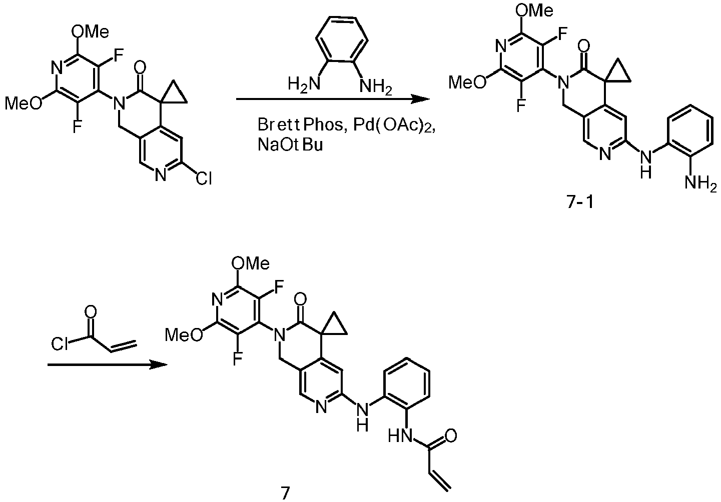

### Step 1: Synthesis of intermediate 7-1

Under N₂ protection, add 6'-chloro-2'-(2,6-difluoro-3,5-dimethoxypyridine)-1',2'-dihydro-3'H-spiro[cyclopropane-1,4'-[2,7]naphthyridine]-3'-one (200mg, 0.52mmol), 1,2-phenylenediamine (60mg, 0.55mmol), palladium acetate (25mg, 0.11mmol), Brettphos (59mg, 0.11mmol) and sodium tert-butoxide (200mg, 2.08mmol) to anhydrous dioxane (10mL) and heat at 110°C in microwave for 1h. After the reaction, filter and wash with EA (5 mL) twice, collect the filtrate and separate the thin layer chromatography after concentration to obtain oily matter 7-1 (80mg).

Identification data for specific compounds are as follows:
LC-MS: m/z 454.1 [M+H]⁺ (calculated value 454.2, C₂₃H₂₁F₂N₅O₃).

### Step 2: Synthesis of compound 7

Dissolve the intermediate 7-1 (80mg) in 3mL of dry THF, and cool to 0 °C, add dropwise 10µL of acryloyl chloride, react for 10min and quench with 0.1mL of water. After distillation of the solvent, prepare, separate and purify the residue to obtain the desired product 7 (5.6mg).

Identification data for specific compounds are as follows:
¹H NMR (500 MHz, DMSO-*d₆*): δ 9.71 (s, 1H), 8.22 (s, 1H), 7.99 (s, 1H), 7.66 (d, *J* = 8.1 Hz, 1H), 7.57 (d, *J* = 7.9 Hz, 1H), 7.15 (td, *J* = 7.7, 1.6 Hz, 1H), 7.05 (td, *J* = 7.6, 1.5 Hz, 1H), 6.48 (dd, *J* = 17.0, 10.2 Hz, 1H), 6.30 (s, 1H), 6.24 (dd, *J* = 17.0, 2.0 Hz, 1H), 5.73 (dd, *J* = 10.2, 2.0 Hz, 1H), 4.88 (s, 2H), 3.98 (s, 6H), 1.69 (q, *J* = 4.0 Hz, 2H), 1.38 (q, *J* = 4.1 Hz, 2H).
LC-MS: m/z 508.2 [M+H]⁺ (calculated value 508.2, C₂₆H₂₃F₂N₅O₄).

### Example 8: Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)amino)-3-methylphenyl)acrylamide (compound 8)

The synthesis route is as follows:

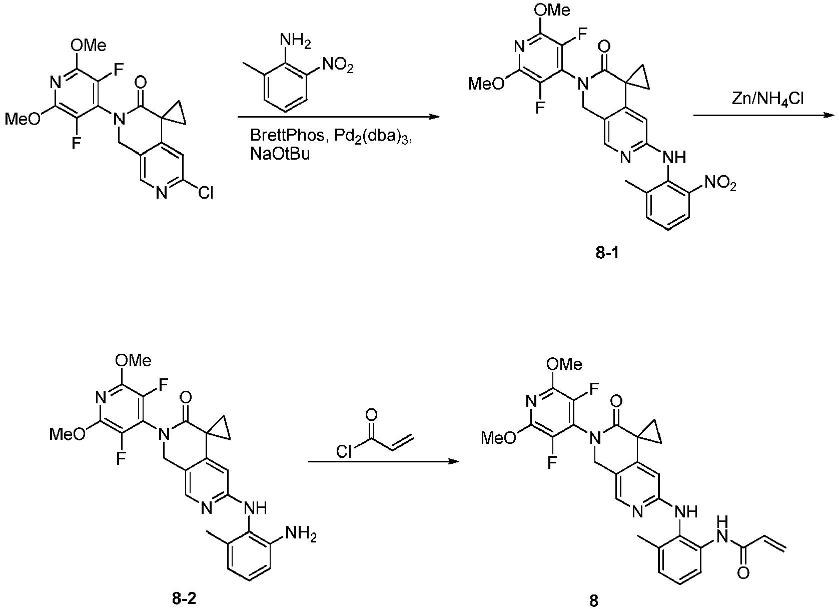

### Step 1: Synthesis of intermediate 8-1

Stir the ultra-dry toluene mixture of 6'-chloro-2'-(2,6-difluoro-3,5-dimethoxypyridine)-1',2'-dihydro-3'H-spiro[cyclopropane-1,4'-[2,7]naphthyridine]-3'-one (100 mg, 0.26 mmol), 2-methyl-6-nitroaniline (47 mg, 0.31 mmol), sodium tert-butoxide (50 mg, 0.52 mmol) and Brettphos (14 mg, 0.026 mmol),Pd₂(dba) 3(24 mg, 0.026 mmol) (5 mL) for 1 hour at 110 °C in N₂ atmosphere. After the TLC proves that reaction is complete, cool down, add EA (5 mL) dilution, filter with diatomaceous earth, wash with EA (5 mL) twice, collect the filtrate, concentrate to dryness under reduced pressure, obtain reddish-brown oil, and purify the residue on silica gel (eluted with hexane containing 0-0-20% EtOAc) to obtain intermediate **8-1** (80 mg).

Identification data for specific compounds are as follows:
LC-MS: m/z 498.2 [M+H]⁺ (calculated value 498.2, C₂₄H₂₁F₂N₅O₅).

### Step 2: Synthesis of intermediate 8-2

At room temperature, add intermediate **8-1** (80mg, 0.16 mmol) to a mixed solvent of tetrahydrofuran (6mL) and water (3mL), add ammonium chloride (500mg, 9.35 mmol) and zinc powder (600mg, 9.17 mmol), and react for 1h at room temperature. After the TLC proves that reaction is complete, filter with diatomaceous earth, wash with THF (5mL) twice, collect the filtrate, alkalize the filtrate with saturated NaHCO₃ aqueous solution, and extract with ethyl acetate (3×10mL). Combine the organic phases, dry, and concentrate to obtain intermediate **8-2** (60mg).

Identification data for specific compounds are as follows:
LC-MS: m/z 468.3 [M+H]⁺ (calculated value 468.2, C₂₄H₂₃F₂N₅O₃).

### Step 3: Synthesis of compound 8

At 0-5°C, add acryloyl chloride (14 µL, 0.17 mmol) dropwise to the stirred tetrahydrofuran (4.0 mL) solution of intermediate **8-2** (80 mg, 0.17 mmol). After 5 minutes, quench the reaction with saturated NaHCO₃ aqueous solution, and extract with dichloromethane. Dry the combined organic layer with Na₂SO₄, filter, and concentrate to dryness under reduced pressure. Prepare, separate and purify the crude product to obtain the desired product **8** (40 mg).

Identification data for specific compounds are as follows:
¹H NMR (500 MHz, DMSO-*d₆*): δ 9.43 (s, 1H), 7.87 (d, *J* = 4.3 Hz, 2H), 7.65 (d, *J* = 8.1 Hz, 1H), 7.15 (t, *J* = 7.8 Hz, 1H), 7.07 (d, *J* = 7.3 Hz, 1H), 6.45 (dd, *J* = 17.0, 10.2 Hz, 1H), 6.18 (dd, *J* = 17.0, 2.0 Hz, 1H), 5.90 (s, 1H), 5.67 (dd, *J* = 10.2, 2.0 Hz, 1H), 4.83 (s, 2H), 3.97 (s, 6H), 2.13 (s, 3H), 1.66 (q, *J* = 4.0 Hz, 2H), 1.27 (q, J= 4.1 Hz, 2H).
LC-MS: m/z 522.3 [M+H]⁺ (calculated value 522.2, C₂₇H₂₅F₂N₅O₄).

### Example 9: Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)-amino)-4-morpholinophenyl) acrylamide (compound 9)

The synthesis route is as follows:

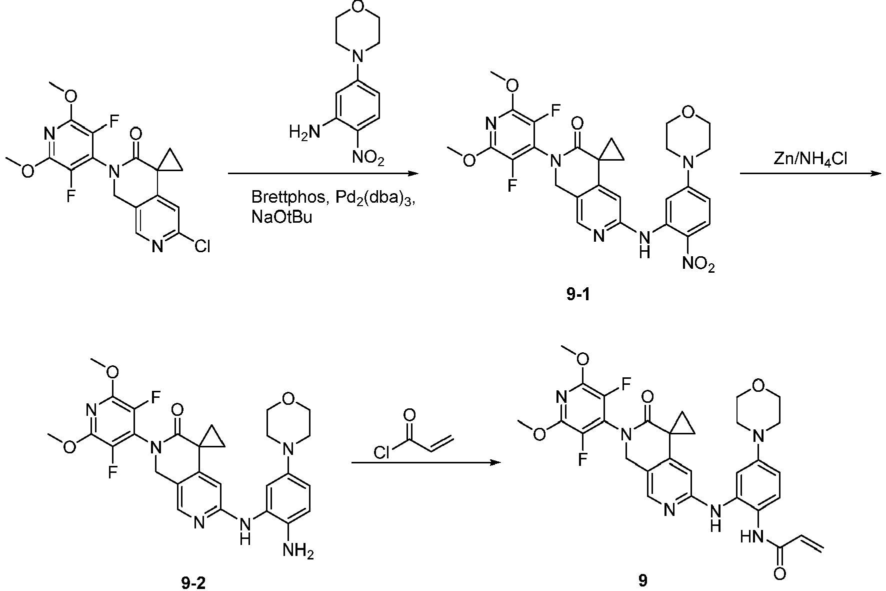

### Step 1: Synthesis of intermediate 9-1

In N₂ atmosphere, stir the ultra-dry toluene (5 mL) mixture of 6'-chloro-2'-(2,6-difluoro-3,5-dimethoxypyridine)-1',2'-dihydro-3'H-spiro[cyclopropane-1,4'-[2,7] naphthyridine]-3'-one (100mg, 0.26 mmol), 5-morpholino-2-nitroaniline (69mg, 0.31 mmol), sodium tert-butoxide (50mg, 0.52 mmol), Brettphos (14 mg, 0.026 mmol) and Pd₂(dba)₃ (24 mg, 0.026 mmol) at 110°C for 1h. After the TLC proves that reaction is complete, cool down, add EA (5 mL) to dilute, filter with diatomaceous earth, wash with EA (5 mL) twice, collect the filtrate, concentrate to dryness under reduced pressure, obtain reddish-brown oil, and purify by column chromatography (eluted in gradient with n-hexane solution containing 0-20% EtOAc) to obtain intermediate **9-1** (80 mg).

Identification data for specific compounds are as follows:
LC-MS: m/z 569.2 [M+H]⁺ (calculated value 569.2, C₂₇H₂₆F₂N₆O₆).

### Step 2: Synthesis of intermediate 9-2

At room temperature, add intermediate **9-1** (80mg, 0.14 mmol) to a mixed solvent of tetrahydrofuran (6mL) and water (3mL), add ammonium chloride (500mg, 9.35 mmol) and zinc powder (600mg, 9.17 mmol), react for 1h, filter with diatomaceous earth, wash with THF (5mL) twice, collect the filtrate, alkalize the filtrate with saturated NaHCO₃ aqueous solution, extract with ethyl acetate (3×10mL), dry and concentrate to obtain intermediate **9-2** (60mg).

Identification data for specific compounds are as follows:
LC-MS: m/z 539.3 [M+H]⁺ (calculated value 539.2, C₂₇H₂₈F₂N₆O₄).

### Step 3: Synthesis of compound 9

Prepare compound **9** according to the method in step 3 of Example 8.

Identification data for specific compounds are as follows:
¹H NMR (500 MHz, DMSO-*d₆*): δ 9.60 (s, 1H), 8.15 (s, 1H), 8.00 (s, 1H), 7.34 (d, *J* = 8.8 Hz, 1H), 7.23 (d, *J* = 2.7 Hz, 1H), 6.68 (dd, *J* = 8.9, 2.7 Hz, 1H), 6.43 (dd, *J* = 17.0, 10.1 Hz, 1H), 6.30 (s, 1H), 6.21 (dd, *J* = 17.0, 2.1 Hz, 1H), 5.69 (dd, *J* = 10.1, 2.1 Hz, 1H), 4.87 (s, 2H), 3.98 (s, 6H), 3.73 (t, *J* = 4.7 Hz, 4H), 3.06 (t, *J* = 4.8 Hz, 4H), 1.69 (q, *J* = 4.0 Hz, 2H), 1.36 (q, *J* = 4.1 Hz, 2H).
LC-MS: m/z 593.3 [M+H]⁺ (calculated value 593.2, C₃₀H₃₀F₂N₆O₅).

### Example 10:

### Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)-amino)-5-morpholinophenyl) acrylamide (compound 10)

The synthesis route is as follows:

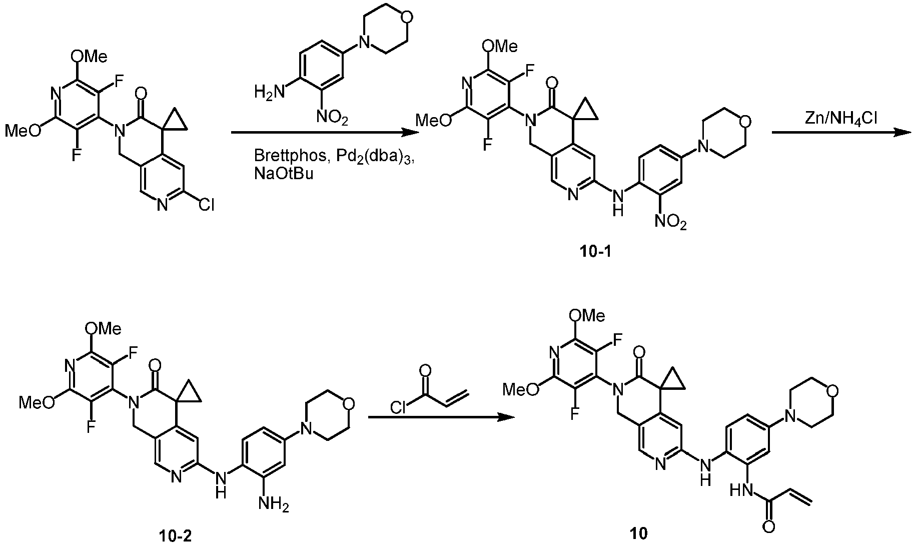

### Step 1: Synthesis of intermediate 10-1

Under nitrogen protection, react the ultra-dry toluene (10 mL) mixture of 6'-chloro-2'-(2,6-difluoro-3,5-dimethoxypyridine)-1',2'-dihydro-3'H-spiro[cyclopropane-1,4'- [2,7] naphthyridine] -3 '-one (130mg, 0.35 mmol), 4-morpholinyl-2-nitroaniline (100mg, 0.45 mmol), palladium (II) acetate (11.8mg, 0.05 mmol), Brettphos (28.1 mg, 0.05 mmol) and sodium tert-butoxide (134 mg, 1.4 mmol) at 130°C for 30 min under microwave condition. After the reaction, carry out suction filtration, wash twice with EA (10 mL), collect the filtrate, concentrate to dryness under reduced pressure, and purify by column chromatography to obtain intermediate **10-1** (47mg, 0.08 mmol).

Identification data for specific compounds are as follows:
LC-MS: m/z 569.3 [M+H]⁺ (calculated value 569.2, C₂₇H₂₆F₂N₆O₆).

### Step 2: Synthesis of intermediate 10-2

At room temperature, add intermediate **10-2** (47mg, 0.08 mmol), zinc powder (250mg, 3.83 mmol) and ammonium chloride (300mg, 5.61 mmol) to methanol (3mL) and stir for 2h, filter by suction, evaporate the solvent to dryness under reduced pressure, add THF (3mL), and evaporate again to dryness under reduced pressure to obtain solid intermediate **10-2** crude product (55mg).

Identification data for specific compounds are as follows:
LC-MSC m/z: 539.3 [M+H]⁺ (calculated value 539.2, C₂₇H₂₈F₂N₆O₄).

### Step 3: Synthesis of compound 10

Dissolve the intermediate **10-2** (55mg) in dry THF (5mL), cool in an ice bath, add 7µL of acryloyl chloride dropwise, react for 10min and quench with 0.1mL of water. After evaporation of the solvent, prepare, separate and purify the residue to obtain the desired product **10** (13.62mg).

Identification data for specific compounds are as follows:
¹H NMR (500 MHz, DMSO-*d₆*): δ 9.61 (s, 1H), 7.95 (s, 1H), 7.92 (s, 1H), 7.34 (d, *J* = 8.8 Hz, 1H), 7.27 (d, *J* = 2.9 Hz, 1H), 6.79 (dd, *J* = 8.9, 2.9 Hz, 1H), 6.45 (dd, *J* = 17.0, 10.2 Hz, 1H), 6.22 (dd, *J* = 17.0, 2.0 Hz, 1H), 6.10 (s, 1H), 5.71 (dd, *J* = 10.1, 2.0 Hz, 1H), 4.84 (s, 2H), 3.98 (s, 6H), 3.74 (t, *J* = 4.7 Hz, 4H), 3.06 (t, *J* = 4.8 Hz, 4H), 1.66 (q, *J* = 4.0 Hz, 2H), 1.32 (q, *J* = 4.2 Hz, 2H).
LC-MS: m/z 593.3 [M+H]⁺ (calculated value 593.2, C₃₀H₃₀F₂N₆O₅).

### Example 11: Synthesis of N-((2'-(3,5-difluoro-2,6-dimethoxypyridine-4-yl)-3'-oxo-2',3'-dihydro-1'H-spiro[cyclopentane-1,4'-[2,7]naphthyridine]-6'-yl) methyl) acrylamide (compound 11)

The synthesis route is as follows:

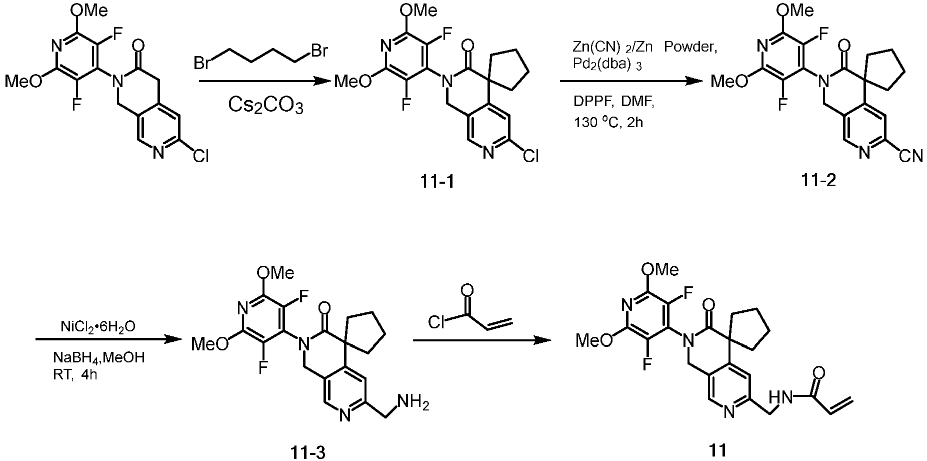

### Step 1: Synthesis of intermediate 11-1

Stir the DMF (5 mL) mixture of 6'-chloro-2'-(2,6-difluoro-3,5-dimethoxypyridine)-1',2'-dihydro-3'H-spiro[cyclopropane-1,4'-[2,7] naphthyridine]-3'-one (355mg, 1.00 mmol), 1,4-dibromobutane (432mg, 2.00 mmol), cesium carbonate (651mg, 2 mmol) at room temperature for 2h. After complete reaction on TLC plate, add 20mL of water to the reaction solution, extract with EA (3x20mL), combine the organic phases, and wash once each with 20mL of water and 20mL saturated salt solution. Dry the organic phase with anhydrous MgSO₄, carry out suction filtration and concentrate under reduced pressure, and purify by column chromatography to obtain intermediate **11-1** (350mg, 0.85 mmol).

Identification data for specific compounds are as follows:
LC-MS: m/z 410.1 [M+H]⁺ (calculated value 410.1, C₁₉H₁₈ClF₂N₃O₃).

### Step 2: Synthesis of intermediate 11-2

Under N₂ protection, add the intermediate **11-**1 (330mg, 0.80 mmol), Pd₂(dba)₃ (110mg, 0.12 mmol), DPPF (67mg, 0.12 mmol), Zn(CN)₂ (140mg, 1.2 mmol) and Zn powder (20mg, 0.30 mmol) to a reaction flask, add DMF (6mL), and react at 140°C for 45 min under microwave condition. After the reaction, add 20mL of water, extract with EA (3x20mL), and combine the organic phases. Wash the organic phase once each with 20mL of water and 20mL of saturated salt solution, dry with anhydrous MgSO₄, carry out suction filtration and concentrate under reduced pressure, and purify the residue by column chromatography to obtain intermediate **11**-**2** (190mg, 0.48 mmol).

Identification data for specific compounds are as follows:
LC-MS: m/z 401.1 [M+H]⁺ (calculated value 401.1, C₂₀H₁₈F₂N₄O₃).

### Step 3: Synthesis of intermediate 11-3

Under N₂ protection, add intermediate **11-2** (90 mg, 0.23 mmol) and nickel chloride hexahydrate (9 mg, 0.04 mmol) to anhydrous methanol (5 mL), add 0.1 mL of trifluoroacetic acid dropwise, stir for 10 minutes, add sodium borohydride (90mg, 2.4 mmol) in three batches and react for 90min. After the reaction, remove the solids by suction filtration, remove the methanol by vacuum distillation, add 5mL of anhydrous THF, and evaporate again to dryness. Proceed directly to the next step of the reaction without further purification. The mass of the residue is about 100 mg.

Identification data for specific compounds are as follows:
LC-MS: m/z 405.2 [M+H]⁺ (calculated value 405.2, C₂₀H₂₂F₂N₄O₃).

### Step 4: Synthesis of compound 11

Prepare compound **11** according to the method in step 3 of Example 10.

Identification data for specific compounds are as follows:
¹H NMR (500 MHz, DMSO-*d₆*): δ 8.71 (t, *J* = 5.9 Hz, 1H), 8.45 (s, 1H), 7.30 (s, 1H), 6.34 (dd, *J* = 17.1, 10.2 Hz, 1H), 6.13 (dd, *J* = 17.1, 2.1 Hz, 1H), 5.64 (dd, *J* = 10.3, 2.1 Hz, 1H), 4.92 (s, 2H), 4.48 (d, *J* = 5.9 Hz, 2H), 3.98 (s, 6H), 2.37 (dt, *J* = 13.1, 6.5 Hz, 2H), 1.98 (dt, *J* = 12.4, 5.6 Hz, 2H), 1.81-1.78 (m, 2H), 1.78 -1.69 (m, 2H).
LC-MS: m/z 459.2 [M+H]⁺ (calculated value 459.2, C₂₃H₂₄F₂N₄O₄).

### Example 12: Synthesis of N-((7-(3,5-difluoro-2,6-dimethoxypyridine-4-yl)-5,5-dimethyl-6-oxo-5,6,7,8 tetrahydro-2,7-naphthyridine-3-yl) methyl) acrylamide (compound 12)

The synthesis route is as follows:

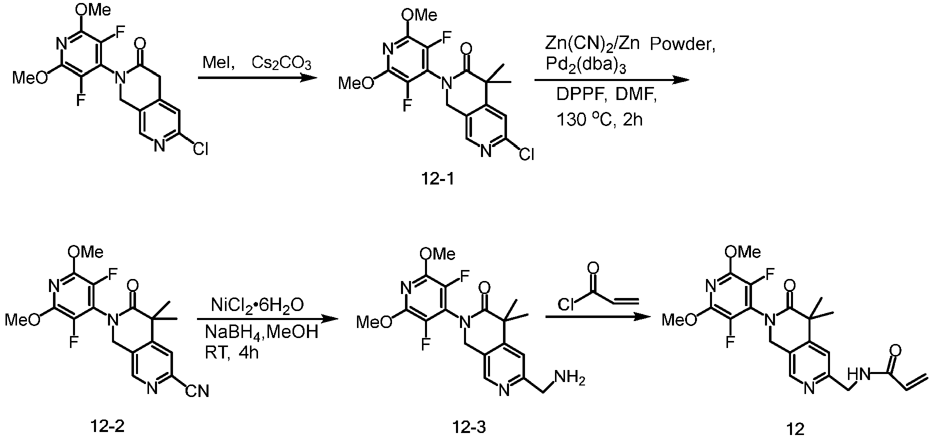

### Step 1: Synthesis of intermediate 12-1

Stir the DMF (10 mL) mixture of 6'-chloro-2'-(2,6-difluoro-3,5-dimethoxypyridine)-1',2'-dihydro-3'H-spiro[cyclopropane-1,4'-[2,7] naphthyridine]-3'-one (500mg, 1.41 mmol), methyl iodide (700mg, 5.00 mmol), cesium carbonate (1.10g, 3.38 mmol) at room temperature for 2h. After the TLC proves that reaction is complete, add 40mL of water to the reaction solution, extract with EA (3x40mL), combine the organic phases, and wash once each with 40mL of water and 40mL saturated salt solution. Dry the organic phase with anhydrous MgSO₄, carry out suction filtration and concentrate to obtain intermediate **12-1** crude product (460mg, 1.20 mmol).

Identification data for specific compounds are as follows:
LC-MS: m/z 384.0 [M+H]⁺ (calculated value 384.1, C₁₇H₁₆ClF₂N₃O₃)

### Step 2: Synthesis of intermediate 12-2

Under N₂ protection, react the mixture of the intermediate **12-1** (400mg, 1.04mmol), Pd₂(dba)₃ (140mg, 0.15 mmol), DPPF (83.1mg, 0.15 mmol), Zn(CN)₂ (176mg, 1.5 mmol), Zn powder (30mg, 0.46 mmol) and DMF (6mL) at 140°C for 45 min under microwave condition. After the reaction, add 20mL of water to quench, extract three times with EA (3x20mL), combine the organic phases, and wash once each with 20mL of water and 20mL of saturated salt solution. Dry the organic phase with anhydrous MgSO₄, carry out suction filtration and concentrate under reduced pressure, and purify the residue by column chromatography to obtain intermediate **12-2** (210mg, 0.56 mmol).

Identification data for specific compounds are as follows:
LC-MS: m/z 375.1 [M+H]⁺ (calculated value 375.1, C₁₈H₁₆F₂N₄O₃).

### Step 3: Synthesis of intermediate 12-3

Under N₂ protection, add intermediate **12-2** (150mg, 0.40 mmol) and nickel chloride hexahydrate (14.2mg, 0.06 mmol) to anhydrous methanol (5 mL), add 0.2 mL of trifluoroacetic acid dropwise, stir for 10 minutes, add sodium borohydride (151mg, 4.0 mmol) in three batches, react for 90min. After the reaction, remove the solids by suction filtration, remove the methanol by vacuum distillation, add 5mL of anhydrous THF, and evaporate again to dryness. The residual intermediate **12-3** crude product (160mg) is proceeded directly to the next step of the reaction without further purification.

Identification data for specific compounds are as follows:
LC-MS: m/z 379.1 [M+H]⁺ (calculated value 379.2, C₁₈H₂₀F₂N₄O₃).

### Step 4: Synthesis of compound 12

Dissolve the residue **12-3** (160mg) from the previous step in 5mL of THF, cool in an ice bath to about 0°C, add 15µL of acryloyl chloride dropwise, and stir for 10min. After the reaction, add 0.1 mL of water to quench. Evaporate under reduced pressure until there is no obvious solvent residue, prepare, separate and purify the residue to obtain the desired product **12** (20.29mg).

Identification data for specific compounds are as follows:
¹H NMR (500 MHz, DMSO-*d₆*): δ 8.71 (t, *J=* 5.9 Hz, 1H), 8.46 (s, 1H), 7.41 (s, 1H), 6.34 (dd, *J =* 17.1, 10.2 Hz, 1H), 6.13 (dd, *J =* 17.1, 2.1 Hz, 1H), 5.63 (dd, *J =* 10.2, 2.1 Hz, 1H), 4.95 (s, 2H), 4.48 (d, *J* = 5.8 Hz, 2H), 3.99 (s, 6H), 1.49 (s, 6H).
LC-MS: m/z 433.2 [M+H]⁺ (calculated value 433.2, C₂₁H₂₂F₂N₄O₄).

### Example 13: Synthesis of N-((2'-(3,5-difluoro-2,6-dimethoxypyridine-4-yl)-3'-oxo-2,2',3,3',5,6-hexahydro-1'H-spiro[pyran-4,4'-[2,7]naphthyridine]-6'-yl) methyl) acrylamide (compound 13)

The synthesis route is as follows:

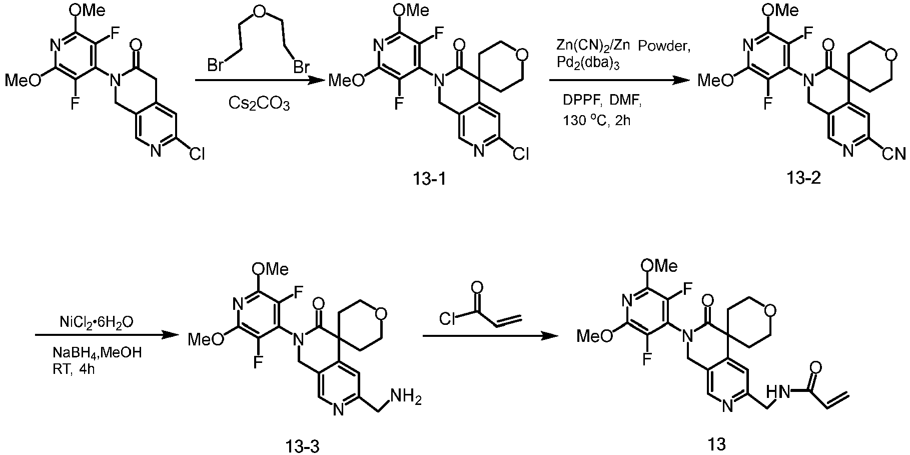

### Step 1: Synthesis of intermediate 13-1

Stir the DMF (5mL) mixture of 6'-chloro-2'-(2,6-difluoro-3,5-dimethoxypyridine)-1',2'-dihydro-3'H-spiro[cyclopropane-1,4'-[2,7] naphthyridine]-3'-one (150mg, 0.42 mmol), bis (2-bromoethyl) ether (195mg, 0.84 mmol), cesium carbonate (390mg, 1.20 mmol) at room temperature for 2h. After the TLC proves that reaction is complete, add 20mL of water to the reaction solution, extract with EA (3x20mL), combine the organic phases, and wash once each with 20mL of water and 20mL saturated salt solution. Dry the organic phase with anhydrous MgSO₄, carry out suction filtration and concentrate to obtain intermediate **13-1** crude product (153mg, 0.36 mmol).

Identification data for specific compounds are as follows:
LC-MS: m/z 426.0 [M+H]⁺ (calculated value 426.1, C₁₉H₁₈ClF₂N₃O₄).

### Step 2: Synthesis of intermediate 13-2

Prepare intermediate **13-2** according to the method in step 2 of Example 12.

Identification data for specific compounds are as follows:
LC-MS: m/z 417.1 [M+H]⁺ (calculated value 417.1, C₂₀H₁₈F₂N₄O₄).

### Step 3: Synthesis of intermediates 13-3

Prepare intermediate **13-3** according to the method in step 3 of Example 11.

Identification data for specific compounds are as follows:
LC-MS: m/z 421.1 [M+H]⁺ (calculated value 421.2, C₂₀H₂₂F₂N₄O₄).

### Step 4: Synthesis of compound 13

Prepare compound **13** according to the method in step 3 of Example 10.

Identification data for specific compounds are as follows:
¹H NMR (500 MHz, DMSO-*d₆*): δ 8.70 (t, *J=* 5.9 Hz, 1H), 8.50 (s, 1H), 7.50 (s, 1H), 6.34 (dd, *J=* 17.1, 10.2 Hz, 1H), 6.14 (dd, *J =* 17.1, 2.1 Hz, 1H), 5.64 (dd, *J =* 10.2, 2.1 Hz, 1H), 4.96 (s, 2H), 4.50 (d, *J* = 5.8 Hz, 2H), 3.99 (s, 6H), 3.90 - 3.77 (m, 4H), 2.18 - 2.08 (m, 2H), 2.01 - 1.92 (m, 2H).
LC-MS: m/z 475.2 [M+H]⁺ (calculated value 475.2, C₂₃H₂₄F₂N₄O₅).

### Example 14: Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)-amino)-4-fluoro-5-morpholinophenyl) acrylamide (compound 14)

The synthesis route is as follows:

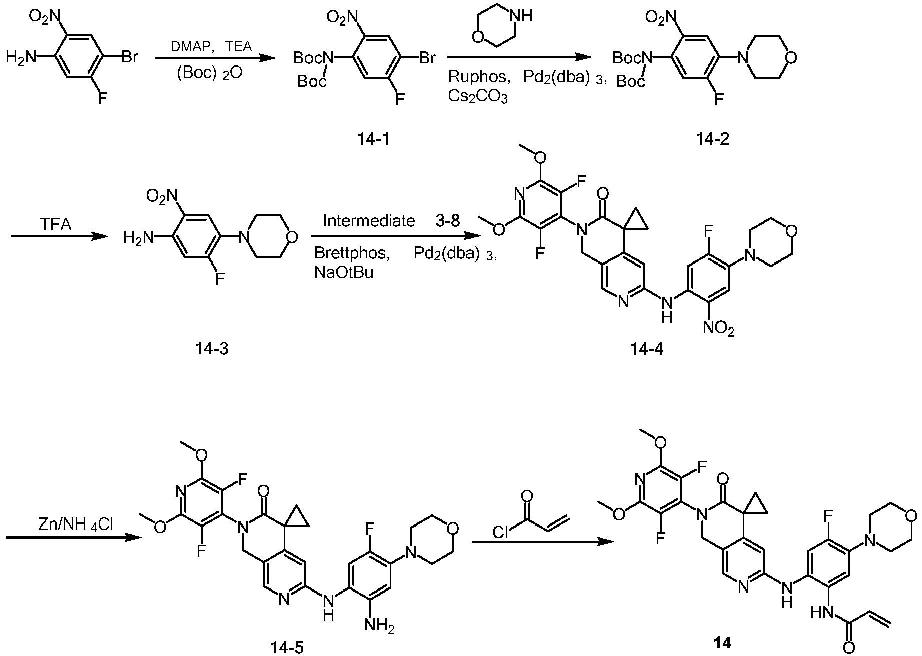

### Step 1: Synthesis of intermediate 14-1

Dissolve 4-bromo-5-fluoro-2-nitroaniline (4.70g, 20 mmol), DMAP (732mg, 6 mmol) and TEA (4mL) in DCM (60mL), add Boc anhydride (21.8g, 100 mmol) dropwise, and react at room temperature for 1h. After the reaction, add saturated ammonium chloride solution to quench, take the organic layer, and wash it once each with 30mL of water and 30mL of saturated salt solution. Dry the organic phase with anhydrous magnesium sulfate, filter by suction and evaporate under reduced pressure to obtain yellow solid **14-1** (5.22g, 15.5 mmol).

Identification data for specific compounds are as follows:
LC-MS: m/z 335.0 [M+H]⁺ (calculated value 335.0, C₁₆H₂0BrFN₂O₆).

### Step 2: Synthesis of intermediate 14-2

Under nitrogen protection, add the intermediate **14-1** (2.51g, 7.50 mmol), morpholine (1.26g, 15.0 mmol), Ruphos (0.50g, 1.14 mmol), Pd₂(dba)₃ (1.00g, 1.14 mmol) and cesium carbonate (7.3g, 23 mmol) to dry dioxane (15mL), and react at 110°C for 30 min. After the reaction, filter by suction, evaporate to dryness under reduced pressure, and purify the residue by column chromatography to obtain intermediate **14-2** (2.00g, 5.86 mmol).

Identification data for specific compounds are as follows:
LC-MS: m/z 442.2 [M+Na]⁺(calculated value 442.2, C₂₀H₂₈FN₃O₇).

### Step 3: Synthesis of intermediate 14-3

At room temperature, dissolve intermediate **14-2** (1.50g, 4.4 mmol) in 20mL DCM, stir, add 12mL of trifluoroacetic acid dropwise, and react for 1h. After the reaction, evaporate to completely remove the solvent to obtain intermediate **14-3** (942mg, 4.01 mmol).

Identification data for specific compounds are as follows:
LC-MS: m/z 242.2 [M+H]₊ (calculated value 242.1, C₁₀H₁₂FN₃O₃).

### Step 4: Synthesis of intermediate 14-4

Under nitrogen protection, add the intermediate **3-8** (178mg, 0.5 mmol, see Example 3), intermediate **14-3** (144mg, 0.6 mmol), palladium (II) acetate (18.6mg, 0.08 mmol), Ruphos (37.3mg, 0.08 mmol) and sodium tert-butoxide (192mg, 2 mmol) to 10mL of toluene, react at 130°C in a microwave reactor for 30min, then evaporate to dryness under reduced pressure, and purify the residue by column chromatography to obtain intermediate **14-4** (96mg, 0.17 mmol).

Identification data for specific compounds are as follows:
LC-MS: m/z 587.3 [M+H]⁺ (calculated value 587.2, C₂₇H₂₅F₃N₆O₆).

### Step 5: Synthesis of intermediate 14-5

At room temperature, add intermediate **14-4** (96mg, 0.17 mmol), zinc powder (500mg, 7.65 mmol) and ammonium chloride (600mg, 9.18 mmol) to methanol (10mL) and stir for 2h, filter by suction, evaporate the solvent to dryness under reduced pressure, add THF (10mL), and evaporate again to dryness to obtain solid intermediate **14-5** crude product (108mg).

Identification data for specific compounds are as follows:
LC-MS: m/z 557.4 [M+H]⁺ (calculated value 557.2, C₂₇H₂₇F₃N₆O₄).

### Step 6: Synthesis of compound 14

Prepare compound **14** according to the method in step 3 of Example 10.

Identification data for specific compounds are as follows:
1HNMR (500 MHz, DMSO-d6): δ 9.63 (s, 1H), 8.12 (s, 1H), 7.99 (s, 1H), 7.61 (d, *J* = 14.7 Hz, 1H), 7.22 (d, *J =* 9.3 Hz, 1H), 6.47 (dd, *J =* 17.0, 10.2 Hz, 1H), 6.33 (s, 1H), 6.24 (dd, *J =* 17.0, 2.0 Hz, 1H), 5.77-5.71 (m, 1H), 4.88 (s, 2H), 3.98 (s, 6H), 3.74 (t, *J=* 4.6 Hz, 4H), 2.95 (t, *J=* 4.6 Hz, 4H), 1.70 (q, *J=* 4.0 Hz, 2H), 1.39 (q, *J* = 4.2 Hz, 2H).
LC-MS: m/z 611.3 [M+H]⁺ (calculated value 611.2, C₃₀H₂₉F₃N₆O₅).

### Example 15: Synthesis of N-(2-((2'-(3,5-difluoro-2,6-dimethoxypyridine-4-yl)-3'-oxo-2',3'-dihydro-1'H-spiro[cyclopropane-1,4'-[2,7]naphthyridine]-6'-yl)-amino)-5-(3-methomorpholinyl) phenyl) acrylamide (compound 15)

The synthesis route is as follows:

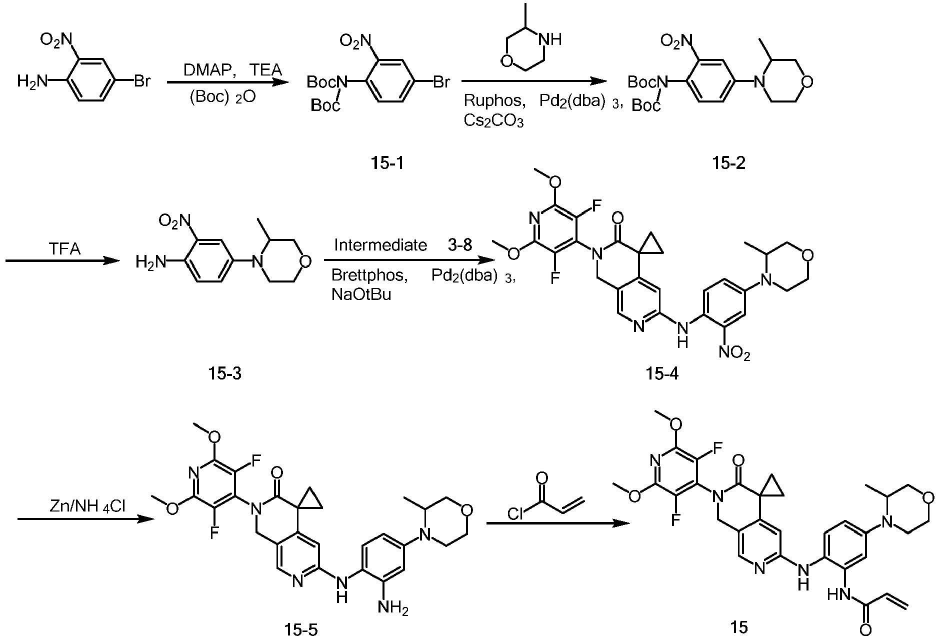

### Step 1: Synthesis of intermediate 15-1

Prepare the yellow solid compound **15-1** (5.12g, 16.2 mmol) according to the method in step 1 of Example 14.

Identification data for specific compounds are as follows:
LC-MS: m/z 439.0 [M+Na]⁺(calculated value 439.0, C₁₆H₂₁BrN₂O₆).

### Step 2: Synthesis of intermediate 15-2

Replace morpholine with 3-methylmorpholine, and prepare the yellow solid compound 15-2 (2.45g, 5.86 mmol) according to the method in step 2 of Example 14.

Identification data for specific compounds are as follows:
LC-MS: m/z 438.3 [M+H]⁺ (calculated value 438.2, C₂₁H₃₁N₃O₇).

### Step 3: Synthesis of intermediates 15-3

Prepare intermediate **15-3** (1104mg, 4.65 mmol) according to the method in step 3 of Example 14. Identification data for specific compounds are as follows:
LC-MS: m/z 238.2 [M+H]⁺ (calculated value 238.1, C₁₁H₁₅N₃O₃).

### Step 4: Synthesis of intermediates 15-4

Prepare intermediate **15-4** (482mg, 0.83 mmol) according to the method in step 4 of Example 14. Identification data for specific compounds are as follows:
LC-MS: m/z 583.2 [M+H]⁺ (calculated value 583.2, C₂₈H₂₈F₂N₆O₆).

### Step 5: Synthesis of intermediates 15-5

Prepare solid residue **15-5** (508mg) according to the method in step 5 of Example 14.

Identification data for specific compounds are as follows:
LC-MS: m/z 553.3 [M+H]⁺ (calculated value 553.2, C₂₈H₃₀F₂N₆O₄).

### Step 6: Synthesis of compound 15

Prepare compound **15** (256.48mg, 0.42 mmol) according to the method in step 3 of Example 10. Identification data for specific compounds are as follows:
¹H NMR (500 MHz, DMSO-*d₆*): δ 9.59 (s, 1H), 7.92 (d, *J =* 4.4 Hz, 2H), 7.33 (d, *J =* 8.8 Hz, 1H), 7.24 (d, *J =* 2.9 Hz, 1H), 6.75 (dd, *J =* 8.9, 2.8 Hz, 1H), 6.45 (dd, *J* = 16.9, 10.2 Hz, 1H), 6.22 (dd, *J =* 17.0, 2.0 Hz, 1H), 6.09 (s, 1H), 5.71 (dd, *J=* 10.1, 2.0 Hz, 1H), 4.85 (s, 2H), 3.98 (s, 6H), 3.92-3.86 (m, 1H), 3.76-3.69 (m, 2H), 3.67-3.61 (m, 1H), 3.58 (td, *J =* 10.8, 3.2 Hz, 1H), 3.10-3.04 (m, 1H), 3.00 (td, *J =* 11.9, 11.2, 3.5 Hz, 1H), 1.66 (q, *J* = 3.9 Hz, 2H), 1.32 (q, *J* = 4.1 Hz, 2H), 1.00 (d, *J* = 6.3 Hz, 3H).
LC-MS: m/z 607.3 [M+H]⁺ (calculated value 607.2, C₃₁H₃₂F₂N₆O₅).

### Example 16: Synthesis of N-((3-(3,5-difluoro-2,6-dimethoxypyridine-4-yl)-2-oxo-1-phenyl-1,2,3,4-tetrahydropyridine [4,3-d]pyrimidin-7-yl) methyl) acrylamide (compound 16)

The synthesis route is as follows:

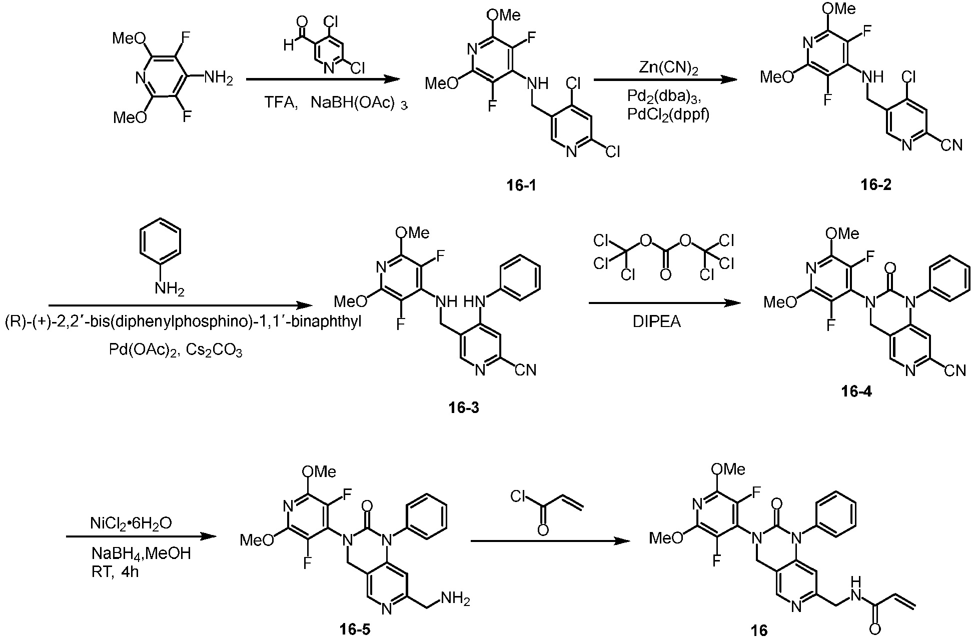

### Step 1: Synthesis of intermediate 16-1

At 0-5°C, add sodium triacetoxyborohydride (635mg, 3.00 mmol) to the stirring mixture of 3,5-difluoro-2,6-dimethoxypyridine-4-amine (200mg, 1.05 mmol), 4,6-dichloronicotinaldehyde (185mg, 1.05 mmol) and dichloromethane (SmL)/trifluoroacetic acid (600µL), remove the ice bath 1 hour later, and react at room temperature. After the TLC proves that reaction is complete, add saturated ammonium chloride aqueous solution to quench, and extract with dichloromethane. Dry the combined organic layer with anhydrous Na₂SO₄, filter, and concentrate to dryness under reduced pressure. Purify the crude product by column chromatography to obtain intermediate **16-1** (220mg).

Identification data for specific compounds are as follows:
LC-MS: m/z 350.1 [M+H]⁺ (calculated value 350.0, C₁₃H₁₁Cl₂F₂N₃O₂).

### Step 2: Synthesis of intermediate 16-2

At 125°C-130°C, under N₂ atmosphere, heat the mixture of the intermediate **16-1** (200mg, 0.57 mmol), zinc cyanide (45 mg, 0.38 mmol), Tris (dibenzylideneacetone)-dipalladium (0) (52 mg, 0.057 mmol), 1,1'-Bis(diphenylphosphino)ferrocene-palladium(II)dichloride dichloromethane complex (1:1) (47 mg, 0.057 mmol) and N,N-dimethylformamide (5 mL) for 1.5 hours. Then, cool the reaction mixture to room temperature, quench with saturated NaHCO₃ aqueous solution, and extract with ethyl acetate (3×10mL). Dry the combined organic layer with saline, dry with MgSO₄, filter, and concentrate under reduced pressure. Purify the residue with silica gel (eluted with hexane containing 0-25% EtOAc) to obtain intermediate **16-2** (130 mg).

Identification data for specific compounds are as follows:
LC-MS: m/z 340.2 [M+H]⁺ (calculated value 340.1, C₁₄H₁₁ClF₂N₄O₂).

### Step 3: Synthesis of intermediate 16-3

Add the intermediate 16-2 (100mg, 0.29 mmol), palladium (II) acetate (20 mg, 0.09 mmol), (R)-(+)-2,2'-bis(diphenylphosphino)-1,1'-binaphthyl (40 mg, 0.06 mmol), cesium carbonate (300 mg, 0.92 mmol) and ultra-dry dioxane (3 mL) to a sealed tube, stir with magnetic force, fully displace with the nitrogen, and finally add aniline (54 µL, 0.58 mmol) with a syringe, place in a microwave reactor, stir, react at 130°C for about 1 hour, then cool down, dilute with EA, filtrate with diatomaceous earth, wash with EA, drain and collect the filtrate, and concentrate to dryness under reduced pressure to obtain reddish-brown oil, that is, the intermediate **16-3** crude product (60mg). The crude product is used directly in the next step without further purification.

Identification data for specific compounds are as follows:
LC-MS: m/z 398.2 [M+H]⁺ (calculated value 398.1, C₂₀H₁₇F₂N₅O₂).

### Step 4: Synthesis of intermediate 16-4

At 0-5°C, add N,N-diisopropylethylamine (116 µL, 0.70 mmol) to the stirred solution of tetrahydrofuran (2.0 mL) of intermediate **16-3** (55 mg, 0.14 mmol), and slowly add triphosgene (42 mg, 0.14 mmol, dissolved with 2 mL of THF). After 5 min, remove the ice bath and react at room temperature. After the reaction, add saturated NaHCO₃ aqueous solution to quench, and extract with ethyl acetate. Dry the combined organic layer with anhydrous Na₂SO₄, filter, and concentrate to dryness under reduced pressure. Purify the crude product by column chromatography to obtain intermediate **16-4** (30mg).

Identification data for specific compounds are as follows:
LC-MS: m/z 424.1 [M+H]⁺ (calculated value 424.1, C₂₁H₁₅F₂N₅O₃).

### Step 5: Synthesis of intermediate 16-5

Prepare compound **16-5** (40mg) according to the method in step 3 of Example 11.

Identification data for specific compounds are as follows:
LC-MS: m/z 428.2 [M+H]⁺ (calculated value 428.2, C₂₁H₁₉F₂N₅O₃).

### Step 6: Synthesis of compound 16

Prepare compound **16** (40mg) according to the method in step 3 of Example 10.

Identification data for specific compounds are as follows:
¹H NMR (500 MHz, DMSO-*d₆*): δ 8.56 (t, *J =* 5.9 Hz, 1H), 8.31 (s, 1H), 7.55 (t, *J* = 7.5 Hz, 2H), 7.50 (t, *J=* 7.3 Hz, 1H), 7.34 (d, *J* = 7.6 Hz, 2H), 6.14 (dd, *J* = 17.1, 10.2 Hz, 1H), 6.07 (s, 1H), 6.02-5.94 (m, 1H), 5.57-5.51 (m, 1H), 5.02 (s, 2H), 4.23 (d, *J =* 6.0 Hz, 2H), 3.98 (s, 6H).
LC-MS: m/z 482.2 [M+H]⁺ (calculated value 482.2, C₂₄H₂₁F₂N₅O₄).

### Example 17: Synthesis of N- ((1-cyclopropyl-3-(3,5-difluoro-2,6-dimethoxypyridine-4-yl)-2-oxo-1,2,3,4-tetrahydropyridine[4,3-d]pyrimidin-7-yl) meth) acrylamide (compound 17)

The synthesis route is as follows:

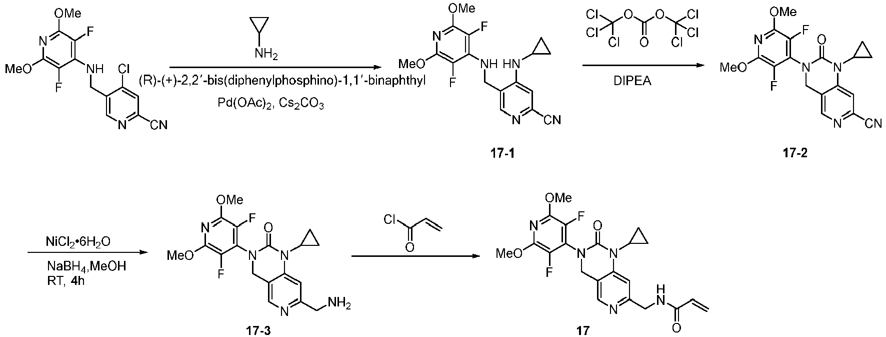

### Step 1: Synthesis of intermediate 17-1

Add 6'-chloro-2'-(2,6-difluoro-3,5-dimethoxypyridine)-1',2'-dihydro-3'H-spiro [cyclopropane-1,4'-[2,7] naphthyridine]-3'-one (100 mg, 0.29 mmol), palladium (II) acetate (20 mg, 0.09 mmol), (R)-(+)-2,2'-bis(diphenylphosphino)-1,1'-binaphthyl (40 mg, 0.06 mmol), cesium carbonate (300 mg, 0.92 mmol) and ultra-dry dioxane (3 mL) to a sealed tube, stir with magnetic force, fully displace with the nitrogen, and finally add cyclopropylamine (40 µL, 0.58 mmol) with a syringe, completely react in a microwave reactor at 130°C for about 1 hour, then cool down, dilute with EA, filtrate with diatomaceous earth, wash with EA, drain and collect the filtrate, and concentrate to dryness under reduced pressure to obtain reddish-brown oil, that is, the intermediate **17-1** crude product (60mg). The crude product is used directly in the next step without further purification.

Identification data for specific compounds are as follows:
LC-MS: m/z 362.1 [M+H]⁺ (calculated value 362.1, C₁₇H₁₇F₂N₅O₂).

### Step 2: Synthesis of intermediate 17-2

Prepare intermediate **17-2** (30mg) according to the method in step 4 of Example 16.

Identification data for specific compounds are as follows:
LC-MS: m/z 388.2 [M+H]⁺ (calculated value 388.1, C₁₈H₁₅F₂N₅O₃).

### Step 3: Synthesis of intermediate 17-3

Prepare intermediate **17-3** (40mg) according to the method in step 3 of Example 11.

Identification data for specific compounds are as follows:
LC-MS: m/z 392.2 [M+H]⁺ (calculated value 392.2, C₁₈H₁₉F₂N₅O₃).

### Step 4: Synthesis of compound 17

Prepare compound **17** (40mg) according to the method in step 3 of Example 10.

Identification data for specific compounds are as follows:
¹H NMR (500 MHz, DMSO-*d₆*): δ 8.73 (t, *J=* 5.9 Hz, 1H), 8.24 (s, 1H), 7.23 (s, 1H), 6.35 (dd, *J=* 17.1, 10.2 Hz, 1H), 6.14 (dd, *J =* 17.1, 2.1 Hz, 1H), 5.64 (dd, *J =* 10.2, 2.1 Hz, 1H), 4.73 (s, 2H), 4.44 (d, *J =* 5.9 Hz, 2H), 3.97 (s, 6H), 2.79 (tt, *J =* 6.9, 3.7 Hz, 1H), 1.04 (td, *J =* 7.3, 5.4 Hz, 2H), 0.64-0.55 (m, 2H).
LC-MS:
   LC-MS: m/z 446.2 [M+H]⁺ (calculated value 446.2, C₂₁H₂₁F₂N₅O₄).

### Pharmacological Tests

### Test Case A: Kinase test

The effect of compounds of the present invention on tyrosine kinase FGFR1 and FGFR4 activity is evaluated by an in vitro kinase test. The method used in the test was homogeneous time-resolved fluorescence (HTRF).

Compound preparation: Add 45 µL of DMSO to 5 µL of stock solution at a concentration of 10 mM to prepare the solution LY2874455 at 1000 µM; then add 12 µL of the compound at 1000 µM to 88 µL of DMSO to prepare the solution LY2874455 at 120 µM as the starting concentration; add 48 µL of DMSO to 2 µL of stock solution at a concentration of 10 mM to prepare the solution BLU554 at 400 µM as the starting concentration; add 15 µL of DMSO to 10 µL of sample compound stock solution at a concentration of 10 mM to prepare a sample compound solution at 4 mM as the starting concentration; transfer the compound solution to the target plate with the dispenser Echo.

Kinase reaction: Add 5 µL of kinase solution to each well, incubate the compound and kinase at room temperature for 60 min, add 5 µL of substrate and ATP mixture to start reacting at 37°C for a certain period of time (30min for FGFR1 kinase test, and 40min for FGFR4 kinase test), and then add 10 µL of X1665 and antibody detection reagent mixture to stop; after 60 min of incubation at room temperature, read the TR-FRET signal with microplate reader SPARK 10M at the transmit wavelength of 665 nM/612 nM. Test enzyme activity at 10 concentrations for each compound, and calculate the IC₅₀ value of the compound with the data with the analysis software, as shown in Table 1.

**Table 1 IC₅₀ Values and Selective Inhibition of Compounds**

| Compound No. | FGFR1 IC₅₀ (nM) | FGFR4 IC₅₀ (nM) | FGFR4/FGFR1 |
|---|---|---|---|
| Compound 1 | 3658.94 | 8.57 | 426 |
| Compound 5 | 6 | 0.3 | 20 |
| Compound 7 | 4243 | 5.42 | 782 |
| Compound 8 | 8567 | 2.7 | 3172 |
| Compound 10 | 984 | 0.67 | 1468 |
| Compound 14 | >9926 | 2.6 | 3817 |
| Compound 15 | 2279 | 0.63 | 3617 |
| BLU554 | 624 | 5.98 | 104 |

| | | | |
|---|---|---|---|
| Note: BLU554 is the Compound 40 disclosed by Blueprint Medicines Corporation in WO2015061572. | | | |

It can be seen from Table 1 that the compounds of the present invention have an inhibitory effect on FGFR4 kinase much stronger than that on FGFR1, which indicates good selectivity.

### Test Example B: Cell proliferation test

The effect of the compounds of the present invention on the proliferation of human hepatoma cells Hep3B is evaluated by an in vitro cell test. The method used in the test was CELL TITER-GLO (CTG).

Cell plating: Take Hep3B cells in the logarithmic growth phase, digest, centrifuge at 1,000 rpm for 5 min at room temperature and collect, resuspend with EMEM medium of 10% FBS and count, and inoculate into a 384-well microplate (#3765) at a density of 800 cells/well, 20 µL in each.

Compound preparation: Dissolve the compound in DMSO to prepare a stock solution at a concentration of 10 mM, dilute the sample compound stock solution to 2 mM with DMSO (6 µL of the stock solution plus 24 µL of DMSO) as the starting concentration, and dilute the control compound stock solution to 200 µM with DMSO (2 µL of the stock solution plus 98 µL of DMSO) as the starting concentration; dilute the compound 9 times with DMSO in gradient by 3 folds, and then dilute the compound to 40 times the final concentration dose with cell culture medium (2 µL of the compound plus 78 µL of DMSO).

Cell dosing: Add 5 µL (5×) of the compound to the corresponding wells, make sure the final concentrations of sample compounds are respectively 10000 nM, 3333 nM, 1111 nM, 370.37 nM, 123.46 nM, 41.15 nM, 13.72 nM, 4.57 nM, 1.52 nM, 0.50 nM, take 0.5% DMSO as the negative control, and incubate the microplate at 37°C in 5% CO₂ for 3 days.

CTG detection: After 3 days of incubation, add 5 µL of Cell Titer-Glo^{®} reagent per well, incubate on a shaker at 300 rpm for 60 min protected from light at room temperature, read the luminescence signals with microplate reader Spark 10M, and calculate the semi-inhibitory concentration of the compound on cell proliferation, that is, the IC₅₀ value, with the analysis software, as shown in Table 2.

**Table 2 IC₅₀ Values of Compounds for Hep3B Cells**

| Compound No. | IC₅₀ Value of Compound for Hep3B Cells (nM) |
|---|---|
| Compound 1 | 52.58 |
| Compound 8 | 56.49 |
| Compound 10 | 13.99 |
| Compound 14 | 38.07 |
| Compound 15 | 15.30 |
| BLU554 | 38.37 |

As can be seen from Table 2, the compounds of the present invention have a good inhibitory effect on the proliferation of Hep3B cells.

## Claims

1. The compound in formula (I) or pharmaceutically acceptable salts, esters, stereoisomers, tautomers, solvates, chelates, non-covalent complexes or prodrugs thereof. 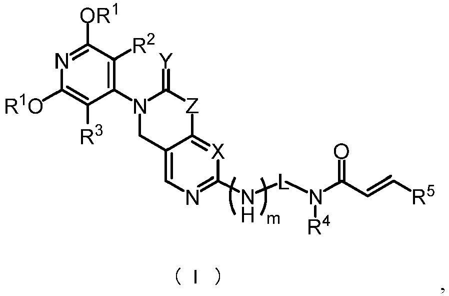 in which,
X is CH or N;
Y is 2 Hs or 1 O;
Z is C(R⁶)₂ or N(R⁶);
m is 0 or 1;
L is -C(R⁷)₂- or 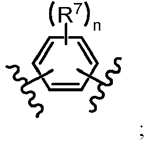
n is 0, 1, or 2;
Each R¹ is independently C₁₋₃ alkyl or C₁₋₃ haloalkyl;
Each R² and R³ are independently hydrogen, halogen, C₁₋₃ alkyl, C₁₋₃ haloalkyl, cyano, or C₁₋₃ alkoxy;
R⁴ is hydrogen, C₁₋₆ alkyl or C₆₋₁₀ aryl;
R⁵ is hydrogen, halogen, cyano, nitro, trifluoromethyl, amino, C₁₋₆ alkyl, C₂₋₈ alkenyl, C₆₋₁₀ aryl, C₁₋₈ alkyl amino, bis (C₂₋₈ alkyl) amino, C₂₋₈ alkynyl, C₁₋₈ haloalkyl or C₃₋₈ cycloalkyl;
Each R⁶ is independently hydrogen, C₁₋₈ alkyl, C₂₋₈ alkenyl, C₂₋₈ alkynyl, C₁₋₈ haloalkyl, C₆₋₁₀ aryl, C₆₋₁₀ aryl with substituents, C₃₋₁₀ cycloalkyl, 5 to 10 membered heteroaryl or 4 to 10 membered heterocycloalkyl; wherein each 5 to 10 membered heteroaryl or 4 to 10 membered heterocycloalkyl independently contains 1 to 3 cyclic heteroatoms, the heteroatoms are N, O or S independently;
Or two R⁶ and the carbon atoms connected thereto together form a C₃₋₈ cycloalkyl or 4 to 10 membered heterocycloalkyl; wherein the 4 to 10 membered heterocycloalkyl contains 1 to 3 cyclic heteroatoms, the heteroatoms are N, O or S; each C₃₋₈ cycloalkyl and 4 to 10 membered heterocycloalkyl are independently replaced by 1 to 4 substituents, the substituents are each independently halogen, cyano, hydroxyl, amino, C₁₋₈ carboxyamide, C₁₋₈ carboxylacyl, C₁₋₈ alkyl or C₁₋₈ alkoxy;
Each R⁷ is independently hydrogen, halogen, amino, cyano, C₁₋₈ alkyl, C₁₋₈ alkyl with substituent, C₁₋₈ alkoxy, C₁₋₈ alkoxy with substituents, C₁₋₈ alkyl amino, bis (C₂₋₈ alkyl) amino, C₂₋₈ alkenyl, C₂₋₈ alkenyl with substituents, C₂₋₈ alkynyl, C₂₋₈ alkynyl with substituents, C₆₋₁₀ aryl, C₆₋₁₀ aryl with substituents, C₃₋₈ cycloalkyl, C₃₋₈ cycloalkyl with substituents, 3 to 10 membered heterocycloalkyl, 3 to 10 membered heterocycloalkyl with substituents, 5 to 10 membered heteroaryl or 5 to 10 membered heteroaryl with substituents; wherein each 3 to 10 membered heterocycloalkyl and 5 to 10 membered heteroaryl independently contains 1 to 3 cyclic heteroatoms, the heteroatoms are N, O or S independently.

2. The compound presented in claim 1, or a pharmaceutically acceptable salt, ester, stereoisomer, tautomer, solvate, chelate, non-covalent complex or prodrug thereof, the characteristic is that the compound has a structure in formula (II): 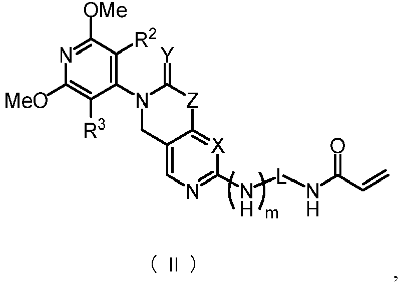 Where X, Y, Z, m, L, R² and R³ are as defined in claim 1.

3. The compound presented in claim 1 or 2, or a pharmaceutically acceptable salt, ester, stereoisomer, tautomer, solvate, chelate, non-covalent complex or prodrug thereof, the characteristic is that the compound has a structure in formula (III): 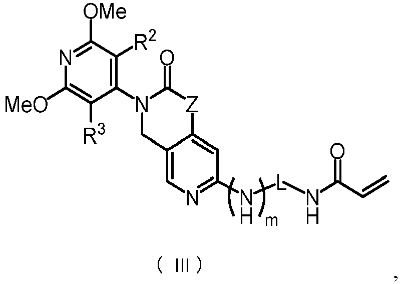 Where Z, m, L, R² and R³ are as defined in claim 1 or 2.

4. The compound presented in claim 1 or 2, or a pharmaceutically acceptable salt, ester, stereoisomer, tautomer, solvate, chelate, non-covalent complex or prodrug thereof, the characteristic is that the compound has a structure in formula (III-1) or formula (III-2): 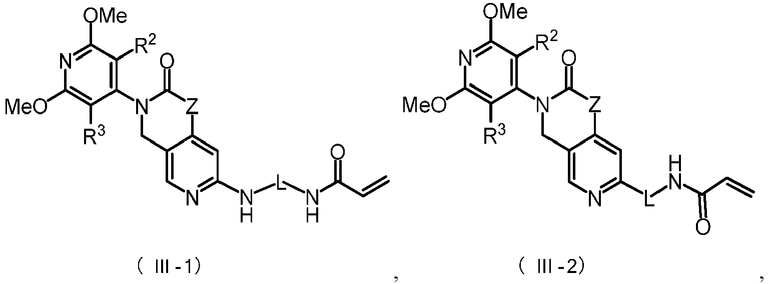
Where Z, L, R² and R³ are as defined in claim 1 or 2.
Preferably, the compound of formula (III-1) has a structure as shown in formula (III-1-1): 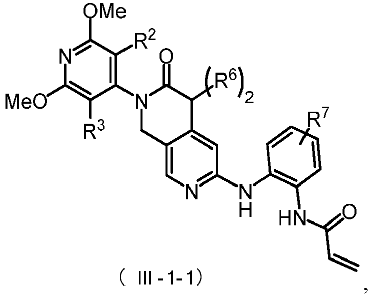
Where R², R³ and R⁶ are as defined in claim 1 or 2, R⁷ is hydrogen, C₁₋₄ alkyl, piperazinyl, piperidinyl or morpholinoyl, wherein each C₁₋₄ alkyl, piperazinyl, piperidinyl or morpholinoyl are optionally replaced by at least one R⁸. Each R⁸ is independently hydrogen, C₁₋₄ alkyl, morpholinyl, bridged-ring morpholinyl, piperazinyl, piperazinyl with substituents, bridge-ring piperazinyl, bridged-ring piperazinyl with substituents, oxetalkyl or oxetalkyl with the substituents, the substituent is C₁₋₄ alkyl.
Preferably, R², R³ and R⁶ are as defined in claim 1 or 2, R⁷ is hydrogen, C₁₋₄ alkyl, piperazinyl, piperidinyl or morpholinoyl, wherein each C₁₋₄ alkyl, piperazinyl, piperidinyl or morpholinoyl are optionally replaced by at least one R⁸. Each R⁸ is independently hydrogen, morpholinyl, bridged-ring morpholinyl, piperazinyl, piperazinyl with substituents, bridge-ring piperazinyl, bridged-ring piperazinyl with substituents, oxetalkyl or oxetalkyl with substituents, the substituent is C₁₋₄ alkyl.
More preferably, R², R³ and R⁶ are as defined in claim 1 or 2, R⁷ is one of the following fragments: 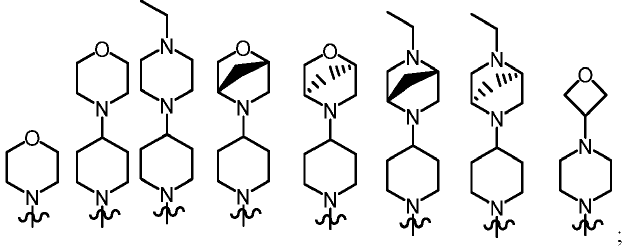
Preferably, the compound of formula (III-2) has a structure as shown in formula (III-2-1) or formula (III-2-2): 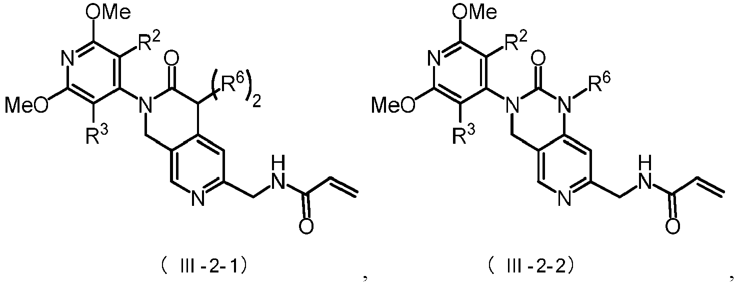
Where R², R³ and R⁶ are as defined in claim 1 or 2.

5. The compound presented in claim 1 or 2, or a pharmaceutically acceptable salt, ester, stereoisomer, tautomer, solvate, chelate, non-covalent complex or prodrug thereof, the characteristic is that the compound has a structure in formula (IV): 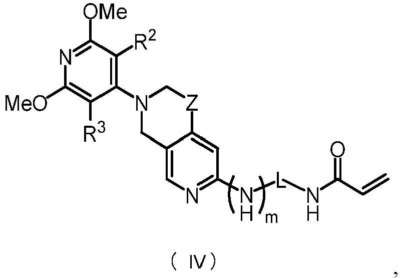 Where Z, m, L, R² and R³ are as defined in claim 1 or 2.

6. The compound presented in claim 1 or 2, or a pharmaceutically acceptable salt, ester, stereoisomer, tautomer, solvate, chelate, non-covalent complex or prodrug thereof, the characteristic is that the compound has a structure in formula (IV-1) or formula (IV-2): 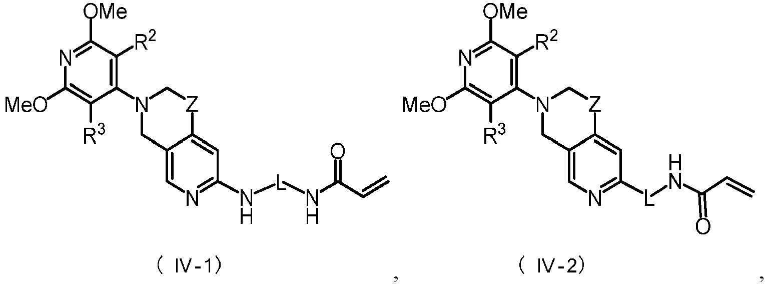
Where Z, L, R² and R³ are as defined in claim 1 or 2.
Preferably, the compound of formula (IV-1) has a structure as shown in formula (IV-1-1): 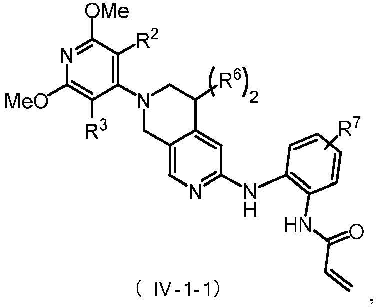
Where, R², R³ and R⁶ are as defined in claim 1 or 2, R⁷ is hydrogen, C₁₋₄ alkyl, piperazinyl, piperidinyl or morpholinoyl, wherein each C₁₋₄ alkyl, piperazinyl, piperidinyl or morpholinoyl are optionally replaced by at least one R⁸. Each R⁸ is independently hydrogen, morpholinyl, bridged-ring morpholinyl, piperazinyl, piperazinyl with substituents, bridge-ring piperazinyl, bridged-ring piperazinyl with substituents, oxetalkyl or oxetalkyl with substituents, the substituent is C₁₋₄ alkyl.
More preferably, R², R³ and R⁶ are as defined in claim 1 or 2, R⁷ is one of the following fragments: 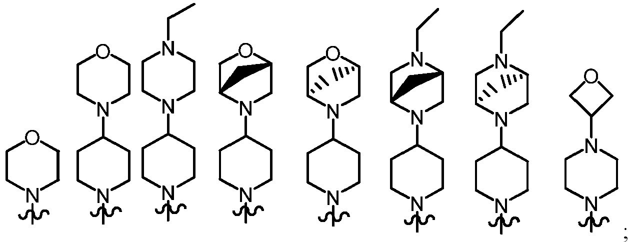
Preferably, the compound of formula (IV-2) has a structure as shown in formula (IV-2-1) or formula (IV-2-2): 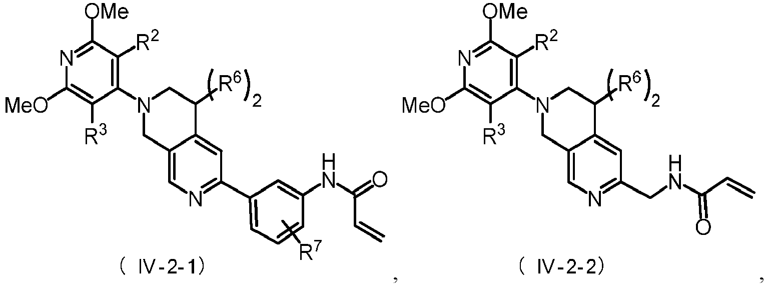
Where, R², R³ and R⁶ in formula (IV-2-1) are as defined in claim 1 or 2, and R⁷ is hydrogen, C₁₋₄ alkyl or 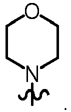
R², R³ and R⁶ in formula (IV-2-2) are as defined in claim 1 or 2.

7. The following compounds or pharmaceutically acceptable salts, esters, stereoisomers, tautomers, solvates, chelates, non-covalent complexes or prodrugs thereof. 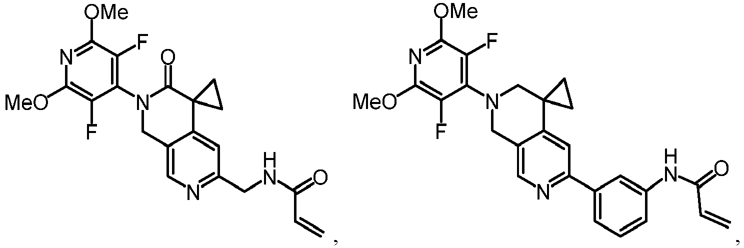 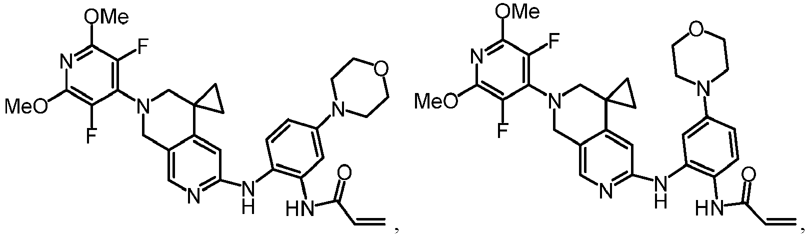 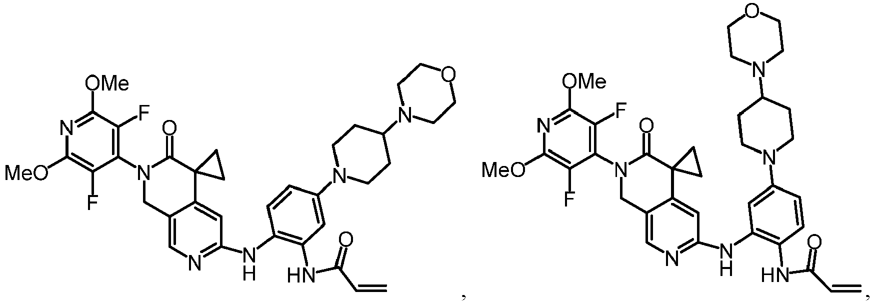 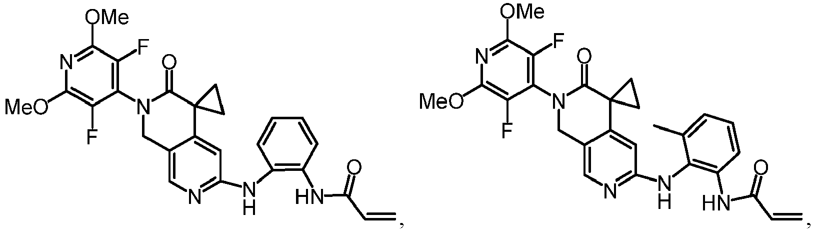 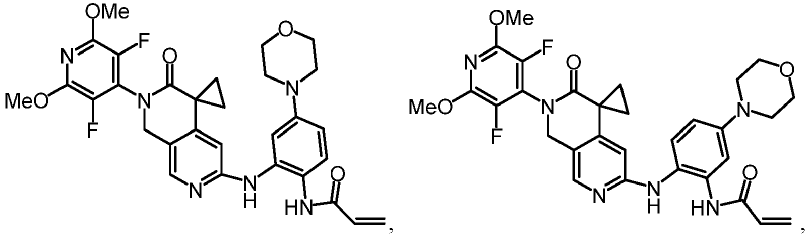 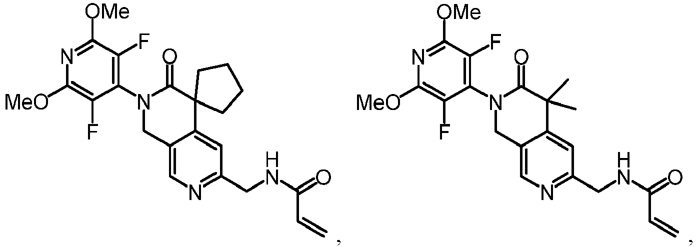 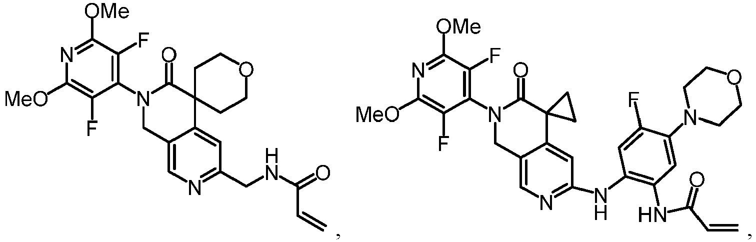 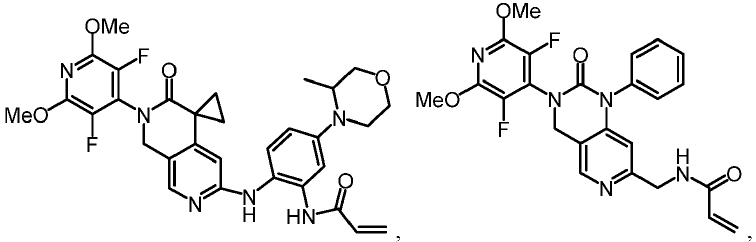 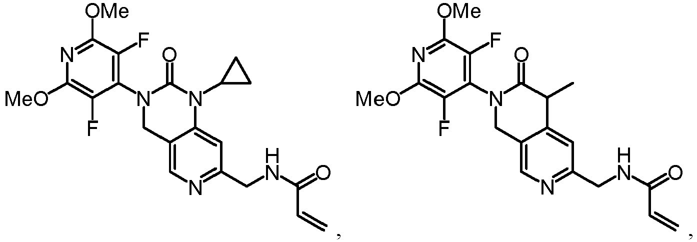 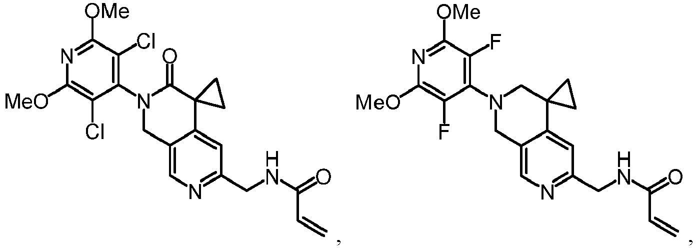 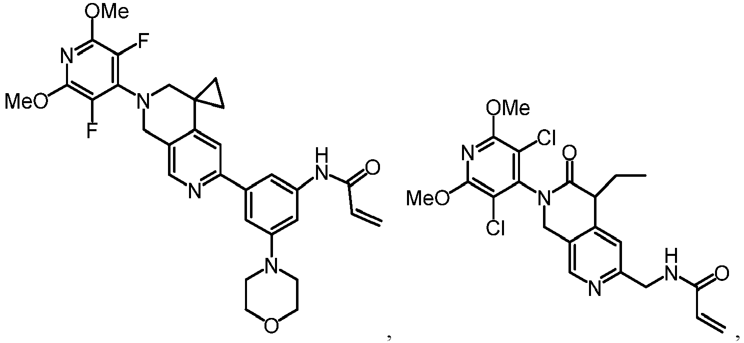 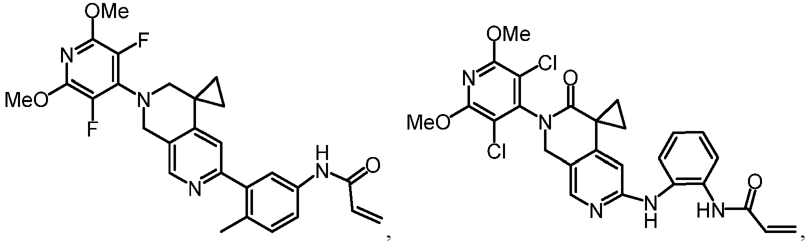 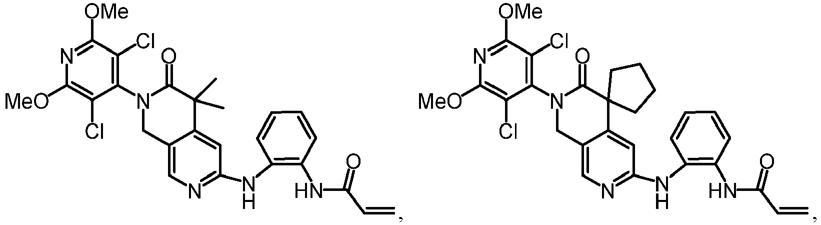 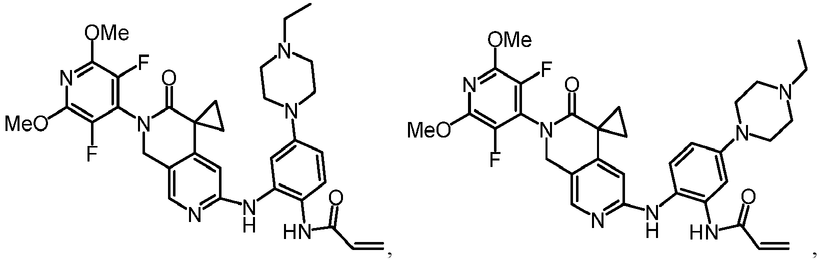 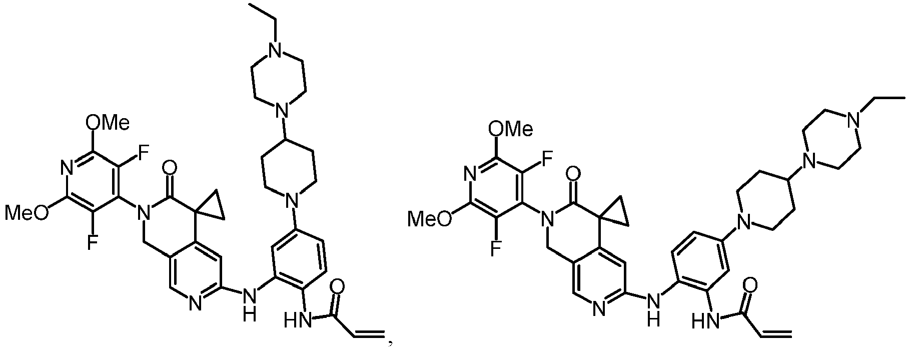 and

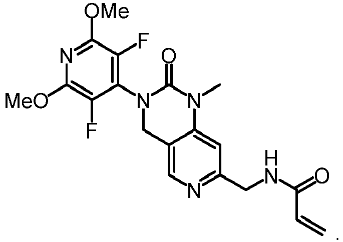

8. A pharmaceutical composition comprising any compound specified in claim 1-7, or the pharmaceutically acceptable salts, esters, stereoisomers, tautomers, solvates, chelates, non-covalent complexes or prodrugs thereof.

9. A pharmaceutical preparation comprising any compound specified in claim 1-7, or the pharmaceutically acceptable salts, esters, stereoisomers, tautomers, solvates, chelates, non-covalent complexes or prodrugs thereof, or the pharmaceutical composition according to claim 8, the pharmaceutical preparation is any one of tablet, capsule, injection, granule, powder, suppository, pill, gel, pulvis, oral solution, inhalant, suspension, or dry suspension.

10. The use of any compound specified in claim 1-7, or the pharmaceutically acceptable salts, esters, stereoisomers, tautomers, solvates, chelates, non-covalent complexes or prodrugs thereof, or the pharmaceutical composition described in claim 8, or the pharmaceutical preparation described in claim 9 in the preparation of drugs for preventing and/or treating the disease at least partially mediated by FGFR4.
Preferably, the disease at least partially mediated by FGFR4 includes cancer;
Preferably, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

11. The use of any compound specified in claim 1-7, or the pharmaceutically acceptable salts, esters, stereoisomers, tautomers, solvates, chelates, non-covalent complexes or prodrugs thereof, or the pharmaceutical composition described in claim 8, or the pharmaceutical preparation described in claim 9, as an EGFR4 inhibitor.

12. A method for preventing and/or treating the disease at least partially mediated by FGFR4, which comprises the following steps: the prevention / or treatment of effective amounts of any compound specified in claim 1-7, or the pharmaceutically acceptable salts, esters, stereoisomers, tautomers, solvates, chelates, non-covalent complexes or prodrugs thereof, or the pharmaceutical composition described in claim 8, or the pharmaceutical preparation described in claim 9, administered to patients in need
Preferably, the disease at least partially mediated by FGFR4 includes cancer;
Preferably, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

13. A method for preventing and / or treating cancer, which comprises the following steps: the prevention / or treatment of effective amounts of any compound specified in claim 1-7, or the pharmaceutically acceptable salts, esters, stereoisomers, tautomers, solvates, chelates, non-covalent complexes or prodrugs thereof, or the pharmaceutical composition described in claim 8, or the pharmaceutical preparation described in claim 9, and at least one additional cancer therapeutic agent, administered to patients in need.
Preferably, the cancer is selected from the group consisting of hepatocellular carcinoma, bladder cancer, breast cancer, cervical cancer, colorectal cancer, endometrial cancer, gastric cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin or non-Hodgkin's lymphoma, Waldenstrom macroglobulinemia, hairy cell lymphoma, Burket lymphoma, glioblastoma, melanoma, mesothelioma, neuroblastoma, testicular cancer, squamous cell carcinoma, glioblastoma, and rhabdomyosarcoma.

14. A drug combination comprising any compound specified in claim 1-7, or the pharmaceutically acceptable salts, esters, stereoisomers, tautomers, solvates, chelates, non-covalent complexes or prodrugs thereof, or the pharmaceutical composition described in claim 8, or the pharmaceutical preparation described in claim 9, and at least one additional cancer therapeutic agent.
